(19) Europäisches Patentamt
European Patent Office
Office européen des brevets

(11) EP 3 856 156 B1

(12) # EUROPEAN PATENT SPECIFICATION

(45) Date of publication and mention of the grant of the patent:
**29.10.2025 Bulletin 2025/44**

(21) Application number: **19790145.7**

(22) Date of filing: **30.09.2019**

(51) International Patent Classification (IPC):
***A61K 31/00*** *(2006.01)* ***A61K 31/40*** *(2006.01)*
***A61K 31/454*** *(2006.01)* ***A61K 31/495*** *(2006.01)*
***A61K 31/496*** *(2006.01)* ***A61K 31/55*** *(2006.01)*
***A61K 31/551*** *(2006.01)* ***A61P 25/16*** *(2006.01)*
***A61P 25/28*** *(2006.01)*

(52) Cooperative Patent Classification (CPC):
**A61K 31/495; A61K 31/00; A61K 31/40; A61K 31/454; A61K 31/496; A61K 31/55; A61K 31/551; A61P 25/16; A61P 25/28**

(86) International application number:
**PCT/EP2019/076397**

(87) International publication number:
**WO 2020/065090 (02.04.2020 Gazette 2020/14)**

(54) **5-HT7 ANTAGONIST FOR THE TREATMENT OF DEMENTIA-ASSOCIATED TAUOPATHIES**

5-HT7 ANTAGONIST ZUR BEHANDLUNG VON DEMENZASSOZIIERTEN TAUOPATHIEN

ANTAGONISTE DE 5-HT7 POUR LE TRAITEMENT DES TAUOPATHIES ASSOCIÉES À LA DÉMENCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2018 EP 18197401**

(43) Date of publication of application:
**04.08.2021 Bulletin 2021/31**

(73) Proprietors:
- **Medizinische Hochschule Hannover (MHH)**
  **30625 Hannover (DE)**
- **Deutsches Zentrum für Neurodegenerative Erkrankungen e.V. (DZNE)**
  **53127 Bonn (DE)**

(72) Inventors:
- **PONIMASKIN, Evgeni**
  **30627 Hannover (DE)**
- **ROEHRS, Kian-Fritz**
  **27283 Verden/Aller (DE)**
- **BUTZLAFF, Malte**
  **31311 Uetze (DE)**
- **LABUS, Josephine**
  **30159 Hannover (DE)**
- **DITYATEV, Alexander**
  **39175 Wahlitz (DE)**
- **VARBANOV, Hristo**
  **55122 Mainz (DE)**
- **JIA, Shaobo**
  **39104 Magdeburg (DE)**
- **SUN, Weilun**
  **39104 Magdeburg (DE)**

(74) Representative: **Schiweck Weinzierl Koch Patentanwälte Partnerschaft mbB**
**Ganghoferstraße 68 B**
**80339 München (DE)**

(56) References cited:
**WO-A1-00/37082** **WO-A1-02/062788**
**WO-A2-2013/038200**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **GASBARRI A ET AL: "Effect of 5-HT"7 antagonist SB-269970 in the modulation of working and reference memory in the rat", BEHAVIOURAL BRAIN RESEARCH, ELSEVIER, AMSTERDAM, NL, vol. 195, no. 1, 16 December 2008 (2008-12-16), pages 164 - 170, XP025471553, ISSN: 0166-4328, [retrieved on 20080109], DOI: 10.1016/J.BBR.2007.12.020**
- **ALEXANDRE V. IVACHTCHENKO ET AL: "AVN-101: A Multi-Target Drug Candidate for the Treatment of CNS Disorders", JOURNAL OF ALZHEIMER'S DISEASE, vol. 53, no. 2, 13 July 2016 (2016-07-13), NL, pages 583 - 620, XP055568533, ISSN: 1387-2877, DOI: 10.3233/ JAD-151146**
- **MALTE BUTZLAFF ET AL.: "The Role of Serotonin Receptors in Alzheimer's Disease", OPERA MEDICA ET PHYSIOLOGICA, vol. 2, no. 1, January 2016 (2016-01-01), pages 77 - 86, XP002789898**
- **KATHRIN JAHREIS, JOSEPHINE LABUS, EVGENI PONIMASKIN: "The role of 5-HT7-receptor signalling in neurodegenerativediseases", March 2019 (2019-03-01), pages T11-12B, XP002789899, Retrieved from the Internet <URL:https://www. nwg-goettingen.de/2019/upload/file/ Proceedings-NWG-2019.pdf> [retrieved on 20190320]**


Remarks:

The complete document including Reference Table(s) and the Sequence Listing(s) can be downloaded from the EPO website

## Description

### TECHNICAL FIELD OF THE INVENTION

The present invention relates to a 5-HT7 receptor antagonist for use in preventing or treating a tauopathy. Further, the present invention also relates to a pharmaceutical composition for use in preventing or treating a tauopathy comprising the 5-HT7 receptor antagonist as mentioned above.

### BACKGROUND OF THE INVENTION

Aggregation of the microtubule-associated tau protein has been reported in a large portion of neurodegenerative diseases. These so-called tauopathies are characterized by the deposition of hyper-phosphorylated, aggregated tau within the neurons and/or glial cells (Arendt et al., 2016, Brain Res. Bull. 126, 238-292). The most prominent members in this class of disease are Alzheimer's disease (AD) and frontotemporal lobar degeneration (FTLD), causing the majority of dementia illnesses worldwide (Duthey, 2013, Background paper 6.11: Alzheimer disease and other dementias. A public Healthy Approach to Innovation). Intracellular inclusions comprising tau are also found in several other neurodegenerative diseases, including Pick disease, progressive supranuclear palsy, corticobasal degeneration, and frontotemporal dementia with parkinsonism linked to chromosome 17 (FTDP-17) (Josephs, 2017, Mayo Clin. Proc. 92, 1291-1303).

Under physiological conditions, tau participates in regulating microtubule network dynamics by binding and stabilizing microtubules. It also promotes polymerization of tubulin and is known to influence cell morphology, axonal outgrowth and the axonal cargo transport (Wang and Mandelkow, 2015, Nat. Rev. Neurosci. 17, 22-35). Tau's functions are mainly regulated by phosphorylation at multiple sites mediated by a variety of kinases (Buee et al., 2000, Brain Res Brain Res Rev 33, 95-130).

Under pathological conditions, tau phosphorylation is increased, thus resulting in reduced microtubule binding affinity and protein aggregation. In turn, this leads to destabilization of the microtubule network, impairment of axonal transport, inhibition of proteasomal degradation pathways and mitochondrial dysfunction (Arendt et al., 2016, Brain Res. Bull. 126, 238-292).

Gasbarri A et Al; Behavioural Brain Research; vol. 195, no. 1, 16 December 2008 (2008-12-16), pages 164-170, discloses the effect of 5-HT7 antagonist SB-269970 in the modulation of working and reference memory in the rat.

In recent years, the serotonergic system regained scientific attention as a potential target for the treatment of neurodegenerative diseases associated with increased tau phosphorylation and accumulations of tau proteins.

Since the prevalence of neurodegenerative diseases such as tauopathy is clearly on the rise, and no disease-modifying drugs for the treatment of these diseases are available, the identification of new molecular targets seems to be urgent.

Therefore, there is a need in the art to provide new ways for the prevention and treatment of tauopathy.

Therefore, the objective of the present invention is to comply with this need.

The solution of the present invention is described in the following, exemplified in the appended examples, illustrated in the figures and reflected in the claims.

### SUMMARY OF THE INVENTION

The present invention deals with the target serotonin (5-hydroxytryptamine) receptor 7 (5-HT7R) for treating or preventing tauopaties, such as Alzheimer's disease. It was demonstrated that 5-HT7 receptor antagonists prevent hyperphosphorylation of the tau protein and through that mechanism the formation of tau-tangles in nerve cells is inhibited as well as neurotoxic effects abolished. These findings demonstrate a significant role for 5-HT7R signaling in tau-induced pathology. Following these observations, the application of 5-HT7 receptor antagonists could likely represent an innovative treatment strategy, with potentially disease modifying properties by reducing tau aggregation and subsequent neuronal cell death. Thus, targeting the 5-HT7R reduces abnormal aggregation of tau protein and resulting neurotoxicity, as well as restores neuronal functioning. Therefore, it represents a new promising therapeutic strategy in tau-mediated neurological disorders such as Alzheimer's disease.

In particular, it was demonstrated that 5-HT7 receptor antagonists that target and block 5-HT7R may be used for the prevention or treatment of diseases associated with the accumulation of hyperphosphorylated tau protein (p-tau), such as tauopathies.

The present invention provides evidence that targeting the 5-HT7R may reduce abnormal aggregation of tau protein and resulting neurotoxicity, as well as restore neuronal functioning, thus representing a new promising therapeutic strategy in tau-mediated neurological disorders.

Thus, the present invention relates to a 5-HT7 receptor antagonist for use in preventing or treating a tauopathy by prevention of both the receptor-induced phosphorylation and accumulation of Tau proteins, wherein the 5-HT7 receptor

antagonist has a structure according to the following formula (I),

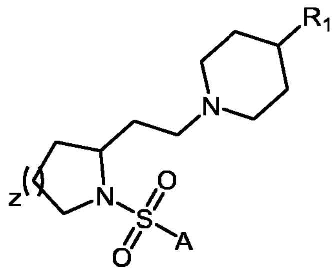

(I)

wherein in formula (I)

$R_1$ is -($C_1$-$C_6$)alkyl, preferably methyl, ($C_6$-$C_{10}$)aryl, -O($C_6$-$C_{10}$)aryl, or ($C_5$-$C_{10}$)heteroaryl, which are optionally substituted with one or more substituents independently selected from the group consisting of: halogen, preferably -Cl, or ($C_1$-$C_6$)Alkyl;
A is ($C_6$-$C_{10}$)aryl, or ($C_5$-$C_{10}$)heteroaryl, which are optionally substituted with one or more substituents independently selected from the group consisting of: halogen, -OH, ($C_1$-$C_6$)alkyl, or -O($C_1$-$C_6$)alkyl, preferably the at least one substituent is a meta-substituent;
z is 1
or is a pharmaceutically acceptable salt, enantiomer, diastereomer, racemic mixture, crystalline form, amorphous, unsolved form or solvate of the general formula (I).

Most preferably, the 5-HT7 receptor antagonist is SB-269970.

Further, the present invention may comprise the 5-HT7 receptor antagonist for use as mentioned above, wherein the antagonist is a small organic molecule comprising at least two carbon atoms having a molecular weight in the range between 100 and 1000 Dalton.

The present invention also comprises a pharmaceutical composition for use in preventing or treating a tauopathy comprising the 5-HT7 receptor antagonist as mentioned above.

Contemplated by the present invention is the 5-HT7 receptor antagonist for use as mentioned above, or the pharmaceutical composition as mentioned above, for use in preventing or treating a tauopathy by prevention of both the receptor-induced phosphorylation and accumulation of tau proteins.

Additionally, the present invention may comprise the 5-HT7 receptor antagonist for use as mentioned above, or the pharmaceutical composition as mentioned above, wherein tauopathy is dementia-associated tauopathy.

Further, the present invention may also comprise the 5-HT7 receptor antagonist for use as mentioned above, or the pharmaceutical composition as mentioned above, wherein tauopathy is selected from the group consisting of Alzheimer's disease, frontotemporal dementia, primary age-related tauopathy (PART), chronic traumatic encephalopathy, progressive supranuclear palsy (PSP), corticobasal degeneration, dementia with Lewy Bodies (DLB), frontotemporal dementia and parkinsonism linked to chromosome 17 (FTDP-17), argyrophilic grain disease (AGD), Huntington disease, glial globular tauopathy, amyotrophic lateral sclerosis (ALS), Parkinson's disease, spinal muscular atrophy (SMA), cerebral amyloid angiopathy (CAA).

## BRIEF DESCRIPTION OF THE FIGURES

**Fig. 1: 5-HT7R induces tau hyperphosphorylation and accumulation in a Ga12-independent manner.**
**A and B.** Representative Western blots and corresponding quantifications are shown. N1E-115 cells were transfected with eGFP-Tau[R406W] mutant alone or together with (HA)5-HT7R, and treated with either $H_2O$, 20 $\mu$M 5-HT, 100 nM SB-269970 alone or in combination with 5-HT. Membranes were probed with phospho-specific tau antibody AT270 **(A)** or 5A6 antibody **(B)** for assessing total tau levels. Results were normalized to GAPDH expression and represented as mean + SD. Water-treated cells where set as 100% (N=5). **C and D.** Representative Western blots and corresponding quantifications are shown. N1E-115 cells were co-transfected with eGFP-Tau[R406W] mutant and (HA)5-HT7R together with either pcDNA (ctrl), scrambled shRNA (scr-shRNA) or specific shRNA directed against the G$\alpha$12 subunit of heterotrimeric G-Protein ($G_{12}$-shRNA, see also **Fig. 8).** Membranes were probed with phospho-specific tau antibody AT270 **(C)** or 5A6 **(D)** for assessing total levels of tau. Results were normalized to GAPDH expression and are

represented as mean + SD (N=3). ** indicates statistical significance of p<0.01, *** p<0.001 (one-way ANOVA, followed by Bonferroni's post hoc test ).

**Fig. 2: 5-HT7R-induced increase in phosphorylation and accumulation of tau is mediated by CDK5, but not GSK3β.**

**A and B.** Representative Western blots and quantifications are shown. N1E-115 cells co-transfected with eGFP-Tau [R406W] mutant and (HA)5-HT7R were treated overnight with 20 μM CDK5 inhibitor Roscovitine or 10 μM GSK3 inhibitor SB-216763. Membranes were probed with phospho-specific tau antibody AT270 **(A)** or 5A6 **(B)** for assessing total levels of tau. Results were normalized to GAPDH expression and represented as mean + SD (N=4). **C and D.** N1E-115 cells were co-transfected with plasmids encoding (HA)5-HT7R and wild-type CDK5-mCherry (CDK5) or dominant negative mutant CDK5-T33-mCherry (CDK5-DN). AT270 antibody was used to detect tau phosphorylation **(C)** and 5A6 antibody to assess total tau levels **(D).** Results were normalized to the GAPDH expression and represented as mean + SD (N=6). ** indicates statistical significance of p<0.01, *** p<0.001 (one-way ANOVA, followed by Bonferroni's post hoc test).

**Fig. 3: 5-HT7R directly interacts with CDK5.**

**A.** N1E-115 cells overexpressing either (HA)5-HT7R or CDK5-mCherry alone, a mixture of cells individually expressing (HA)5-HT7R or CDK5-mCherry (mix) as well as cells co-expressing (HA)5-HT7R and CDK5-mCherry (co-trans) were subjected to immunoprecipitation (IP) with anti-mCherry antibody followed by Western blotting with either anti-mCherry or anti-HA antibody. Input (lysates w/o IP) was assessed in parallel with the same antibodies. Representative Western blot is shown (N=4). **B and C.** Representative Total Internal Reflection Fluorescence (TIRF) images and corresponding quantification showing apparent FRET efficiencies $EF_{DA}$ between CDK5-eCFP and 5-HT7R-eYFP in transfected N1E-115 cells. Cells were treated with (+SB) or without 100 nM SB-269970 (-SB) overnight. Cells co-transfected with cytosolic eCFP and 5-HT7R-eYFP were used as a negative control (neg), while cells co-expressing 5-HT1AR-eCFP and 5-HT7R-eYFP served as positive control (pos; N=3; total analyzed cells $14 \leq n \leq 32$). ** indicates statistical significance of p<0.01, *** p<0.001 (one-way ANOVA, followed by Bonferroni's post hoc test).

**Fig. 4: 5-HT7R induces tau aggregation and tangle formation.**

**A.** Representative Western blot with lysates of N1E-115 cells co-transfected with eGFP-Tau[R406W] mutant and (HA) 5-HT7R after sarkosyl fractionation and corresponding quantification. Cells were pre-treated with 100 nM SB-269970 overnight or left untreated, following application of 20 μM 5-HT for 1 h. HRP-conjugated anti-GFP antibody was used to detect total tau levels in the sarkosyl-unsoluble fraction and detected intensities were normalized to GAPDH expression in the soluble fraction. Water-treated and eGFP-Tau[R406W] mutant transfected cells were set 100%. Results are represented as mean + SD (N=3). *** indicates statistical significance of p<0.001 (one-way ANOVA, followed by Bonferroni's post hoc test). **B.** Quantification of tau tangles in N1E-115 cells. Cells were transfected with the indicated plasmids and treated overnight with 100 nM SB-269970 or 20 μM Roscovitine. The fraction of cells with eGFP-positive filamentous tangles was calculated from all eGFP-positive cells in a confined area in 7 independent experiments (N=7; total analyzed cells $2343 \leq n \leq 2799$). Experiments were done in a double-blind fashion. See also **Figure 9** showing representative cells with and without tangles. ** indicates statistical significance of p<0.01, *** p<0.001 (one-way ANOVA). **C.** Representative maximum intensity projections of N1E-115 cells co-transfected with eGFP-Tau[R406W] mutant and (HA)5-HT7R with and without CDK5-DN, treated overnight with 100 nM SB-269970 or 20 μM Roscovitine and stained with phospho-specific tau antibody AT8. Corresponding representative fluorescence intensity profiles display localization of eGFP-Tau[R406W] mutant (eGFP, green) and phosphorylated tau (AT8, red) within the cell.

**Fig. 5: Tau phosphorylation in cortical neurons is regulated via 5-HT7R and CDK5. A and B.** Quantifications and representative Western blots of lysates from cultured primary cortical neurons of wild type (WT) or 5-HT7R-KO mice (DIV 4). AT270 antibody was used to detect phosphorylation of tau **(A)** and tau-5 was used for determination of endogenous total protein levels **(B).** Results were normalized to GAPDH expression and represented as mean + SD. * indicates statistical significance of p<0.05, n.s. not statistically significant (N=4, student's t-test). **C and D.** Representative Western blots with lysates of cultured primary cortical neurons of WT mice (DIV 4) after treatment with 100 nM SB-269970, 20 μM Roscovitine or $H_20$ overnight. AT270 antibody was used to detect phosphorylation of tau **(C)** and tau-5 was used for determination of endogenous total protein levels **(D).** Results were normalized to GAPDH expression and represented as mean + SD. * indicates statistical significance of p<0.05 (N=3, one-way ANOVA, followed by Bonferroni's post hoc test). **E.** Primary cortical neurons of wild type mice (DIV 14) were stained with antibodies against 5-HT7R, CDK5 and VGLUT. White box is magnified in the lower panels. Arrow heads indicate co-localization. **F.** Cortical slices of P6 wild type mice were stained with antibodies against 5-HT7R and CDK5. Arrow

heads indicate co-localization. **G.** Co-immunoprecipitation experiments for endogenous CDK5 and 5-HT7R. Cortex homogenates were prepared from P6 wild type mice and subjected to immunoprecipitation (IP) with anti-5-HT7R antibody or with rabbit IgG, followed by Western blotting using either anti-5-HT7R or anti-CDK5 antibody. Representative Western blot is shown (N=4).

**Fig. 6: Increased tau tangle formation in eGFP-Tau[R406W]-infected primary cortical neurons correlates with increased neuronal apoptosis.**

**A.** Representative images of DIV 14 primary cortical neurons isolated form WT and 5-HT7R-KO mice infected at DIV10 with AAV constructs encoding eGFP-Tau[R406W] and treated with $H_2O$, 100 nM SB-269970 or 20 μM Roscovitine for 3 days. White boxes and its corresponding magnifications show representative neurons with and without tau tangles. Arrow heads indicate cells that were counted as tangle-positive. **B.** Quantification of tau tangles. The number of tangle-positive neurons was counted in a confined area in 6 independent experiments and is represented as a fraction of total number of infected neurons. * indicates statistical significance of $p<0.05$; ** $p<0.01$ (N=6; total analyzed cells $355 \leq n \leq 897$, one-way ANOVA, followed by Dunnett's *post hoc* test). **C.** Representative images of DIV14 primary cortical WT and 5-HT7R-KO neurons infected at DIV 10 with AAV-eGFP-Tau [R406W], treated with 100 nM SB-269970 or 20 μM Roscovitine for 3 days and stained with phospho-specific tau antibody AT8. **D.** Representative images of DIV14 primary cortical WT neurons infected at DIV10 either with AAV constructs encoding eGFP (white) or eGFP-Tau[R406W] (green). After treatment either with $H_3O$, 100 nM SB-269970 or 20 μM Roscovitine for 3 days, apoptosis assay was performed. Fluorescence from cleaved substrate of active caspase3/7 indicating apoptosis is shown. **E.** Quantification of apoptosis. Apoptotic cells showing caspase3/7 activity were counted in a confined area in 6 independent experiments and are represented as a fraction of total number of infected neurons. Data are shown as mean + SD. ** indicates statistical significance of $p<0.01$ (N=6; total analyzed cells $123 \geq n \leq 409$, one-way ANOVA, followed by Bonferroni's post hoc test ).

**Fig. 7: Silencing of 5-HT7R reduces tau pathology *in vivo.***

**A.** Scheme showing experimental design: different AAVs (see **Fig. 10A)** were injected stereotactically into the pre-frontal cortex (PFC) of WT mice. After one month, various experiments were performed. **B.** Normalized mean slopes and representative examples of field excitatory postsynaptic potentials (fEPSPs) in brain slices from WT mice that were injected with AAV-eGFP, AAV-eGFP-Tau[R406W], AAV-eGFP-Tau[R406W] + AAV-scramble-shRNA, or AAV-eGFP-Tau[R406W] + AAV-5-HT7R-shRNA (see also **Fig. 10B).** The arrow denotes the time point at which five trains of TBS were applied. The insets represent averages of 30 fEPSPs recorded during 0-10 min before TBS (black, baseline) and 50-60 min after TBS (gray), respectively, in each group. **C.** Quantification of mean LTP levels recorded 50-60 min after TBS delivery in all groups. Data are presented as mean + SEM from the recorded slices: 8 slices from 6 mice for AAV-eGFP, 8 slices from 6 mice for AAV-eGFP-Tau[R406W], 7 slices from 5 mice for AAV-eGFP-Tau[R406W] + AAV-scramble-shRNA, and 8 slices from 6 mice for AAV-eGFP-Tau[R406W] + AAV-5-HT7R-shRNA. ** indicates statistical significance of $p<0.01$ (unpaired Student's *t* test). **D.** Staining of pre-frontal cortical slices of mice after AAV injection with phospho-specific tau antibody T205 (see also **Fig. 10). E.** Recency test with mice one month after injection of the indicated AAVs in the PFC. Quantification of discrimination and exploration time spent with less recent (L) and recent (R) object. Data are presented as mean + SEM ($10 \leq N \leq 12$). * indicates statistical significance of $p<0.05$, ** $p<0.01$ and ** $p<0.001$ (one-way ANOVA).

**Fig 8: Efficiency of $G_{12}$-shRNA.**

**A.** Quantification of relative $G_{12}$ mRNA levels by quantitative Real-Time PCR in N1E-115 cells transfected with 3 different shRNAs against $G_{12}$. Cells transfected with src-shRNA were set to 100%. Data are represented as mean + SD (N=5). *** indicates statistical significance of $p<0.001$ (one-way ANOVA, followed by Dunnett's *post hoc* test). **B.** Western blot and quantification of $G_{12}$ protein levels in N1E-115 cells transfected scr-shRNA or $G_{12}$-shRNA#2. Signals were normalized to GAPDH expression and cells with scr-shRNA were set to 100%. Data are represented as mean + SD (N=6). *** indicates statistical significance of $p<0.01$ (Student's t-test).

**Fig. 9: Tau tangles in N1E-115 cells.**

Live cell imaging of N1E-115 cells transfected with eGFP-Tau[R406W]. Representative cells with (left panel) and without tau tangles (right panel) are shown.

**Fig. 10: Validation of tau pathology *in vivo.***

**A.** Scheme of AAV constructs used for stereotactic injections in mice prefrontal cortex. **B.** Quantification of relative 5-HT7R mRNA levels by quantitative Real-Time PCR in primary cortical neurons infected with AAVs endcoding 2 different shRNAs against 5-HT7R. Cells infected with AAV-src-shRNA were set to 100%. Data are represented as mean + SD (N=3). *** indicates statistical significance of $p<0.001$ (one-way ANOVA, followed by Dunnett's *post hoc*

test). **C.** Staining of pre-frontal slices after injection of AAV-eGFP-Tau[R406W] (eGFP, green) with phospho-specific tau antibody T205 (ptau, red). Right panel shows secondary antibody control. **D and E.** Open field test with mice one month after injection of the indicated AAVs. No significant differences (n.s.) in the distance travelled **(D)** and the time spent in the center (C) or the periphery (P) of the arena **(E)** were observed. Data are presented as mean + SEM ($10 \leq N \leq 12$, one-way ANOVA).

**Fig 11: 5-HT7R inverse agonists decrease 5-HT7R-induced Tau aggregation in HEK293 Tau-BiFCs.**
**A.** Scheme showing experimental design. **B.** Quantification of tau-BiFC fluorescence intensity in pcDNA or (HA)5-HT7R-transfected HEK293 Tau-BiFC cells upon treatment with DMSO or 50 μM SB-269970, Amisulpride, Clozapine, Lurasidone, Miaserine or Tiapride for 24 h. Signals are normalized to DMSO-treated pcDNA-transfected cells. Data are represented as means + SDs (N = 3). *** indicates statistical significance of $p<0.001$, n.s. not statistically different (one-way ANOVA, followed by Turkey's *post hoc* test). **C.** Representative maximum intensity projections of HEK293 Tau-BiFC cells transfected with control vector (mCerulean, blue) or 5-HT7R (CFP-5-HT7R, blue) upon the indicated treatment. Tangles of Venus-Tau (VN-Tau, yellow) presented in 5-HT7R-transfected cells disappeared after treatment with SB-269970 and amisulpride. **D.** Representative Western blot and quantification of HEK293 Tau-BiFC transfected with pcDNA or (HA)5-HT7R upon treatment with DMSO, SB-269970, Amisulpride, Clozapine, Lurasidone, Miaserine, Vortioxetine or Tiapride for 24 h. Membranes were probed with total Tau (Abcam) and GAPDH antibody (Millipore). Signals are normalized to DMSO-treated pcDNA-transfected cells. Data are represented as means + SDs (N = 3). ** indicates statistical significance of $p<0.01$, *** $p<0.001$, n.s. not statistically different (one-way ANOVA, followed by Turkey's *post hoc* test). **E.** Dose-response curve for inhibition of Tau aggregation by Amisulpride. HEK293 Tau-BiFC cells were transfected with (HA)5-HT7R and treated with the indicated concentration of Amisulpride.

**Fig 12: 5-HT7R-induced Tau tangle formation and apoptosis in eGFP-Tau[R406W]-infected primary cortical neurons is reduced after treatment with Amisulpride.**
**A.** Scheme showing experimental design. Primary cortical neurons were infected with AAV-eGFP-Tau[R406W] at DIV 10 and treated with $H_2O$, 100 nM SB-269970, 50 μM Amisulpride, 1 μM Vortioxetine or 50 μM Tiapride for three days. Neurons were analyzed at DIV 13. **B.** Representative images of neurons infected with AAV-eGFP-Tau[R406W] (green) and treated as indicated. Arrowheads indicate cells that were counted as tangle-positive. **C.** Quantification of Tau tangles. The number of tangle-positive neurons was counted in a confined area in at least four independent experiments and is represented as a fraction of the total number of infected neurons. Data are shown as mean + SEM. **** indicates statistical significance of $p<0.0001$, n.s. not statistically different ($4 \leq N \leq 8$; one-way ANOVA, Dunnett's *post hoc* test). **D.** Representative Western blot with neuronal lysates after sarkosyl fractionation. Tau signals in soluble, sarkosyl-soluble and sarkosyl-insoluble fractions (i.e. Tau-tangles) were detected by HRP-GFP antibody. GAPDH expression was detected in a soluble fraction. **E.** Quantification of apoptosis. Apoptotic cells showing caspase3/7 activity were counted in a confined area in at least four independent experiments and are represented as a fraction of total number of infected neurons. Data are shown as means + SEMs. **** indicates statistical significance of $p<0.0001$; n.s., not statistically different ($4 \leq N \leq 8$; one-way ANOVA, Dunnett's *post hoc* test).

**Fig 13: Amisulpride injections ameliorates Tau[R406W]-induced Tau hyperphosphorylation and memory impairments.**
**A.** Scheme showing experimental design: AAV-eGFP-Tau[R406W] was injected stereotactically into the prefrontal cortex (PFC) of WT mice. After 3 weeks of recovery time, animals were intraperitoneally injected with either Vehicle or Amisulpride once daily for 16 days. B. Staining of prefrontal cortical slices of mice after AAV injection and treatment with phospho-specific Tau antibody T205. Representative overview image and magnification are shown. **C.** Quantification of the mean intensities of eGFP-Tau and pTau over the whole slice and calculation of the pTau/Tau ratio. Data are shown as mean + SEM (N=5). * indicates statistical significance of $p<0.05$ (one-tailed unpaired t-test). **D.** Results of test for temporal order recognition memory (recency test). Quantification of exploration time spent with less recent (L) and recent (R) object and discrimination index. Data are presented as mean + SEM ($9 \geq N \geq 7$). * indicates statistical significance of $p<0.05$, ** $p<0.01$; n.s., not statistically different (paired t-test and Wilcoxon signed rank test were applied to analyze exploration time, one-way ANOVA was applied to analyze the discrimination ratio).

## DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments described throughout the specification should not be construed to limit the present invention to only the explicitly described embodiments. This description should be understood to support and encompass embodiments which combine the explicitly described embodiments with any number of the disclosed and/or preferred elements. Furthermore, any permutations and combinations of all elements described herein should be considered disclosed by the description of the present application unless the context indicates otherwise.

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. When used herein the term "comprising" can be substituted with the term "containing" or "including" or sometimes when used herein with the term "having". When used herein "consisting of" excludes any element, step, or ingredient not specified.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Unless otherwise indicated, the term "at least" preceding a series of elements is to be understood to refer to every element in the series. The term "at least one" refers to one or more such as two, three, four, five, six, seven, eight, nine, ten and more. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

When used herein "consisting of" excludes any element, step, or ingredient not specified in the claim element. When used herein, "consisting essentially of" does not exclude materials or steps that do not materially affect the basic and novel characteristics of the claim.

The term "including" means "including but not limited to". "Including" and "including but not limited to" are used interchangeably.

The term "about" means plus or minus 10%, preferably plus or minus 5%, more preferably plur or minus 2%, most preferably plus or minus 1%.

Throughout the description and claims of this specification, the singular encompasses the plural unless the context otherwise requires. In particular, where the indefinite article is used, the specification is to be understood as contemplating plurality as well as singularity, unless the context requires otherwise.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

A better understanding of the present invention and of its advantages will be gained from the examples, offered for illustrative purposes only. The examples are not intended to limit the scope of the present invention in any way.

*Compound*

In general, serotonin receptors (5-HTRs) are divided into seven groups based on their structure, function, cellular response and distribution. They are mainly located in the hippocampus and prefrontal cortex of the brain (Strac et al., Transi Neurosci. 2016; 7(1): 35-492). In particular, the 5-HT7 receptor is coupled to two heterotrimeric G-proteins, G$\alpha$S and G$\alpha$12 to facilitate formation of cyclic adenosine monophosphate (cAMP) by activating adenylyl cyclase and to activate small GTPases of the Rho family. In detail, when the 5-HT7 receptor is activated by serotonin, it sets off a cascade of events starting with release of the stimulatory G protein G$\alpha$s from the GPCR complex. G$\alpha$s in turn activates adenylate cyclase

which increases intracellular levels of the second messenger cAMP.

In the present invention, it was found that the 5-HT7R activity induces tau-related pathomechanisms, including tau phosphorylation, tangle formation and neurotoxicity in a recombinant system as well as in primary cortical neurons and acute brain slices from mice.

Thus, by the present invention the importance of 5-HT7R signaling in tau-related neurodegeneration has been demonstrated. 5-HT7R-induced tau phosphorylation may be sufficient to promote the formation of sacrosyl-insoluble tau aggregates and obvious tangle-like structure within the cytoplasm. It was further shown that it may easily be envisioned that increased tau phosphorylation and tangle formation induced by 5-HT7R activity might reduce the capacity to degrade tau protein within cells. The increased number of tangle-like structures might additionally act as a "seed" for unphosphorylated, soluble tau species. Not only phosphorylated but also total tau levels may therefore increase.

Further, CDK5 as a critical mediator being responsible for 5-HT7R signaling upon tau has also been identified. Clear evidence has been found for an interaction of CDK5 with 5-HT7R through co-localization, FRETand co-precipitation. Genetic inhibition of CDK5, but not GSK3β activity, may abolish 5-HT7R elicited effect on tau phosphorylation in cells, demonstrating that Gα12 activation is not necessary for 5-HT7R-induced tau phosphorylation, thereby also speaking against a Gα12-mediated GSK3β activation. The same may apply to GαS, which may also not influence tau phosphorylation. An involvement of CDK5 activity in 5-HT7R signaling may clearly be emphasized.

Thus, a significant contribution of the 5-HT7R with regard to tau pathology has been demonstrated, thereby identifying a new putative molecular target from the family of serotonin receptors and, by now, the only one influencing tau deposition.

Most importantly, it has been found within the present invention that by blocking the basal activity of said 5-HT7R with an antagonist, hyperphosphorylation of the tau protein is prevented and through that mechanism the formation of tau-tangles in nerve cells is inhibited.

In particular, the 5-HT7 receptor antagonist may be used in preventing or treating a tauopathy by inhibiting both the receptor-induced phosphorylation and thus the accumulation of tau proteins.

Thereby, said 5-HT7 receptor antagonist of the present invention is able to halt the progression of a tauopathy or inhibit the development of a tauopathy at all and does not only treat the symptoms which are caused by said disease (e.g. cognitive functions, psychosis and behavioral problems).

In general, a tauopathy belongs to a class of neurodegenerative diseases associated with the pathological aggregation of tau protein in neurofibrillary or gliofibrillary tangles in the human brain. The tau protein undergoes several post-translational modifications, including acetylation, myristoylation with phosphorylation being the major modification of tau and its cellular functions. In tauopathies, tau aggregates are found to be extensively phosphorylated at several serine, threonine or tyrosine residues. Hyperphosphorylation of tau decreases its ability to bind to microtubules thereby disturbing transport of cargos and induces the accumulation of tau within the cell.

Thus, the term tauopathy encompasses both the loss-of-function effects on the microtubules and the gain-of-function effects of the toxic tau species. In detail, the consequences of tau hyperphosphorylation include not only loss of its function to bind and stabilize microtubule but also a gain of neurotoxic properties by tau aggregation into neurofibrillary tangles.

In this context and as used throughout the entire description, the term "prevent / preventing a tauopathy" refers to completely inhibiting the development of a tauopathy. By using a 5-HT7 receptor antagonist or a pharmaceutical composition comprising the 5-HT7 receptor antagonist, such antagonist or such pharmaceutical composition inhibits receptor-induced (hyper)-phosphorylation of a microtubule-associated protein known as tau protein, thereby preventing that the tau protein dissociates from microtubules and forms aggregates in an insoluble form, which are called tangles. Thus, the 5-HT7 receptor antagonist or the pharmaceutical composition comprising the 5-HT7 receptor antagonist inhibits the accumulation of phosphorylated tau proteins, thereby preventing that a tauopathy might occur anyway.

The term "treat / treating a tauopathy" as used throughout the entire description refers to halting the progression of a tauopathy in a subject which has been suffering from a tauopathy when the treatment with a 5-HT7 receptor antagonist or with a pharmaceutical composition comprising the 5-HT7 receptor antagonist has been initiated. Thereby, the 5-HT7 receptor antagonist or the pharmaceutical composition comprising the 5-HT7 receptor antagonist of the present invention prevents further receptor-induced (hyper)-phosphorylation of a tau protein, thereby preventing that the protein dissociates from microtubules and forms aggregates in an insoluble form, which are called tangles. Thus, the 5-HT7 receptor antagonist or the pharmaceutical composition comprising the 5-HT7 receptor antagonist reduces the accumulation of phosphorylated tau proteins, thereby halting the progression of a tauopathy.

There are a number of specific tauopathies, each of which vary by the distribution and morphological appearances of the protein-containing inclusions, as well as the relative burden of pathology affecting neurons and neuronal processes versus glial and glial processes. Tauopathies may present either with dementia, parkinsonism, or both.

In a preferred embodiment tauopathy is a dementia-associated tauopathy. Dementia-associated tauopathy refers to a tauopathy, whereby the patient suffering from said tauopathy also suffers from dementia. Dementia is a broad category of brain diseases that causes a long-term and often gradual decrease of the cognitive and memory functions that

is great enough to affect a person's daily functioning.

Dementia-associated tauopathy may be selected from the group consisting of Alzheimer's disease, frontotemporal dementia, primary age-related tauopathy (PART), chronic traumatic encephalopathy, progressive supranuclear palsy (PSP), corticobasal degeneration, dementia with Lewy Bodies (DLB), frontotemporal dementia and parkinsonism linked to chromosome 17 (FTDP-17), argyrophilic grain disease (AGD), Huntington disease, glial globular tauopathy. In a preferred embodiment dementia-associated tauopathy is Alzheimer's disease or frontotemporal dementia, more preferably Alzheimer's disease.

Alzheimer's disease accounts for 50% to 70% of all cases of dementia, being characterized by short-term memory loss and word-finding difficulties. In Alzheimer's disease, it is well established that filamentous tau protein deposits form within nerve cells that degenerate and that a good correlation exists between the number of tau deposits and the presence of dementia (Goedert et al, 1997, The Neuroscientist. 3, 131-141; Braak and Braak, 1991, Acta Neuropathologica. 82, 239-259; Arriagada et al, 1992, Neurology, 42(3) 631).

Therefore, in a preferred embodiment the present invention may comprise a 5-HT7R antagonist for use in treating or preventing a dementia-associated tauopathy, wherein said dementia-associated tauopathy is selected from the group consisting of Alzheimer's disease, frontotemporal dementia, primary age-related tauopathy (PART), chronic traumatic encephalopathy, progressive supranuclear palsy (PSP), corticobasal degeneration, dementia with Lewy Bodies (DLB), frontotemporal dementia and parkinsonism linked to chromosome 17 (FTDP-17), argyrophilic grain disease (AGD), Huntington disease, glial globular tauopathy, preferably Alzheimer's disease or frontotemporal dementia, more preferably Alzheimer's disease.

In another preferred embodiment tauopathy may be selected from the group consisting of Alzheimer's disease, frontotemporal dementia, primary age-related tauopathy (PART), chronic traumatic encephalopathy, progressive supranuclear palsy (PSP), corticobasal degeneration, dementia with Lewy Bodies (DLB), frontotemporal dementia and parkinsonism linked to chromosome 17 (FTDP-17), argyrophilic grain disease (AGD), Huntington disease, glial globular tauopathy, amyotrophic lateral sclerosis (ALS), Parkinson's disease, spinal muscular atrophy (SMA), cerebral amyloid angiopathy (CAA), preferably Alzheimer's disease or frontotemporal dementia, more preferably Alzheimer's disease.

Thus, the present invention may also comprise a 5-HT7R antagonist for use in treating or preventing a tauopathy, wherein said tauopathy is selected from the group consisting of Alzheimer's disease, frontotemporal dementia, primary age-related tauopathy (PART), chronic traumatic encephalopathy, progressive supranuclear palsy (PSP), corticobasal degeneration, dementia with Lewy Bodies (DLB), frontotemporal dementia and parkinsonism linked to chromosome 17 (FTDP-17), argyrophilic grain disease (AGD), Huntington disease, glial globular tauopathy, amyotrophic lateral sclerosis (ALS), Parkinson's disease, spinal muscular atrophy (SMA), cerebral amyloid angiopathy (CAA), preferably Alzheimer's disease or frontotemporal dementia, more preferably Alzheimer's disease.

The term "antagonist" as used herein refers to two classes of antagonists. The first class refers to the neutral antagonists, which bind the receptor and have no intrinsic activity but will block the activity of agonists or inverse agonists. In this context, an agonist mimics the effects of the endogenous ligand, which is serotonin at the 5HT7-receptor, thus activating the receptor to produce biological response. Thus, in the context of the present invention at the 5-HT7 receptor, the cAMP level may increase.

The second class of antagonists refers to inverse agonists., which inhibit the constitutive (basal) activity of the receptor, producing functional effects opposite to those of agonists. Thus, in the context of the present invention at the 5-HT7 receptor, cAMP level may decrease. An inverse agonist may bind to the same receptor-binding site as an agonist.

In a preferred embodiment the 5-HT7 receptor antagonist is an antagonist with inverse agonist properties, thus acting as an inverse agonist. In another preferred embodiment, said inverse agonist targets and blocks the basal (constitutive) activity of 5-HT7R.

In 2004 after detailed pharmacological analysis, SB-269970 was declared as a 5-HT7R antagonist with full inverse agonist properties (Mahe et al. 2004, Eur J Pharmacol, 495(2-3):97-102). The same applies *inter alia* to Clozapine, for which also evidence for 5-HT7R inverse agonistic effects has been presented (Thomas et al., 1998, British Journal of Pharmacology 124, 1300-1306).

The term "alkyl" refers to a monoradical of a saturated straight or branched hydrocarbon. Preferably, the alkyl group comprises from 1 to 6 carbon atoms. For example the term "($C_1$-$C_6$) alkyl" means that the respective alkyl group may comprise 1, 2, 3, 4, 5, or 6 carbon atoms. Exemplary alkyl groups include methyl, ethyl, propyl, iso-propyl, butyl (e.g.. n-butyl, iso-butyl, tert-butyl), pentyl (e.g., n-pentyl, iso-pentyl, sec-pentyl, neo-pentyl), 1,2-dimethyl-propyl, isoamyl, n-hexyl, iso-hexyl, sec-hexyl, 2,2-dimethylbutyl, n-heptyl, and the like. A "substituted alkyl" means that one or more (such as 1 to the maximum number of hydrogen atoms bound to an alkyl group, e.g., 1, 2, 3, 4, 5, 6, 7, 8, 9, or up to 10, such as between 1 to 5, 1 to 4, or 1 to 3, or 1 or 2) hydrogen atoms of the alkyl group are replaced with a substituent other than hydrogen (when more than one hydrogen atom is replaced the substituents may be the same or different). Preferably, the substituent other than hydrogen is a $1^{st}$ level substituent, a $2^{nd}$ level substituent, or a $3^{rd}$ level substituent as specified herein, such as halogen or optionally substituted aryl. Examples of a substituted alkyl include trifluoromethyl, 2,2,2-trichloroethyl, arylalkyl (also called "aralkyl", e.g., benzyl, chloro(phenyl)methyl, 4-methylphenylmethyl, (2,4-dimethylphenyl)methyl, o-fluorophenylmethyl, 2-phenylpropyl, 2-, 3-, or 4-carboxyphenylalkyl), or heteroarylalkyl (also called "heteroaralkyl").

The term "cycloalkyl" refers to a monoradical of a saturated, non-aromatic cyclical hydrocarbon. In one embodiment, the cycloalkyl group has 1, 2, or more (preferably 1 or 2) double bonds. Preferably, the alkyl group comprises from 3 to 6 carbon atoms. "For example the term "($C_3$-$C_6$)cycloalkyl" means that the respective alkyl group may comprise 3, 4, 5, or 6 carbon atoms. Exemplary cycloalkyl groups include cyclopropyl, cyclobutyl, cyclopentyl, cyclohexyl and the like. A "substituted cycloalkyl" means that one or more (such as 1 to the maximum number of hydrogen atoms bound to an alkyl group, e.g., 1, 2, 3, 4, 5, 6, 7, 8, 9, or up to 10, such as between 1 to 5, 1 to 4, or 1 to 3, or 1 or 2) hydrogen atoms of the cycloalkyl group are replaced with a substituent other than hydrogen (when more than one hydrogen atom is replaced the substituents may be the same or different). "-($C_3$-$C_8$)cycloalkyl-($C_1$-$C_6$)alkyl" means ($C_3$-$C_8$)cycloalkyl substituted with ($C_1$-$C_6$)alkyl.

The term "alkenyl" refers to a monoradical of an unsaturated straight or branched hydrocarbon having at least one carbon-carbon double bond. For example, the term "($C_2$-$C_6$) alkenyl" means that the respective alkenyl group may comprise 1, 2, 3, 4, 5, or 6 carbon atoms. Generally, the maximum number of carbon-carbon double bonds in the alkenyl group can be equal to the integer which is calculated by dividing the number of carbon atoms in the alkenyl group by 2 and, if the number of carbon atoms in the alkenyl group is uneven, rounding the result of the division down to the next integer. For example, for an alkenyl group having 9 carbon atoms, the maximum number of carbon-carbon double bonds is 4. Preferably, the alkenyl group has 1 carbon-carbon double bond. Preferably, the alkenyl group comprises from 2 to 6 carbon atoms, such as from 2 to 4 or 2 carbon atoms, i.e., 2, 3, 4, 5, or 6 carbon atoms, more preferably 2 to 4 carbon atoms. Thus, in a preferred embodiment, the alkenyl group comprises from 2 to 6 carbon atoms and 1 or 2, carbon-carbon double bonds, more preferably it comprises 2 to 4 carbon atoms and 1 carbon-carbon double bond. The carbon-carbon double bond(s) may be in cis (Z) or trans (E) configuration. Exemplary alkenyl groups include ethenyl (i.e., vinyl), 1-propenyl, 2-propenyl (i.e., allyl), 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1, and the like. If an alkenyl group is attached to a nitrogen atom, the double bond cannot be alpha to the nitrogen atom.

The term "aryl" or "aromatic ring" refers to a monoradical of an aromatic cyclic hydrocarbon. Preferably, the aryl group contains 6 to 10 (e.g., 6 to 10, such as 6, or 10) carbon atoms which can be arranged in one ring (e.g., phenyl) or two or more condensed rings (e.g., naphthyl). For example the term "($C_6$-$C_{10}$)aryl" means that the respective aryl group may comprise 6 to 10 carbon atoms. Exemplary aryl groups include, phenyl, indenyl, naphthyl, azulenyl, fluorenyl, anthryl, and phenanthryl. Preferably, "aryl" refers to a monocyclic ring containing 6 carbon atoms or an aromatic bicyclic ring system containing 10 carbon atoms. Preferred examples are phenyl and naphthyl. A "substituted aryl" means that one or more (such as 1 to the maximum number of hydrogen atoms bound to an aryl group, e.g., 1, 2, 3, 4, 5, 6, 7, 8, 9, or up to 10, such as between 1 to 5, 1 to 4, or 1 to 3, or 1 or 2) hydrogen atoms of the aryl group are replaced with a substituent other than hydrogen (when more than one hydrogen atom is replaced the substituents may be the same or different).

The term "heteroaryl" or "heteroaromatic ring" means an aryl group as defined above in which one or more carbon atoms in the aryl group are replaced by heteroatoms of O, S, or N. Preferably, heteroaryl refers to a five or six-membered aromatic monocyclic ring wherein 1, 2, or 3 carbon atoms are replaced by the same or different heteroatoms of O, N, or S. For example the term "($C_5$-$C_{10}$)heteroaryl" means that the heteroaryl group is based on an aryl group comprising 5 to 10 carbon atoms wherein one or more carbon atoms are replaced by heteroatoms of O, S, or N. Alternatively, it means an aromatic bicyclic or tricyclic ring system wherein 1, 2, 3, 4, or 5 carbon atoms are replaced with the same or different heteroatoms of O, N, or S. Preferably, in each ring of the heteroaryl group the maximum number of O atoms is 1, the maximum number of S atoms is 1, and the maximum total number of O and S atoms is 2. Exemplary heteroaryl groups include furanyl, thienyl, oxazolyl, isoxazolyl, oxadiazolyl, pyrrolyl, imidazolyl, pyrazolyl, triazolyl, tetrazolyl, thiazolyl, isothiazolyl, thiadiazolyl, pyridyl, pyrimidinyl, pyrazinyl, triazinyl, benzofuranyl, indolyl, isoindolyl, benzothienyl, 1H-indazolyl, benzimidazolyl, benzoxazolyl, indoxazinyl, benzisoxazolyl, benzothiazolyl, benzisothiazolyl, benzotriazolyl, quinolinyl, isoquinolinyl, benzodiazinyl, quinoxalinyl, quinazolinyl, benzotriazinyl, pyridazinyl, phenoxazinyl, thiazolopyridinyl, pyrrolothiazolyl, phenothiazinyl, isobenzofuranyl, chromenyl, xanthenyl, pyrrolizinyl, indolizinyl, indazolyl, purinyl, quinolizinyl, phthalazinyl, naphthyridinyl, cinnolinyl, pteridinyl, carbazolyl, phenanthridinyl, acridinyl, perimidinyl, phenanthrolinyl, and phenazinyl. Exemplary 5- or 6-membered heteroaryl groups include furanyl, thienyl, oxazolyl, isoxazolyl, oxadiazolyl, pyrrolyl, imidazolyl (e.g., 2-imidazolyl), pyrazolyl, triazolyl, tetrazolyl,thiazolyl, isothiazolyl, thiadiazolyl, pyridyl (e.g., 4-pyridyl), pyrimidinyl, pyrazinyl, triazinyl, and pyridazinyl. A "substituted heteroaryl" means that one or more (such as 1 to the maximum number of hydrogen atoms bound to a heteroaryl group, e.g., 1, 2, 3, 4, 5, 6, 7, 8, 9, or up to 10, such as between 1 to 5, 1 to 4, or 1 to 3, or 1 or 2) hydrogen atoms of the heteroaryl group are replaced with a substituent other than hydrogen (when more than one hydrogen atom is replaced the substituents may be the same or different).

The term "halogen" or "halo" means fluoro, chloro, bromo, or iodo. The term "hydroxy" means OH. The term "cyano" means the group -CN. The term "isocyano" means the group -NC.

As used herein and throughout the entire description, the term "optional" or "optionally" as used herein means that the subsequently described event, circumstance or condition may or may not occur, and that the description includes instances where said event, circumstance, or condition occurs and instances in which it does not occur.

The 5-HT7 receptor antagonist has structure according to at least one of the general formula (I) to (VI), or is a pharmaceutically acceptable salt, enantiomer, diastereomer, racemic mixture, crystalline form, amorphous, unsolved form or solvate of the general formula (I), (II), (III), (IV), (V) or (VI) as follows.

Formula (I):

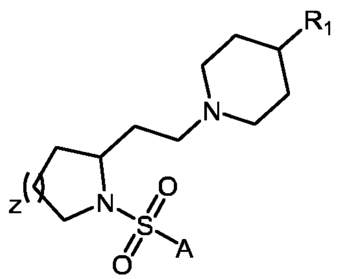

(I)

wherein in formula (I)

$R_1$ is $-(C_1-C_6)$alkyl, preferably methyl, $(C_6-C_{10})$aryl, $-O(C_6-C_{10})$aryl, or $(C_5-C_{10})$heteroaryl,

which are optionally substituted with one or more substituents independently selected from the group consisting of: halogen, preferably -Cl, or $(C_1-C_6)$Alkyl;

A is $(C_6-C_{10})$aryl, or $(C_5-C_{10})$heteroaryl,

which are optionally substituted with one or more substituents independently selected from the group consisting of: halogen, -OH, $(C_1-C_6)$alkyl, or $-O(C_1-C_6)$alkyl, preferably the at least one substituent is a meta-substituent;

z is 1 .

Formula (II):

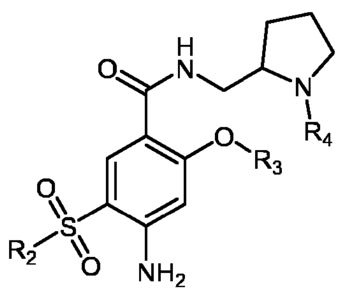

(II)

wherein in formula (II)

$R_2$ and $R_3$ are independently selected from hydrogen, $(C_1-C_6)$alkyl, preferably methyl or ethyl, most preferably ethyl; $R_4$ is hydrogen, $(C_1-C_6)$alkyl, preferably methyl or ethyl, most preferably ethyl, or $(C_2-C_6)$alkenyl.

Formula (III):

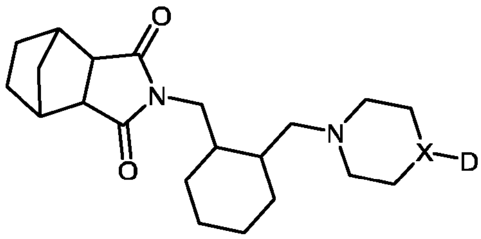

(III)

wherein in formula (III)

X is N or CH;

D is hydrogen, $(C_5\text{-}C_{10})$heteroaryl, preferably selected from

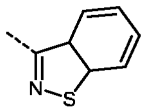

;

$(C_6\text{-}C_{10})$aryl, $-O(C_6\text{-}C_{10})$aryl, or $-S(C_6\text{-}C_{10})$aryl,

which are optionally substituted with one or more substituents independently selected from the group consisting of: halogen, $(C_1\text{-}C_6)$alkyl, or -OH.

Formula (IV):

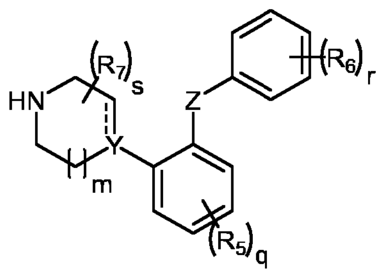

(IV)

wherein in formula (IV)

Z is O or S;

Y is N, C or CH;

s is an integer in the range 0 to 8;

q is an integer in the range 0 to 4;

r is an integer in the range 0 to 5;

m is 1 or 2;

The dotted line represents an optional bond; if the optional bond, indicated as dotted line, is not present, the carbon atom, attached to the optional bond, neighboring Y is saturated with hydrogen and Y is CH or N. If the optional bond is present, Y may be N or C.

$R_7$ is hydrogen, -($C_1$-$C_6$)alkyl, or two $R_7$ attached to the same carbon atom may form a 3 to 6 membered spiro attached cyclo-alkyl;

$R_5$ is hydrogen, halogen, cyano, -$NO_2$, ($C_1$-$C_6$)alkenyl, ($C_1$-$C_6$)alkyl, -O($C_1$-$C_6$)alkyl, -OH, hydroxy($C_1$-$C_6$)alkyl, halo($C_1$-$C_6$)alkyl, ($C_3$-$C_8$)cycloalkyl, ($C_3$-$C_8$)cycloalkyl-($C_1$-$C_6$)alkyl, acyl, - $CO_2$($C_1$-$C_6$)alkyl, -($C_6$-$C_{10}$)aryl, -$SO_2$ ($C_1$-$C_6$)alkyl, or -$NR_xR_y$;

$R_6$ is hydrogen, halogen, -CN, -$NO_2$, ($C_1$-$C_6$)alkenyl, ($C_1$-$C_6$)alkyl, -O($C_1$-$C_6$)alkyl, -OH, hydroxy($C_1$-$C_6$)alkyl, halo($C_1$-$C_6$)alkyl, ($C_3$-$C_8$)cycloalkyl, $C_3$-$C_8$)cycloalkyl-($C_1$-$C_6$)alkyl, acyl, - $CO_2$($C_1$-$C_6$)alkyl, ($C_6$-$C_{10}$)aryl, -$SO_2$ ($C_1$-$C_6$)alkyl, -$NR_xR_y$; -$NR_x$CO($C_1$-$C_6$)alkyl, or -CON$R_xR_y$;

wherein each $R_x$ and $R_y$ is independently selected from hydrogen, ($C_1$-$C_6$)alkyl, ($C_3$-$C_8$)cycloalkyl, ($C_3$-$C_8$)cycloalkyl-($C_1$-$C_6$)alkyl, or ($C_6$-$C_{10}$)aryl;

or $R_x$ and $R_y$, together with the nitrogen to which they are attached from a 3 to 7 membered ring which optionally contains one further heteroatom.

Formula (V):

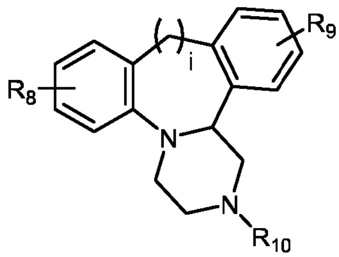

(V)

wherein in formula (V)

$R_8$ and $R_9$ are independently selected from hydrogen, halogen, -OH, -OCO($C_1$-$C_6$)alkyl, ($C_1$-$C_6$)alkyl, -O($C_1$-$C_6$)alkyl, or -$CF_3$;
$R_{10}$ is hydrogen, ($C_1$-$C_6$)alkyl, or -($C_1$-$C_6$)alkylaryl;
i is 0 or 1;

Formula (VI):

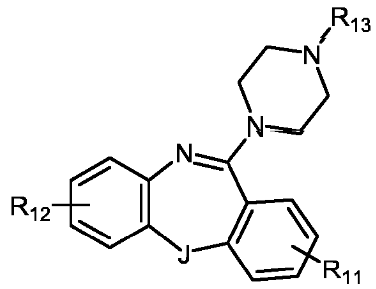

(VI)

wherein in formula (VI)

$R_{11}$ and $R_{12}$ are independently selected from hydrogen, halogen, -OH, $(C_1\text{-}C_6)$alkyl, $-CF_3$, $-O(C_1\text{-}C_6)$alkyl, $-NO_2$, $-NR_oR_z$, $-SO_2NR_oR_z$, or $-S(C_1\text{-}C_6)$alkyl;
wherein each $R_o$ and $R_z$ is independently selected from hydrogen, $(C_1\text{-}C_6)$alkyl;
$R_{13}$ is hydrogen, allyl, $(C_1\text{-}C_6)$alkyl, preferably $(C_1\text{-}C_3)$alkyl, $-O(C_1\text{-}C_6)$alkyl, or hydroxy$(C_1\text{-}C_6)$alkyl, preferably hydroxy$(C_1\text{-}C_3)$alkyl;
J is S or NH.

Only the 5-HT7 receptor antagonists having a structure according to formula (I) are part of the present invention.

Most preferably, the 5-HT7 receptor antagonist is SB-269970.

The *in vitro* experiments of the present invention demonstrate that blocking the basal activity of the 5-HT7R using an antagonist, such as SB-269970, leads to a reduced accumulation of p-tau. In detail, a neuroblastoma cell line transfected with a plasmid expressing mutated tau and 5-HT7R, shows increased protein levels of tau, p-tau and tau aggregates, whereas the simultaneous addition of SB-269970 to the medium prevented this effect. This demonstrates that a treatment with SB-269970 prevents both tangle formation as well as tau hyperphosphorylation.

In a preferred embodiment of the present invention the 5-HT7 receptor antagonist may be used in preventing or treating a tauopathy, wherein the 5-HT7 receptor antagonist is SB-269970.

5-HT7 receptor antagonists having a structure according to at least one of the general formula (I) to (VI), or is a pharmaceutically acceptable salt, enantiomer, diastereomer, racemic mixture, crystalline form, amorphous, unsolved form or solvate of the general formula (I), (II), (III), (IV), (V) or (VI), in particular such as Amisulpride, Lurasidone, Vortioxetine, Mianserin and Clozapine are compounds being approved antidepressants and antipsychotics in the prior art, that act as antagonists of the 5-HT7R such as SB-269970. Until now they have been applied in several other diseases such as schizophrenia, bipolar disorder, depressive disorder or depression in general. However, it has now been surprisingly found that 5-HT7R antagonists may be applied in preventing or treating a tauopathy.

In one embodiment said antagonist of the present invention may be a small organic molecule comprising at least two carbon atoms having a molecular weight in the range between 100 and 1000 Dalton (g/mol). The present invention may provide a 5-HT7 receptor antagonist comprising at least two, three, four, five, six, seven, eight, nine, ten, fifteen, twenty, twenty-five, thirty, thirty-five, fourty, fourty-five, fifty or more carbon atoms. The 5-HT7 receptor antagonist of the present invention may have a molecular weight in the range between 100 and 1000 Dalton, 150 to 800 Dalton, 170 to 600 Dalton, or 200 to 550 Dalton. Preferably, the 5-HT7R antagonist may comprise at least ten carbon atoms having a molecular weight in the range between 200 to 550 Dalton.

*Pharmaceutical compositions*

In a further aspect, the present invention provides a pharmaceutical composition comprising the 5-HT7 receptor antagonist as specified above. Further, said pharmaceutical composition may comprise the 5-HT7 receptor antagonist and one or more pharmaceutically acceptable excipients.

The compounds described in the present invention ( of formula I, or a pharmaceutically acceptable salt, enantiomer, diastereomer, racemic mixture, crystalline form, amorphous, unsolved form or solvate of the general formula I) are preferably administered to a patient in need thereof via a pharmaceutical composition. In one embodiment, the pharmaceutical composition comprises a compound as described above ( having the general formula I, or a pharmaceutically acceptable salt, enantiomer, diastereomer, racemic mixture, crystalline form, amorphous, unsolved form or solvate of the general formula I, ) and one or more pharmaceutically acceptable excipients.

The pharmaceutical composition may be administered to an individual by any route, such as enterally, parenterally or by inhalation.

The expressions "enteral administration" and "administered enterally" as used herein mean that the drug administered is taken up by the stomach and/or the intestine. Examples of enteral administration include oral and rectal administration. The expressions "parenteral administration" and "administered parenterally" as used herein mean modes of administration other than enteral administration, usually by injection or topical application, and include, without limitation, intravenous, intramuscular, intraarterial, intrathecal, intracapsular, intraosseous, intraorbital, intracardiac, intradermal, intraperitoneal, transtracheal, subcutaneous, subcuticular, intraarticular, subcapsular, intracerebral, intracerebroventricular, subarachnoid, intraspinal, epidural and intrasternal administration (such as by injection and/or infusion) as well as topical administration (e.g., epicutaneous, or through mucous membranes (such as buccal, sublingual or vaginal)). However, preferred is an enterally or parenterally administration.

The compounds used in the present invention are generally applied in "pharmaceutically acceptable amounts" and in "pharmaceutically acceptable preparations". Such compositions may contain salts, buffers, preserving agents, carriers and optionally other therapeutic agents.

The term "excipient" when used herein is intended to indicate all substances in a pharmaceutical composition which are not active ingredients (e.g., which are therapeutically inactive ingredients that do not exhibit any therapeutic effect in the amount/concentration used), such as, e.g., carriers, binders, lubricants, thickeners, surface active agents, preservatives, emulsifiers, buffers, flavoring agents, colorants, or antioxidants.

The pharmaceutical compositions comprising the 5-HT7 receptor antagonist described in the present invention may also comprise a pharmaceutically acceptable carrier. As used herein, "pharmaceutically acceptable carrier" includes any and all solvents, dispersion media, coatings, isotonic and absorption delaying agents, and the like that are physiologically compatible. The "pharmaceutically acceptable carrier" may be in the form of a solid, semisolid, liquid, or combinations thereof. Preferably, the carrier is suitable for enteral (such as oral) or parenteral administration (such as intravenous, intramuscular, subcutaneous, spinal or epidermal administration (e.g., by injection or infusion)). Depending on the route of administration, the active compound, i.e., the 5-HT7 receptor antagonist used in the present invention, either alone or in combination with one or more additional active compounds, may be coated in a material to protect the active compound(s) from the action of acids and other natural conditions that may inactivate the active compound.

Thus, a pharmaceutical composition of the present invention may comprise the 5-HT7 receptor antagonist, one or more pharmaceutically acceptable excipient(s) and a pharmaceutically acceptable carrier. Additionally, a pharmaceutical composition of the present invention may comprise the 5-HT7 receptor antagonist, one or more pharmaceutically acceptable excipient(s), a pharmaceutically acceptable carrier and at least one additional active compound, thus being used as a combined preparation.

Examples of suitable aqueous and non-aqueous carriers which may be employed in the pharmaceutical compositions used according to the present invention include water (e.g., water for injection), ethanol, polyols (such as glycerol, propylene glycol, polyethylene glycol, and the like), aqueous solutions of a salt, carbohydrate, sugar alcohol, or an amino acid (such as saline or an aqueous amino acid solution), and suitable mixtures and/or buffered forms thereof, injectable organic esters (such as ethyl oleate) and preferably oils, such as vegetable oils or olive oil). In case of inhalation a suitable carrier for the pharmaceutical composition may be e.g. captisol, sugar alcohol or any other aqueous component.

Pharmaceutically acceptable carriers include sterile aqueous solutions or dispersions and sterile powders for the extemporaneous preparation of sterile injectable solutions or dispersions. The use of such media and agents for pharmaceutically active compounds is known in the art. Except insofar as any conventional media or agent is incompatible with the active compound, use thereof in the pharmaceutical compositions used according to the present invention is contemplated.

In some embodiments, an additional active compounds may also be administered together with, before or after the compound used in the present invention In one embodiment, the pharmaceutical composition described herein comprises a compound as described above ( formula I, or a pharmaceutically acceptable salt, enantiomer, diastereomer, racemic mixture, crystalline form, amorphous, unsolved form or solvate of the general formula I) and at least one additional active compound. Also comprised herein is the pharmaceutical composition described herein comprises a compound as described above ( of formula I, a pharmaceutically acceptable salt, enantiomer, diastereomer, racemic mixture, crystalline form, amorphous, unsolved form or solvate of the general formula I) and at least one additional active compound and one or more pharmaceutically acceptable excipients. Also comprised herein is the pharmaceutical composition described herein comprises a compound as described above ( of formula I, or a pharmaceutically acceptable salt, enantiomer, diastereomer, racemic mixture, crystalline form, amorphous, unsolved form or solvate of the general formula I) and at least one additional active compound and a pharmaceutically acceptable carrier.

The "additional active compound" may be selected from any compound which may be used in the prevention or treatment of tauopathy. The additional active compound may induce an additive or synergistic therapeutic effect. The additional active compound used to prevent or treat a tauopathy may be a CDK5-inhibitor, preferably Roscovitine. Thus, the present invention comprises a pharmaceutical composition for use in preventing or treating a tauopathy comprising the

5-HT7 receptor antagonist as mentioned elsewhere herein and a CDK5-inhibitor, preferably Roscovitine.

The pharmaceutical composition may also comprise adjuvants such as preservatives, wetting agents, emulsifying agents, pH buffering agents, and dispersing agents. Prevention of the presence of microorganisms may be ensured by sterilization procedures and/or by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Regardless of the route of administration selected, the active compounds (the 5-HT7 receptor antagonist), which may be used in a suitable hydrated form, and/or the pharmaceutical compositions used according to the present invention, are formulated into pharmaceutically acceptable dosage forms by conventional methods known to those of skill in the art (cf., e.g., Remington, "The Science and Practice of Pharmacy" edited by Allen, Loyd V., Jr., 22nd edition, Pharmaceutical Sciences, September 2012; Ansel et al., "Pharmaceutical Dosage Forms and Drug Delivery Systems", 7th edition, Lippincott Williams & Wilkins Publishers, 1999).

A pharmaceutical composition can be administered by a variety of methods known in the art. As will be appreciated by the skilled artisan, the route and/or mode of administration will vary depending upon the desired results. The pharmaceutical compositions containing one or more active compounds can be prepared with carriers that will protect the one or more active compounds against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Methods for the preparation of such compositions are generally known to those skilled in the art. See, e.g., Sustained and Controlled Release Drug Delivery Systems, J. R. Robinson, ed., Marcel Dekker, Inc., New York, 1978.

To administer a compound used in the present invention by certain routes of administration, it may be necessary to coat the compound with, or co-administer the compound with, a material to prevent its inactivation. For example, the compound may be administered to an individual in an appropriate carrier, for example, liposomes, oil or a diluent. Pharmaceutically acceptable diluents include saline and aqueous buffer solutions. Liposomes include water-in-oil-in-water CGF emulsions as well as conventional liposomes (Strejan *et al., J. Neuroimmunol. 7:* 27(1984)).

Pharmaceutical compositions typically are sterile and stable under the conditions of manufacture and storage.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the individuals to be treated; each unit contains a predetermined quantity of active compound calculated to produce the desired therapeutic effect in association with the required pharmaceutical carrier. The specification for the dosage unit forms used according to the present invention are dictated by and directly dependent on (a) the unique characteristics of the active compound and the particular therapeutic effect to be achieved, and (b) the limitations inherent in the art of compounding such an active compound for the treatment of sensitivity in individuals.

Generally, out of 100% (for the pharmaceutical formulations/compositions), the amount of active ingredient (in particular, the amount of the compound used according to the present invention, optionally together with other therapeutically active agents, if present in the pharmaceutical formulations/compositions) will range from about 0.01% to about 99%, preferably from about 0.1% to about 70%

The amount of active ingredient, e.g., a compound used according to the present invention, in a unit dosage form and/or when administered to an individual or used in therapy, may range from about 0.1 mg to about 10000 mg (for example, from about 1 mg to about 5000 mg, such as from about 10 mg to about 2000 mg) per unit, administration or therapy.

Actual dosage levels of the active ingredients in the pharmaceutical compositions used according to the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start with doses of the compounds used according to the present invention at levels lower than that required in order to achieve the desired therapeutic effect and gradually increase the dosage until the desired effect is achieved. In general, a suitable daily dose of a composition used according to the present invention will be that amount of the compound which is the lowest dose effective to produce a therapeutic effect. Such an effective dose will generally depend upon the factors described above. It

is preferred that administration be oral, intravenous, intramuscular, intraperitoneal, or subcutaneous. It is even more preferred that administration be oral, intramuscular or intravenous. If desired, the effective daily dose of a pharmaceutical composition may be administered as two, three, four, five, six or more sub-doses administered separately at appropriate intervals throughout the day, optionally, in unit dosage forms. While it is possible for a compound used according to the present invention to be administered alone, it is preferable to administer the compound as a pharmaceutical formulation/composition.

For oral administration, the pharmaceutical composition used according to the present invention can take the form of, for example, tablets or capsules prepared by conventional means with pharmaceutical acceptable excipients such as binding agents (e.g., pregelatinised maize starch, polyvinylpyrrolidone, hydroxypropyl methylcellulose), fillers (e.g., lactose, microcrystalline cellulose, calcium hydrogen phosphate), lubricants (e.g., magnesium stearate, talc, silica), disintegrants (e.g., potato starch, sodium starch glycolate), or wetting agents (e.g., sodium lauryl sulphate). Liquid preparations for oral administration can be in the form of, for example, solutions, syrups, or suspensions, or can be presented as a dry product for reconstitution with water or other suitable vehicle before use. Such liquid preparation can be prepared by conventional means with pharmaceutically acceptable additives such as suspending agents (e.g., sorbitol, syrup, cellulose derivatives, hydrogenated edible fats), emulsifying agents (e.g., lecithin, acacia), non-aqueous vehicles (e.g., almond oil, oily esters, ethyl alcohol, fractionated vegetable oils), preservatives (e.g., methyl or propyl-p-hydroxycarbonates, sorbic acids). The preparations can also contain buffer salts, flavouring, coloring and sweetening agents as deemed appropriate.

In one embodiment, the compound is orally administered in a concentration of at most 1000 mg/kg body weight (such as at most 500 mg/kg body weight, at most 400 mg/kg body weight, at most 300 mg/kg body weight, at most 200 mg/kg body weight, at most 100 mg/kg body weight, at most 50 mg/kg body weight, at most 40 mg/kg body weight, at most 30 mg/kg body weight, at most 20 mg/kg body weight, at most 10 mg/kg body weight or less).

Oral formulation can include standard carriers such as pharmaceutical grades of mannitol, lactose, starch, magnesium stearate, sodium saccharine, cellulose, magnesium carbonate, etc.

The pharmaceutical composition used according to the present invention can be formulated for parenteral administration by injection, for example, by bolus injection, continuous infusion or intraperitoneal injection. In one embodiment, the compounds or compositions used according to the present invention may be administered by slow continuous infusion over a long period, such as more than 24 hours, in order to reduce toxic side effects. The administration may also be performed by continuous infusion over a period of from 2 to 24 hours, such as of from 2 to 12 hours. Such regimen may be repeated one or more times as necessary, for example, after 6 months or 12 months.

In one embodiment, the compound is parenterally administered (e.g., intravenously, intramuscularly, subcutaneously or by inhalation), in a concentration of at most 100 mg/kg body weight (such as at most 50 mg/kg body weight, at most 40 mg/kg body weight, at most 30 mg/kg body weight, at most 20 mg/kg body weight, at most 10 mg/kg body weight, at most 5 mg/kg body weight, at most 4 mg/kg body weight, at most 3 mg/kg body weight, at most 2 mg/kg body weight, at most 1 mg/kg body weight, at most 0.1 mg/kg body weight, at most 0.01 mg/kg body weight).

Formulations for injection can be presented in units dosage form (e.g., in phial, in multidose container), and with an added preservative. The pharmaceutical composition used according to the present invention can take such forms as suspensions, solutions or emulsions in oily or aqueous vehicles, and can contain formulatory agents such as suspending, stabilizing, or dispersing agents. Alternatively, the agent can be in powder form for constitution with a suitable vehicle (e.g., sterile pyrogen-free water) before use. Typically, compositions for intravenous administration are solutions in sterile isotonic aqueous buffer. Where necessary, the composition can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

Dosage forms for the topical, transdermal or inhalation administration of compositions used according to the present invention may include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The pharmaceutical composition used according to the invention can also, if desired, be presented in a pack, or dispenser device which can contain one or more unit dosage forms containing the active compound. The pack can for example comprise metal or plastic foil, such as blister pack. The pack or dispenser device can be accompanied with instruction for administration.

In summary, the pharmaceutical composition comprising the 5-HT7 receptor antagonist of the present invention may be used in preventing or treating a tauopathy by inhibiting both the receptor-induced phosphorylation and thus the accumulation of tau proteins. Thereby, said pharmaceutical composition comprising the 5-HT7 receptor antagonist of the

present invention is able to halt the progression of a tauopathy or completely inhibit the development of a tauopathy and does not only treat the symptoms which are caused by said disease (e.g. cognitive functions, psychosis and behavioral problems).

In some embodiments, the present invention may comprise the pharmaceutical composition comprising the 5-HT7 receptor antagonist for use in treating or preventing a dementia-associated tauopathy, wherein said dementia-associated tauopathy is selected from the group consisting of Alzheimer's disease, frontotemporal dementia, primary age-related tauopathy (PART), chronic traumatic encephalopathy, progressive supranuclear palsy (PSP), corticobasal degeneration, dementia with Lewy Bodies (DLB), frontotemporal dementia and parkinsonism linked to chromosome 17 (FTDP-17), argyrophilic grain disease (AGD), Huntington disease, glial globular tauopathy, preferably Alzheimer's disease or frontotemporal dementia, more preferably Alzheimer's disease.

In another embodiment, the present invention may also comprise the pharmaceutical composition comprising the 5-HT7 receptor antagonist for use in treating or preventing a tauopathy, wherein said tauopathy is selected from the group consisting of Alzheimer's disease, frontotemporal dementia, primary age-related tauopathy (PART), chronic traumatic encephalopathy, progressive supranuclear palsy (PSP), corticobasal degeneration, dementia with Lewy Bodies (DLB), frontotemporal dementia and parkinsonism linked to chromosome 17 (FTDP-17), argyrophilic grain disease (AGD), Huntington disease, glial globular tauopathy, amyotrophic lateral sclerosis (ALS), Parkinson's disease, spinal muscular atrophy (SMA), cerebral amyloid angiopathy (CAA), preferably Alzheimer's disease or frontotemporal dementia, more preferably Alzheimer's disease.

## EXAMPLES OF THE INVENTION

The following Examples illustrate the invention, but are not to be construed as limiting the scope of the invention.

Only the compound having a structure according to formula (I) is part of the present invention.

### Material and Methods

All treatments and behavioral procedures were conducted in accordance with animal research ethics standards defined by German law and approved by the Ethical Committee on Animal Health and Care of the State of Saxony-Anhalt (42502-2-1343 DZNE). For the behavioral experiment, male C57BL/6J mice (2- to 3-month-old) were used. Animals were separately kept in an air-conditioning system (temperature: 20 ± 1 °C; humidity: 50 ± 3 %) and in a reverse light-dark cycle (12:12 hours, light on at 9:00 pm) with food and water ad *libitum,* and they were tested during the dark phase of the cycle, when mice are active.

### Cell Culture and Transfection.

The neuroblastoma cell line N1E-115 (American Type Culture Collection) was cultured and maintained in Dulbecco's modified Eagle's medium with 10% FBS and 1% of penicillin/streptomycin at 37°C and 5% $CO_2$. For transient transfection, cells were plated in 35 mm dishes and transfected with the indicated cmv-driven pcDNA constructs using Lipofectamine 2000 reagent (Life technologies) according to the manufacturer's instructions. Briefly, for a 35 mm Petri dish of N1E-115 cells, a maximum of 0.5 μg of pcDNA encoding eGFP-tagged Tau[R406W] mutant and/or 0.5 μg of pcDNA encoding the HA-tagged 5-HT7R were co-transfected with either 1 μg of CDK5-mCherry or 1 μg of CDK5-T33 (CDK5-DN) or 0.5 μg of shRNA directed against the α-subunit of the G12 protein (Oligoenge). Four hours post-transfection, cells were pre-treated with 100 nM of the selective 5-HT7R inverse agonist SB-269970 (Tocris) or left untreated overnight, following treatment with 20 μM of the CDK5 inhibitor roscovitine, 10 μM of the GSK3 inhibitor SB-216763 or 20 μM 5-Hydroxytryptamine (5-HT) or a combination of these drugs for 1 h.

### Primary Cortical Neurons and viral Infection.

Mice were bred at the central animal facility of Hannover Medical School. Following mice strains were used: C57BL/6J (wild type, WT), C57BL/6J-Htr7tm1Sut (5-HT7R knockout, 5-HT7R KO) (Hedlund et al., 2003, Trends Pharmacol. Sci. *25*, 481-486). Primary cortical neurons were dissected according to an optimized protocol for mouse cortical neurons (Speranza et al, 2013, Neuropharmacology 67, 155-167). Cortices of P0-2 mice were dissected under a binocular microscope in ice-cold Hanks balanced salt solution. Cells were enzymatically dissociated from cortex by incubation in a papain solution for 20 min at 37°C. After two trituration steps cortical neurons of one mouse brain were plated on 35 mm petri dishes coated with 15 μg/ml of poly-D-lysine. Cells were maintained in Neurobasal A medium in a humidified incubator at 37°C for at least 3 days.

Primary cortical neurons were infected at DIV 10 with equal amounts of different Adeno-associated viruses.

**Preparation of adeno-associated viruses.**

The following monocistronic viral vectors with a synapsin promotor as well as bicistronic viral vectors with a H1 and a synapsin promotor were used pAAV_Syn_eGFP, pAAV_Syn_eGFP-Tau[R406W], pAAV_H1_scramble-shRNA_Syn_eGFP pAAV_H1_5-HT7R-shRNA_Syn_ eGFP. Target sequences for the short hairpins are: scramble A CTACCGTTGTTATAGGTG (SEQ ID NO. 1) and 5-HT7R TCCTTTATCATTATGGTGT (SEQ ID NO. 2). Adeno-associated viruses were produced using the AAV-DJ Packaging System from CELL BIOLABS (San Diego). In short, HEK293 cells were transfected with the pAAV-DJ and pHelper constructs along with one pAAV constructs. After three days viral vectors were harvested using freeze-thaw cycles and concentrated using Amicon Ultra-15 Centrifugal Filter Units with a cut off of 10 kDa. Titers were determined using quantitative Real-Time PCR with primers for the WPRE element in the viral plasmid.

**Quantitative Real-Time Polymerase Chain reaction.**

The total RNA of transfected N1E-115 cells and infected neurons was isolated using RNeasy Mini Kit (Quiagen). Equal amounts of RNA were reverse transcribed using SuperScript® III First-Strand Synthesis System (Invitrogen). The expression analysis was performed on a StepOne System (Applied Biosystems) using TaqMan Universal PCR Master Mix (Applied Biosystems). For the detection of 5-HT7 and Ga12 mRNA, corresponding Gene Expression Assays (Applied Biosystems; Cat. no. 4331182 and 4331182) containing gene-specific primers were used. For quantitative analysis, GAPDH was analyzed in parallel with the following primer/probe sequences: GAPDH: fw 5'-TGCACCACCAACTGCT TAGC-3' (SEQ ID NO. 3); rev 5'- GGCATGGACTGTGGTCATGAG-3' (SEQ ID NO. 4); probe 5'-/6-FAM/ CCCTGGCC AAGGTCATCCATGACAAC (SEQ ID NO. 5) /TAMRA/-3' (Sigma). Calculation of relative mRNA levels was performed by using the DDCt method. To determine the titer of different AAV preparations, capsids were digested with proteinase K and virus DNA was analyzed using WPRE primers (fw 5'- CCTGGTTGCTGTCTCTTTATGAGG-3' (SEQ ID NO. 6); rev 5'-TGACAGGTGGTGGCAATGC-3' (SEQ ID NO. 7); probe 5'-/6-FAM/ CGTTGTCAGGCAACGTGGCGTGGTG (SEQ ID NO. 8) /TAMRA/-3' (Sigma). For caculation of the viral gene copy number per μl the relative standard curve method was used.

**Western blotting.**

Cells were placed on ice and washed with ice-cold PBS. Cells were then scraped of in 100 μl of lysis buffer (20 mM HEPES, pH 7.4, 100 mM NaCl, 100 mM NaF, 1 mM sodium ortho-vanadate, 5 mM EDTA, 1% Triton X-100 and protease inhibitors CLAP and PMSF) and centrifuged at 14,000 g at 4°C for 15 min to eliminate cellular debris. Lysates were then mixed with 20 μl of 6 x SDS containing 5% β-mercaptoethanol, boiled at 95°C for 10 min and either directly subjected to SDS-PAGE or stored at -20°C for subsequent analyses. Equal amounts of protein were separated on 10%, 12% or 15% SDS-polyacrylamide gels. Proteins were then transferred onto nitrocellulose membranes (100 mA/gel, 1h) and membranes were blocked in 5% non-fat milk in TBS-T [0.2 M Tris, 5 M NaCl, 0.1% Tween20] or 5% bovine serum albumin in TBS-T for 1 h at room temperature and probed with the following antibodies overnight at 4°C: AT270 (1:2,000), Tau 5A6 (DSHB, 1:500), Tau-5 (1:2,000, Cell signaling), anti-GAPDH (Millipore, 1:10,000), anti-CDK5 (Cell signaling, 1:1,000), anti-GSK3 (1:2,000), anti-pTyr216 GSK3 (1:2,000), anti-Gα12 (Santa Cruz, 1:500), anti-mCherry (1:1,000), anti-HA (1:500), HRP-conjugated anti-EGFP (1:500). After three washing steps the membranes were incubated with the corresponding HRP-conjugated secondary antibody (1:10,000) for 1 h at room temperature and detected on a Fusion SL advanced MP (Peqlab) using "super signal western femto maximum sensitivity substrate" (Thermo Fisher) or "Ace Glow essential substrate" (Peqlab). Relative protein levels were determined by normalizing detected intensities to the corresponding GAPDH signal probed on the same membrane.

**Sarkosyl Fractionation.**

Sarkosyl fractionation was performed following a modified protocol described earlier (Jo et al. Nature Communcations 2013). Briefly, N1E-cells were washed with PBS, scraped of in 100 μl ice-cold MES-Buffer (0.1 M MES (pH 7.0), 1 mM EDTA, 0.5 mM $MgSO_4$, 1 M sucrose, 1 mM NaF, 1 mM $Na_3VO_4$, PMSF, CLAP) and homogenized using a tissue grinder. Homogenates were then centrifuged for 5 min at 500 g at 4°C to remove nuclei and cellular debris. The supernatant was then centrifuged at 50,000 g for 20 min at 4°C and the resulting supernatant was collected as "soluble fraction" and incubated at 95°C with 20 μl 6 x SDS for 5 min. The pellet was resuspended in 50 μl RAB-Buffer (100 mM MES (pH 6.8), 10% sucrose, 2 mM EGTA, 0.5 mM $MgSO_4$, 500 mM NaCl, 1 mM $MgCl_2$, 10 mM $NaH_2PO_4$, 20 mM NaF, 1% N-lauroylsarcosine (Sarkosyl), CLAP, PMSF), vortexed for 1 min at room temperature, incubated overnight at 4°C and then centrifuged at 200,000 g for 30 min at room temperature. The supernatant was removed, the pellet was collected as "sarkosyl insoluble fraction" and incubated in 60 μl 1 x SDS at 95°C for 5 min.

**Analysis of tau tangle formation in N1E cells and primary cortical neurons.**

For quantitative analysis of intracellular tau tangles N1E-115 cells and primary cortical neurons were used. N1E-115 cells were transfected either with eGFP-Tau[R406W], eGFP or eGFP-Tau[R406W] in combination with Cdk5 [T33]-mCherry, then 4 hours afterwards treated with 20 μM roscovitine, 100 nM SB-269970 or $H_2O$ and fixed the following day using ice-cold methanol for 5 minutes. In case of primary cortical neurons, cultures of P0-3 WT or 5-HT7R KO mice were infected with AAV-eEGFP-Tau-R406W or AAV-eGFP at DIV10. Treatment with 20 μM roscovitine, 100 nM SB-269970 or $H_2O$ started immediately at the day of infection and was repeated the following two days. At DIV13 neurons were fixed in the same manner as the N1E-115 cells. Fixation of N1E-115 cells and neurons was followed by covering with Fluomount-G and analysis was performed in a blinded fashion using a Till ID IMIC white-field microscope. EGFP positive cells with tangles (rod-like inclusions) as well as the total number of eGFP positive cells were counted and the fraction of tau tangle containing cells was calculated.

To visualize hyperphosphorylation of tau in tangle structures N1E-115 cells and neurons were infected, treated and fixed as described but additionally stained prior to mounting. Fixed cells were blocked with 5% normal donkey serum (NDS) in PBS for 1 hour at room temperature and incubated overnight with phospho-tau antibody AT8 (Thermofisher, 1:100) in 1% NDS in PBS at 4°C. The following day three washing steps with PBS were performed and cells were incubated with the secondary antibody DyLight 649 (Jackson ImmunoResearch, 1:400) for 1 hour at room temperature. After three more washing steps with PBS, cells were covered with Fluomount-G and images were recorded using a Zeiss LSM780 confocal microscope with a 40x water-immersion objective.

**Co-Immunoprecipitation.**

For Co-Immuniprecipitation N1E-cells were grown and transfected in 35mm-dishes. Cells were placed on ice, washed 1x with ice-cold PBS and lysed by incubation in cold RIPA-buffer (20 mM Tris-HCl pH 7.4, 150 mM NaCl, 10 mM EDTA, 10 mM iodacetamide, 1% Triton x-100, 1% deoxycholic acid, 0.1% SDS, 1mM PMSF, 5 μg/ml aprotinin, 2 μg/ml leupeptin) for 10 min. Afterwards lysates were centrifuged for 15 min at 4°C and 14,000 g to eliminate cellular debris. Antibodies were added to lysates and incubated overnight at 4°C under constant rotation. Precipitation was performed by addition of 40 μl of Protein-A sepharose suspension (Sigma-Aldrich) for 2 h at 4°C under constant rotation. After centrifugation for 2 min at 4°C and 14000 g sepharose was washed extensively. After elution with Thorner buffer (8M urea, 5% SDS, 40 mM Tris/Cl ph 6.5, 0.1 mM EDTA, 0.4 mg/ml BPB, 1% β-mercaptoethanol) for 20 min at 37°C lysates were centrifuged for 10 min at 14,000 g and subjected to SDS-PAGE.

Co-immunoprecipitation from cortex homogenates was performed according to Renner et al. (2012), J. Cell Sci. 125, 2486-2499. Briefly, cortices isolated from P6 C57BL/6J mice were homogenized, and membrane fractions were prepared by differential centrifugation. Lysates were incubated with a rabbit polyclonal antibody directed against the murine 5-HT7R (10 μg; Santa Cruz) followed by incubation with Protein-A sepharose, SDS-PAGE and Western blotting with mouse-anti-CDK5 (1:1,000; Cell Signaling) or rabbit-anti-5-HT7R (1:1,000; Abcam).

**Lux-FRET analysis.**

Linear unmixing Förster resonance energy transfer (Lux-FRET) measurements were in principle conducted as described in Wlodarczyk et al. 2008 (Wlodarczyk et al., 2008, Biophys. J. 94, 986-1000). For image acquisition we used a total internal reflection (TIRF) microscope setup (FEI/Till ID IMIC) which allows to restrict the observed area to the cell membrane (around 100 nm) adjacent to the coverslip surface. For image analysis and evaluation custom-written MATLAB scripts were used. In short, reference measurements of single fluorophore were imaged to allow linear unmixing of the spectra which were acquired with two excitations and two emission channels. To calculate molecular fractions of the two components a tandem construct with one to one stoichiometry was utilized along with the known quantum yields of the fluorophores. In contrast to the common ratiometric FRET method, the lux-FRET method allows the absolute quantification of the apparent FRET efficiency. N1E-115 neuroblastoma cells were grown on 18 mm coverslips and co-transfected with plasmids encoding eCFP-tagged 5-HT7R and eYFP-tagged CDK5. After 24 h, cells were imaged with the TIRF-iMIC setup (excitation wavelengths: 440 nm and 514 nm). We calculated the predicted apparent FRET efficiency

$Ef_{DA} = \frac{1}{2} Ef_D/(1 - x_D) = \frac{1}{2} Ef_A/x_D$ assuming a standard dimerization model.

**Virus injection.**

Acute injection of adeno-associated viruses (AAVs) *in vivo* was done under short isoflurane anesthesia. For intra-mPFC injection, we used a digitally controlled infusion system (UltraMicroPump, UMP3, and Micro4 Controller, WPI, USA) fed with a 10 μl-Hamilton syringe and NanoFil 135 μm (35 GA) beveled needle. The thin (135 μm) part of the needle went

into the mouse brain during injection. Injections were performed as follows: 1) a mouse was anesthetized with 1 - 3 % isoflurane; 2) it was put into the stereotaxic frame (Narishige, Tokyo, Japan) under 1 % isoflurane in combination with $O_2$; 3) the skull of animal was exposure by sagittal cut; 4) the skull plane were horizontally corrected with Bregma and Lambda, and the coordination of PFC were marked onto skull surface bilaterally (AP +1.7 mm, ML ±0.3 mm); 5) holes for intra-PFC injection were made with dental drill (Eickemeyer, Tuttlingen, Germany); 6) a NanoFil injecting needle was carefully placed inside of the hole and advanced further down until when the needle should reach the surface of mouse brain; 7) once the brain surface was reached, a slow (10 μm increment) step dial was used to deepen the needle into the brain; 8) for experiments related to 5HT7R knockdown with AAV-shRNA-5HT7R: appropriate volumes of AAVs (500 nl per site for AAV-EGFP and AAV-Tau-EGFP groups, and 750 nl per site for 1:1 mixtures of AAV-Tau plus AAV-shRNA-5HT7R and AAV-Tau plus AAV-Scramble-shRNA-5HT7R) were bilaterally injected in the prelimbic cortex at an injection rate of 2 nl/s.; for experiments related to 5HT7R inverse agonist treatment: AAV-eGFP-Tau[R406W] ($5x10^7$ vg/μl) or eGFP-AAV($6x10^7$ vg/μl) was injected with the following settings: 500 nl per site as deep as 2.2 mm from the brain surface at injection rate of 50 nl/min; 9) after injection was complete (~ 10 min), the needle was left in the injection site for another 5 min and then gently removed; 10) the same procedure was performed for the other hemisphere. The wound was closed using surgical tissue adhesive (Vetbond™, 3M™, Germany), and Carpofen (Rimadyl, Pfizer Pharma GmbH, Germany) was subcutaneously injected (5 mg/kg of body weight). The whole injection process took approximately 40 - 50 min under isoflurane anesthesia, and the mice recovered rather quickly, starting to move in approximately 2 - 3 min after being returned to their home cages. Complete recovery of a mouse after such short anesthesia usually occurs within 3 - 5 min. In experiments with Amisulpride injection, the mice remained in their home cage for three weeks of recovery and then received Amisulpride treatment (1 mg/kg, i.p.) or vehicle control every day for 16 days until the start of recency test.

**Immunocytochemistry.**

For the analysis of 5-HT7R, CDK5 and VGLUT-1 distribution in dissociated neurons derived from prefrontal cortex, cultured cells were fixed at DIV14 with 4% PFA with 4% sucrose for 10 min at RT, after blocking of non-specific binding sites with 5% normal donkey serum (NDS) diluted in PBS for 45 min at RT, primary antibody rabbit polyclonal anti-5-HT7R (1:100; Acris) diluted in PBS containing 3% BSA, were applied for 4 h RT. After washing with PBS, secondary antibody was applied at RT for 30 min: donkey anti-rabbit DyLight 649 conjugated (Jackson ImmunoResearch Laboratories, diluted 1:400 in PBS containing 3% BSA). After following washing with PBS, neurons were permeabilized with 0.1% Triton X-100 diluted in PBS for 10 min. After blocking of non-specific binding sites with 3% bovine serum albumin (BSA) diluted in PBS for 30 min at RT, primary antibodies mouse monoclonal anti-CDK5 (1:100, Cell Signaling), guinea pig polyclonal anti-VGLUT-1 (1:1,000; Synaptic Systems) diluted in PBS containing 3% BSA, were applied at 4°C overnight. After washing with PBS, appropriate secondary antibodies were applied at RT for 30 min: donkey anti-mouse DyLight 549 conjugated and donkey anti-guinea pig DyLight 488 conjugated antibodies (Jackson ImmunoResearch Laboratories, all diluted 1:400 in PBS containing 3% BSA). After following washing with PBS, the coverslips were mounted in anti-quenching medium (Fluoromount G, Southern Biotechnology Associates, Biozol, Eching, Germany) and subjected for imaging analysis with a confocal microscope Zeiss LSM780 with a 40x water-immersion objective.

**Apoptosis assay.**

To investigate neuronal cell death after tau aggregation we utilized the CellEvent™ Caspase-3/7 Green Detection Reagent (Thermo Fisher) in infected primary cortical neurons of P0-1 mice. Infection and treatment were performed using the paradigm described above for analysis of tau tangle formation. On DIV13 fluorogene substrate of caspase 3 and 7 was added to the culturing medium to a final concentration of 2 μM. After incubation for 30 min at cell culture conditions (37°C, 5% $CO_2$), cells were fixed in 3.7 % paraformaldehyde for 10 min, washed three times with PBS and covered with Fluomount-G. Using a Zeiss LSM780 confocal microscope, apoptotic neurons were identified by substrate cleavage leading to visible fluorescence emission at 530 nm. Apoptotic neurons and the total number of infected neurons were counted in a blinded fashion and the fraction of apoptotic cells was determined. Representative images were taken prior to fixation for maximum fluorescence intensity.

**Electrophysiological recordings in prefrontal brain slices.**

Cortical brain slices were prepared from 3-5-month-old C57BL6/J mice 4-6 weeks after AAV injection to allow for sufficient virus expression. After quick cervical dislocation and decapitation, the brain was isolated and transferred into ice-cold artificial cerebrospinal fluid (aCSF) continuously supplied with 95% $O_2$/5% $CO_2$. The aCSF for slicing contained (in mM): 250 sucrose, 24 $NaHCO_3$, 25 glucose, 2.5 KCl, 1.25 $NaH_2PO_4$, 2 $CaCl_2$, and 1.5 $MgCl_2$ (osmolarity 345-350 mOsm/kg, pH 7.4). Depending on the spread of EGFP expression, coronal mPFC slices (400 μm thick), which contained the prelimbic, infralimbic, and cingulate cortices (+1.9-1.1 mm relative to bregma), were cut using a VT1200S vibratome

(Leica, Nussloch, Germany). After cutting, the slices were placed in a custom-built slice holding chamber filled with 95% $O_2$/5% $CO_2$-bubbled recording aCSF (osmolarity 300-305 mOsm/kg, pH 7.4), in which the sucrose content was completely substituted by 120 mM NaCl.

After incubating the slices for >2 h in recording aCSF at room temperature, a 400μm thick-slice was placed in a submerged-type recording MC membrane chamber (Scientific Systems Design Inc., Mississauga, Ontario, Canada), where it was continuously perfused with oxygenated aCSF (4 ml/min flow rate). All recordings were performed at room temperature (22-25°C). Only slices expressing EGFP in the mPFC were taken for recordings.

It has been shown that the input fibers of pyramidal neurons are located in layer II-III, whereas the dendrites and cell bodies of pyramidal cells are found in layer V (Huang et al., 2004). Thus, field excitatory postsynaptic potentials (fEPSPs) were evoked in layer II-III and recorded in layer V of the mPFC using thin-walled glass electrodes (borosilicate glass, 0.188 x 100 x 1.5 mm, Hilgenberg, Malsfeld, Germany). When filled with recording aCSF, the tip resistance for the stimulation and recording glass electrodes was 0.3-0.5 MΩ and 2-2.5 MΩ, respectively.

For each slice, the basal excitatory synaptic transmission was assessed by recording 3-5 fEPSPs at increasing stimulus intensity (range 40-80 μA, 5 μA increments). Then, stimulus-response curves were computed by plotting the relationship between mean fEPSP slope and stimulus intensity. For baseline recording before LTP induction, we recorded 30 fEPSPs evoked at 0.05 Hz (10 min). If the fEPSP slope was instable during this period, the baseline recording was repeated. The supramaximal intensity was defined as the intensity level, at which a population spike in the fEPSP started occurring. In each slice, baseline fEPSP recording and the following long-term potentiation (LTP) were recorded at ~50% of the supramaximal stimulation intensity. For induction of LTP, five trains of theta-burst stimulation (TBS) with an inter-theta-train interval of 20 s were applied (8 bursts per train at 5 Hz; 4 pulses per burst at 100 Hz; 0.2 ms burst duration) (Brennaman et al., 2011, Neurobiology of Disease, 43, 372-378).

Using PatchMaster software (Heka Elektronik, Lambrecht, Germany), the rising fEPSP slope was measured within the linear, early phase of the fEPSP. The fEPSP slope (%) was calculated by normalizing each fEPSP slope value to the mean fEPSP slope during 0-10 min before TBS. In all graphs showing fEPSP slope over time, each data point represents the mean fEPSP slope per minute. The mean LTP magnitude (%) for each experimental group was assessed by averaging the fEPSP slope during 50-60 min after the delivery of TBS.

Electrophysiological fEPSP recordings were acquired at a sampling rate of 10 kHz and low-pass filtered at 500 Hz using an EPC10 amplifier and the PatchMaster software. All electrophysiological data were analyzed and presented using PatchMaster, SigmaPlot 12 (Systat Software Inc., San Jose, CA, USA), and Adobe Illustrator CS5 (Adobe Systems Inc., San Jose, CA, USA). For clarity, the stimulus artefacts in all examples for mPFC fEPSPs were blanked. Recordings were not included in data analysis, if they showed changes (>20%) in the amplitude of presynaptic fiber volley (before and after TBS) or if the level short-term potentiation (first 1 min after TBS) was <150%.

For statistical analysis, SigmaPlot 12 software was used to perform statistical comparisons of LTP levels between independent groups using unpaired Student's t tests (significance level of 0.05).

**Immunohistochemistry.**

To analyze 5-HT7R and CDK5 localization, brains were isolated from wild-type P6 mice and fixed with 4% formaldehyde in PBS, pH 7.3, for 3 days at 4°C and then immersed in 15% sucrose solution in 0.1 M cacodylate buffer, pH 7.3, for 1 day at 4°C, followed by 30% sucrose solution for 1 day at 4°C. Afterwards the tissue was frozen for 2 min in 2-methyl-butane (isopentane, Carl Roth, Karlsruhe, Germany) precooled to -30°C. For sectioning, the brain was attached to a cryostat specimen holder using TissueTek® (Sakura Finetek Europe, Zoeterwoude, Netherlands). Serial frontal brain sections of 20 μm thickness were cut on a cryostat (Microm HM560 M, Microm GmbH, Walldorf, Germany) and picked up on SuperFrost®Plus glass slides (Roth, Karlsruhe, Germany). The sections were air-dried for at least 1 hour at room temperature (RT) and used for immunofluorescence analysis. Immunofluorescence analysis was performed using commercial antibodies at optimal dilutions as described before (Guseva et al., 2009, Brain 132, 2180-95).

) The sections were subjected to antigen retrieval by immersion into 0.01 M citric acid (pH 6.0), heated to 95°C in a water bath for 30 min. Blocking of non-specific binding sites was then performed using PBS containing 0.2% Triton X-100 (Sigma), 0.02% sodium azide (Merck, Darmstadt, Germany) and 5% normal donkey serum for 1 hour at RT. Incubation with primary antibodies against Cdk5 (SCBT, J-3, sc-6247, 1:100) and 5-HT7R (Acris, 24430, 1:100), diluted in PBS containing 0.5% lambda-carrageenan (Sigma) and 0.02% sodium azide, was carried out at 4°C for 3 days. After washing in PBS, appropriate Alexa594- and Alexa488-conjugated secondary antibodies (Jackson ImmunoResearch Europe, Suffolk, UK) diluted 1:200 in PBS-carrageenan solution were applied for 2 hours at RT. After a subsequent washing in PBS, cell nuclei were stained for 10 min at RT with bis-benzimide solution (Hoechst 33258 dye, 5 μg/ml in PBS, Sigma) and the sections were mounted in anti-quenching medium. Specificity of staining was controlled by omitting the primary antibody or replacing it by an equivalent amount of non-immune IgG or serum derived from the same species as the specific antibody. These controls were negative.

To investigate the expression of tau in the mPFC of AAV-injected mice after electrophysiological recordings, coronal 400-

μm-thick mPFC slices (two slices per mouse) were fixed in 4% paraformaldehyde in phosphate buffer (PB, 0.24 M, pH 7.2) for 1-2 hours at room temperature (RT) and cryoprotected using 30% sucrose in PB at 4°C overnight. The sections were then mounted on a CM1950 cryostat (Leica Biosystems) using a tissue freezing medium (Jung, Leica Microsystems), and 35 μm-thick subsections were prepared (5-8 per a 400-μm-slice). After cryo-sectioning, slices were stored in cryo-protective solution (25% glycerin (Carl Roth, Karlsruhe, Germany), 25% ethylene glycol (Carl Roth) in 0.24 M PB) and stained "free-floating" using immunohistochemistry, as described below. After washing in PBS (3x 10 min, at RT), the slices were incubated in blocking solution (10% normal goat serum (NGS, Gibco®, Thermo Fisher Scientific, New Zealand origin) and 0.2% Triton X-100 (Sigma Aldrich) in PBS) at RT for 2 hours to reduce unspecific staining. For staining of phosphorylated tau protein, we used a rabbit anti-tau (phospho T205) primary antibody (ab4841, Abcam, Cambridge, UK; dilution 1:500 in PB containing 5% NGS and 0.2% Triton X-100, at 4°C for 48 hours). Sections were then washed again in PBS (3x 10 min, at RT) and incubated in the secondary antibody (goat anti-rabbit Alexa 546 (Invitrogen), dilution 1:250 in PB containing 5% NGS, at RT for 3 hours). After washing in PB, the slices were stained for DAPI (dilution 1:1,000, at RT for 10 min) and mounted on glass slides using Vectashield medium (Vector Laboratories, Burlingame, CA, USA). Imaging of phospho-tau staining was performed on a confocal laser-scanning microscope (LSM 700, Carl Zeiss, Germany) using 10x and 63x objectives, and the confocal images were analyzed using Zen software (Carl Zeiss, Jena, Germany) and ImageJ 1.46 software (NIH, USA).

**Behavioral experiments.**

In 5-HT7R knockdown experiments, mice were subjected to open field and recent object recognition test ("recency test") one month after AAV injection.

Animals were kindly handled for 3 days before the recency test. One day before the recency test, animals were given 10 minutes to get familiar with the white open field (50 x 50 x 30 cm) test arena.

To evaluate if AAV-eGFP-Tau[R406W] virus injection could influence the locomotor activity of experimental animals and induce anxiety, distance travelled and time spent in central and peripheral (10 cm wide from wall) area of the openfield arena were used as parameter for openfield test.

The recency test comprised two encoding phases followed by a retrieval phase (modified from Nelson et al., Behavioral neuroscience 2011, 125, 396-403). In the first encoding phase, animals were placed into the open field arena and allowed to explore a pair of identical objects for 10 min. Then, a different pair of identical objects was presented in the second encoding phase (10 min total exploration time). During the retrieval phase, two different objects, one object from each encoding phase, were placed into the apparatus and animals were given 10 min to explore them. The intervals between all the phases were 1.5 h.

Exploration time and discrimination ratio were used to evaluate animals' ability for memorizing temporal order of given objects. Exploration was defined as the animal approaching the object at a distance of equal to or less than 2 cm. The behavioral performances of animals were video recorded and analyzed automatically by software (ANY-maze, version 4.99, Stoelting Co., Wood Dale, IL). The animal climbing onto the object with both hind- and forelimbs was not considered as exploration. In the same trial, objects were counterbalanced, and between trials, the placements of novel and familiar objects were changed. The discrimination ratio was calculated as follows: [least recent object time - most recent object time] / [least object time + most recent object time] x 100%.

Animal excluding criteria were the follows: if animals spent in total ≤ 10 s near to both objects, they were excluded from analysis; discrimination ratio measurements were considered as outliers by Grubbs test (GraphPAD QuickCalcs, https://www.graphpad.com/quickcalcs/Grubbs1.cfm). After exclusion, there were 8 mice in eGFP+Veh group, 7 mice in AAV-eGFP-Tau[R406W]+Veh group, and 9 mice in AAV-eGFP-Tau[R406W]+Amisulpride group.

Results are expressed as the mean ± SEM. SigmaPlot 13 (Systat Software, Inc., San Jose, CA, USA) and GraphPad 8 (La Jolla, CA, USA) software were used for data analysis and presentation. Differences between times spent exploring object shown recently and less recently were assessed using a paired Student's t-test for Tau/Veh and Tau/Ami treatment; for GFP/Veh group Wilcoxon Signed Rank Test was applied because the data distribution pattern failed in Normality Test (Shapiro-Wilk). Differences between discrimination ratios of the three treatment groups were transformed into rankings then assessed by one-way ANOVA followed by the Holm-Sidak *post hoc* test. For all comparisons, values of $p < 0.05$ were considered significant.

**Statistical analysis for Western blotting and cellular assay.**

SigmaPlot (Systat Software Inc., Chicago, IL, USA) or GraphPad Prism version 7.00 for Windows (GraphPad Software, La Jolla California USA) was used for statistical evaluation. The data are presented as mean and standard error of the mean (SEM) or standard deviation (SD) of at least three independent experiments. GraphPad Prism version 7.00 for Windows (GraphPad Software, La Jolla California USA) was used for statistical evaluation. Pair-wise datasets were tested for significant differences using unpaired Student's t-test, and group-wise datasets were compared using analysis of

variance (ANOVA) followed by the indicated post hoc test. Indicators of significance correspond to $p < 0.05$ (*), $p < 0.01$ (**), and $p < 0.001$ (***).

**5-HT7R inverse agonist assay.**

The inverse agonist properties of compounds at 5-HT7R were evaluated using their ability to inhibit constitutive 5-HT7R activity in cAMP production in HEK293 cells overexpressing 5-HT7R. Each compound was tested in triplicate at 7 concentrations ($10^{-11}$-$10^{-5}$ M). Cells (transfected with the use of Lipofectamine 2000) were maintained at 37°C in a humidified atmosphere with 5% CO2 and were grown in Dulbecco's Modified Eagle Medium containing 10% dialyzed fetal bovine serum and 500 μg/ml G418 sulfate. For functional experiments, cells were subcultured in 150 $cm^2$ flasks, grown to 90% confluence, washed twice with prewarmed to 37°C phosphate-buffered saline (PBS) and centrifuged for 5 min (160 × g). The supernatant was aspirated and the cell pellet was resuspended in stimulation buffer (1 × HBSS, 5 mM HEPES, 0.5 mM IBMX, 0.1% BSA). Total cAMP was measured using the LANCE cAMP detection kit (PerkinElmer), according to the manufacturer's directions. For cAMP levels quantification, cells (5 μl) were incubated with compounds (5 μl) for 30 min at room temperature in 384-well white opaque microtiter plate. After incubation, the reaction was stopped and cells were lysed by the addition of 10 μl working solution (5 μl Eu-cAMP and 5 μl ULight-anti-cAMP). The assay plate was incubated for 1h at room temperature. Time-resolved fluorescence resonance energy transfer (TR-FRET) was detected by an Infinite M1000 Pro (Tecan) using instrument settings from LANCE cAMP detection kit manual.

**Tau-BiFC Assay.**

To study the effects of 5-HT7R inverse agonists on Tau aggregation, we utilized a HEK293 cell line that stably expresses the Tau Bimolecular Fluorescence Complementation (Tau-BiFC) sensor (Tak et al., 2013, Bimolecular Fluorescence Complementation; Lighting-Up Tau-Tau Interaction in Living Cells 2013, PLOS ONE 8). HEK293 Tau-BiFC cells were maintained in Dulbecco's modified Eagle's medium containing 10% FBS, 100 units/ml penicillin, 100 μg/mL streptomycin, and 100 μg/mL Geneticin (G418, Sigma) at 37 °C in a humidified atmosphere containing 5% CO2. For quantitative high-throughput analysis, cells were plated in μ-clear 96-well plates and transfected either with empty pcDNA vector or pcDNA encoding HA-tagged 5-HT7R. Twenty-four hours post-transfection, cells were stimulated with either DMSO or 50 μM SB-269970 (Tocris), Amisulpride (Tocris), Clozapine (Sigma-Aldrich), Lurasidone (Sigma-Aldrich), Miaserine (TCI), Vortioxetine (Selleckchem) or Tiapride (TCI) for 24 h. To determine the inhibitory effects of Amisulpride on Tau aggregation in a concentration-dependent manner, transfected HEK293 Tau-BiFC cells were stimulated with 0.01, 0.03, 0.1, 0.3, 1, 3, 10, 30, or 100 μM Amisulpride for 24 h. BiFC intensity was automatically imaged by Operetta (PerkinElmer) and analyzed using a Harmony 3.1 software (PerkinElmer).

**Amisulpride administration *in vivo.***

Amisulpride (ab142480, Abcam, Berlin, Germany) was first dissolved to 2 mg/mL stock solution in 100% DMSO and then dissolved to 0.1mg/mL in 0.9% NaCl. Vehicle control was 0.9 % NaCl solution containing 5 % DMSO.
For intraperitoneal injection, animals were given 0.1 ml solution every 10 g body weight. The injection started in three weeks after virus injection and lasted for 16 days until behavioral test. The person who performed the behavioral test was blind to the information about treatments, which was unblinded after statistical analysis was completed.

**Results**

**Example 1: Constitutive 5-HT7R activity induces hyperphosphorylation of tau.**

The ability of tau protein to bind and stabilize microtubules is regulated by its phosphorylation at defined serine, threonine, and tyrosine residues. An increase in phosphorylation results in decreased microtubule binding capacity and destabilization of the microtubule network. In several neurodegenerative diseases, e.g. FTDP-17 and Alzheimer's disease, tau is found to be hyperphosphorylated, leading to an increased tau aggregation, intracellular tau accumulations and formation of neurofibrillary tangles (Maccioni et al., 2001, 2010, Arch. Med. Res. 41, 226-231).

To investigate a possible link between 5-HT7R-mediated signaling and tau phosphorylation, neuroblastoma N1E-115 cells were co-transfected with HA-tagged 5-HT7R and eGFP-tagged Tau[R406W] mutant, which facilitates tau aggregation. It is noteworthy that, under basal conditions, this mutant exhibits similar phosphorylation levels compared to the wild-type tau (Hong et al., 1998, Science 282, 1914; Perez et al., 2000, J. Neurochem. 74, 2583-2589; Sakaue et al., 2005, J. Biol. Chem. 280, 31522-31529). Tau phosphorylation was determined using phospo-specific (Thr181) antibody AT270. As shown in **Figure 1,** co-expression of 5-HT7R results in pronounced tau hyperphosphorylation **(Fig. 1A),** which was accompanied by an increase in the amount of total tau **(Fig. 1B).** Interestingly, these effects were not further promoted

after receptor stimulation with serotonin, suggesting that, even under basal condition, 5-HT7R evokes maximal response. The role of constitutive receptor activity was further supported by the observation that application of the highly selective 5-HT7R inverse agonist SB-269970 prevented both receptor-induced tau accumulation and hyperphosphorylation **(Fig. 1A** and **B).**

The 5-HT7R is coupled to two different heterotrimeric G-proteins, Gs and G12 (Kvachnina, 2005, J. Neurosci. 25, 7821-7830). Because G12 protein is known to activate the tau kinase glycogen synthase kinase 3 beta (GSK3β) (Sayas et al., 2002a, J. Neurosci. 22, 6863-6875), next it was analyzed whether the 5-HT7R/G12 signaling might be involved in the receptor-induced tau hyperphosphorylation. To this aim, short hairpin RNAs (shRNAs) was employed to silence endogenously expressed GαNA subunit **(Fig. 8).** Surprisingly, knockdown of Gα12 did not affect 5-HT7R-induced tau phosphorylation and accumulation **(Fig. 1C** and **D),** suggesting a G12- and/or GSK3β independent pathway.

**Example 2: 5-HT7R-induced tau hyperphosphorylation is mediated by CDK5.**

Phosphorylation of tau can be mediated by tau kinases, GSK3β and CDK5, both of which assumed to be critically involved in the pathogenesis of Alzheimer's Disease. Both kinases recognize different phosphorylation sites within tau with a particular preference to Thr181 (Lund et al., 2001, Neurochem. 76, 1221-1232; Liu et al., 2002, Lett. 530, 209-214). To investigate the role of these kinases in the 5-HT7R-induced tau hyperphosphorylation, N1E-115 cells expressing eGFP-Tau[R406W] alone or together with 5-HT7R were treated overnight either with 20 μM roscovitine, a potent and selective CDK5 inhibitor or with 10 μM SB-216763, a potent and highly selective GSK3β inhibitor. These experiments revealed that, in the absence of 5-HT7R, pharmacological inhibition of GSK3β resulted in a decrease of basal tau phosphorylated and total tau levels **(Fig. 2A** and **B).** However, GSK3β inhibition did not abolish 5-HT7R-mediated increase in tau phosphorylation and accumulation **(Fig. 2A** and **B),** suggesting that receptor signaling towards tau is GSK3β-independent. In case of roscovitine treatment, it was also obtained decreased levels of basal phosphorylation and total tau expression in cells expressing eGFP-Tau[R406W] alone. However, in contrast to results obtained with GSK3β inhibitor, treatment with roscovitine completely abolished 5-HT7R-mediated tau hyperphosphorylation and increase of total tau levels **(Fig. 2A** and **B).** To further evaluate the role of CDK5, the dominant negative CDK5-T33 mutant was used (Nikolic et al., 1996, Genes Dev. 10, 816-825). In line with pharmacological inhibition of CDK5, co-expression of CDK5-T33 mutant together with 5-HT7R and eGFP-Tau[R406W] abolished the 5-HT7R-mediated effects on tau phosphorylation and accumulation **(Fig. 2C** and **D).** These combined results indicate that the observed increase in tau phosphorylation and total tau levels are mediated by the 5-HT7R-induced activation of CDK5 but not GSK3β.

**Example 3: 5-HT7R forms a complex with CDK5.**

CDK5 is known to interact with several neurotransmitter receptors. Therefore, next it was investigated whether the above described signaling involves direct interactions between 5-HT7Rs and CDK5. Co-IP experiments in neuroblastoma N1E-115 cells overexpressing both proteins were performed and interaction between the mCherry-tagged CDK5 (CDK5-mCherry) and the hemagglutinin-tagged 5-HT7R (HA-5-HT7R) was observed **(Fig. 3A).** To rule out unspecificity of the co-IP procedure cells expressing either HA-5-HT7R or CDK5-mCherry as negative controls were analyzed, as well as a mixture of these cells prepared before lysis to examine artificial binding of the two proteins during immunoprecipitation **(Fig. 3A).**

Nevertheless the co-IP procedure can result in artificial oligomerization of proteins. To further verify the specificity of the obtained results a FRET-based approach was applied to analyze the interaction of 5-HT7R and CDK5 in living cells.

FRET efficiencies between eCFP-labeled CDK5 (CDK5-eCFP, donor) and eYFP-labeled 5-HT7Rs (5-HT7R-eYFP, acceptor) were measured in living N1E-115 using the lux-FRET method in combination with total internal reflection fluorescent microscopy (TIRF). This combined approach is capable of detecting physical proximity of individual molecules at the plasma membrane on the nanoscale. Analysis of N1E-115 cells co-expressing CDK5-eCFP and 5-HT7R-eYFP reveal a high apparent FRET efficiency $Ef_{DA}$ **(Fig. 3A** and **B,** $Ef_{DA}$ = 13% + 2%, $n_{Cell}$ = 26) pointing to a close proximity of both proteins. In contrast, cells expressing cytosolic eCFP and eYFP-tagged 5-HT7R (negative control) show significantly lower FRET efficiency (Fig. 3B and C, $Ef_{DA}$ = 2% + 1%, $n_{Cell}$ = 14). As a positive control, 5-HT7R and 5-HT1AR were used, proteins which are known to form heterodimers at the membrane. FRET-TIRF analysis of cells co-expressing 5-HT1A-CFP and 5-HT7R-YFP confirmed receptor heterodimerization **(Fig. 3B** and **C,** $Ef_{DA}$ = 10% + 1%, $n_{Cell}$ = 22). These experiments demonstrate a specific and direct contact of recombinant CDK5 and 5-HT7R at the plasma membrane. Intriguingly, this interaction occurs independently of the 5-HT7R constitutive activity because the application of SB-269970 did not change FRET efficiency **(Fig. 3B** and **C,** $Ef_{DA}$ = 14% + 2%, $n_{Cell}$ = 33).

**Example 4: 5-HT7R causes tau aggregation and tangle formation in neuroblastoma cells.**

To analyze whether the receptor-mediated tau hyperphosphorylation results in increased tau aggregation (which

is a characteristic feature for all tauopathies), first sarkosyl fractionation was applied, an approach developed for biochemical detection of insoluble tau aggregates (Greenberg and Davies, 1990, Proc. Natl. Acad. Sci. 87, 5827-5831; Ren and Sahara, 2013, Front. Neurol. 4). As shown in **Figure 4A,** expression of eGFP-Tau[R406W] mutant in the presence of the 5-HT7R leads to an increase of sarkosyl-insoluble tau, resembling its higher-order oligomers and aggregates. In line with the results presented in **Figure 1,** receptor stimulation with 5-HT does not promote tau aggregation, while treatment with the inverse agonist SB-269970 significantly reduces the amount of insoluble tau aggregates **(Fig. 4A).**

To investigate whether the increased amount of insoluble tau aggregates is accompanied by tangle formation, N1E-115 cells expressing eGFP-Tau[R406W] mutant in the presence or absence of 5-HT7R were analyzed by confocal microscopy. These experiments demonstrated that co-expression of eGFP-Tau[R406W] mutant together with 5-HT7R leads to formation of characteristic tangle-like structures, while most of the cells expressing eGFP-Tau[R406W] alone possess a diffuse GFP fluorescence **(Fig. 9).** For quantitative analysis, fraction of cells with GFP-positive filamentous tangles was calculated from all eGFP-positive cells. The percentage of cells with intracellular tau tangles was significantly increased in cells co-expressing 5-HT7R, while treatment of these cells with the receptor inverse agonist SB-269970 (100 nM) prevented this effect **(Fig. 4B).** Decreased tangle formation was also observed by pharmacological inhibition of CDK5 by roscovitine (20 μM) as well as by co-expression of the dominant-negative CDK5-T33 mutant **(Fig. 4B).** Moreover, analysis of fluorescence intensity profiles across the cell stained with phospho-specific tau antibody AT8 revealed overlay of eGFP-Tau[R406W] and phosphorylated tau only in cells co-expressing 5-HT7R, and not in cells transfected with eGFP-Tau[R406W] alone, although these cells also occasionally contained tangle-like structures resulting from the massive overexpression of Tau[R406W] **(Fig. 4C).** This data suggests that the receptor-mediated tau phosphorylation being observed in neuroblastoma cells is an important co-factor increasing the probability of tau to aggregate and form neurofibrillary tangle-like structures.

It is noteworthy that treatment of cells co-expressing eGFP-Tau[R406W] and 5-HT7R either with SB-269970 (100 nM) or the CDK5 inhibitor roscovitine (20 μM) prevented both tangle formation as well as tau hyperphosphorylation **(Fig 4C).** Taken together, these results demonstrate the importance of 5-HT7R/CDK5-mediated signaling for tau hyperphosphorylation, tau aggregation, and tangle formation.

**Example 5: 5-HT7R/CDK5 signaling regulates tau phosphorylation in primary cortical neurons.**

To investigate the impact of 5-HT7R on tau phosphorylation in neurons, primary cultures of cortical neurons isolated from wild-type and 5-HT7R-deficient mice were established. A time window from DIV4 to DIV6 was selected, when neurons start to develop dendrites and undergo branching. Using phospho-specific antibody AT270, it was demonstrated that phosphorylation of endogenously expressed tau was significantly decreased in 5-HT7R knock-out (KO) neurons, while total tau levels were unaffected **(Fig. 5A** and **B).** Furthermore, treatment of WT neurons with the inverse receptor agonist SB-269970 or with CDK5 inhibitor roscovitine resulted in significantly decreased levels of phosphorylated tau **(Fig. 5C)** without influencing the total tau level **(Fig. 5D),** demonstrating the involvement of 5-HT7R/CDK5 signaling in tau phosphorylation also in primary cortical neurons.

After having observed interaction of the recombinant 5-HT7R and CDK5 in neuroblastoma cells in co-IP experiments as well as nanometer-apposition in Lux-FRET measurements **(Fig. 3),** next it was asked whether the endogenous 5-HT7Rs and CDK5 also show co-localisation in primary cortical neurons. To this aim immunofluorescence stainings using antibodies directed against the 5-HT7R, CDK5 and VGLUT as a presynaptic marker was applied in cultured cortical neurons. The 5-HT7R was partly co-localized with CDK5 at the dendrites and synapses **(Fig. 5E).**

To verify co-localization of endogenous 5-HT7Rs and CDK5 *in situ,* immunohistochemistry was performed in cortical slices prepared from P6 mice. Similarly to results obtained in primary neuronal cultures, 5-HT7Rs and CDK5 were co-localized in the prefrontal cortex **(Fig. 5F).**

To analyze complex-formation between the endogenous 5-HT7R and CDK5, co-IP in cortex lysates prepared from WT and 5-HT7R-deficient mices at postnatal day 6 (P6) was performed, since 5-HT7R expression reaches its maximum approximately in the first postnatal week. These experiments revealed direct interaction of 5-HT7R and CDK5 **(Fig. 5G).**

**Example 6: Inhibition of 5-HT7R/CDK5 signaling reduces tangle formation and apoptosis in neurons overexpressing eGFP-Tau[R406W] mutant.**

Next, a possible effect of 5-HT7R on tau aggregation in neurons was investigated. For this purpose, primary cortical neurons were infected with an adeno-associated virus encoding the eGFP-Tau[R406W] mutant. Treatment of wild-type neurons three days with the inverse receptor agonist SB-269970 (100 nM; SB: 71% + 11%) or with the CDK5 inhibitor roscovitine (20μM; Roscovitine: 62% + 4%) significantly reduced the fraction of tangle-positive neurons compared to control (H2O: 100% + 16%) **(Fig. 6B).** Likewise primary cortical neurons of 5-HT7R Knock-out mice infected with AAV-eGFP-Tau[R406W] display a significantly reduced fraction of tangle-positive neurons compared to control (KO: 60% + 11%) **(Fig. 6B).**

It is noteworthy that tau aggregates were mostly composed by phosphorylated tau as assessed by immunostaining with phosphorylation-specific AT8 antibody **(Fig. 6C).**

Accumulation of tau aggregates in neurons has been shown to correlate with increased levels of apoptosis and cell death (Berger et al., 2007, J. Neurosci. 27, 3650-3662; Roberson et al., 2007, Science 316, 750-754). To elucidate a possible neuroprotective role of 5-HT7R inhibition, primary cortical neurons were infected with an AAV encoding the eGFP-Tau[R406W] and the number of apoptotic cells as fraction of all infected neurons was quantified. Apoptosis was determined by identifying active caspase-3 and 7 **(Fig. 6D).** The fraction of apoptotic neurons was dramatically increased when cells were infected with the eGFP-Tau[R406W] virus compared to neurons infected with AAV encoding cytosolic eGFP (ctrl, **Fig. 6E).** However, application of 5-HT7R inverse agonist SB-269970 as well as CDK5 inhibitor roscovitine reduced apoptosis to the level obtained in control neurons. Taken together, these results demonstrate that tau aggregation as well as tau-mediated neurotoxicity in neurons can be prevented by inhibition of the 5-HT7R/CDK5 signaling.

**Example 7: Knock-down of 5-HT7R rescues LTP impairment induced by overexpression of Tau[R406W] mutant.**

To investigate the effects of tau overexpression on synaptic plasticity in the medial prefrontal cortex (mPFC), AAV-eGFP-Tau[R406W] and control AAV-eGFP viruses were injected into the mPFC of 2-5-months-old C57BL/6J mice **(Fig. 7A, Fig. 10A)** and long-term potentiation (LTP) in the mPFC of these mice 4-6 weeks after AAV injection was recorded. Field excitatory postsynaptic potential (fEPSP) recordings showed a strong reduction of LTP levels in mPFC slices from AAV-eGFP-Tau[R406W]-injected mice (112.62 $\pm$ 5.10%) compared with AAV-eGFP-injected control mice (138.13 $\pm$ 5.28%) **(Fig. 7B** and **C),** suggesting that tau overexpression in the mPFC impaired synaptic plasticity in this brain region.

To assess whether knock-down of the 5-HT7R might restore tau-induced LTP deficits, 2-months-old C57BL/6 mice were co-injected with AAV-eGFP-Tau[R406W] and AAV-5-HT7R-shRNA-eGFP that efficiently reduced 5-HT7R mRNA levels in cortical neurons **(Fig. 10B)** or AAV-scramble-shRNA-eGFP, as a negative control, into the mPFC and LTP in mPFC slices from these mice 4-6 weeks after the AAV injection was recorded. Importantly, LTP levels in AAV-eGFP-Tau [R406W] + AAV-5-HT7R-shRNA-eGFP-injected mice (145.94 $\pm$ 7.26%) were significantly higher than those in mice that were co-injected with AAV-eGFP-Tau[R406W] and AAV- scramble-shRNA-eGFP (99.48 $\pm$ 8.43%) **(Fig. 7B** and **C),** indicating that the knock-down of 5-HT7R could fully rescue the detrimental effects of the Tau[R406W] expression on LTP.

Immunohistochemical analysis of slices collected from the recorded mice confirmed that AAV-eGFP-Tau [R406W] dramatically upregulated tau phosphorylation as compared to AAV-eGFP injected mice **(Fig. 7D).** Moreover, knockdown of 5-HT7R fully normalized tau phosphorylation in AAV-eGFP-Tau[R406W] injected mice, while expression of scrambled shRNA had no effect **(Fig. 7D).** These data together with electrophysiological recordings suggest that 5-HT7R knockdown specifically rescues LTP by abrogating the effects of Tau[R406W] overexpression on tau phosphorylation.

**Example 8: Knock-down of 5-HT7R rescues memory impairment induced by overexpression of Tau[R406W] mutant in the recent object recognition test.**

To assess whether the histopathological and electrophysiological alterations observed in cell culture and brain slices also lead to behavioral abnormalities *in vivo,* either AAV-eGFP or AAV-eGFP-Tau[R406W] were injected together with AAV-scramble-shRNA-eGFP or AAV-5-HT7R-shRNA-eGFP into the mPFC of 10-week-old C57BL/6J mice and these mice were subjected to different behavioural tests. The open field test did not reveal significant differences between the groups for the distance travelled in the arena or the time spent either in the center or periphery (**Figure 10E** and **F**) indicating that virus injection does not influence general locomotor activity and the level of anxiety. Furthermore, the recent object recognition test was performed, for which it is known that mPFC plays an important role (Gareth et al., 2007, J. Neuro. 27 (11) 2948-2957; Nelson et al., 2011, Behavioral neuroscience. 125, 396-403). In this test, a mouse can choose between two previously seen objects and normally spends less time at the most recently seen object (**Fig. 7E**). This was the case for mice co-injected with AAV-eGFP and AAV-scramble-shRNA-eGFP (control group), which spend 30 + 3% and 54 + 4% of time near the most recent and less recent objects, respectively (**Fig. 7E**). However, mice co-injected with AAV-eGFP-Tau[R406W] and AAV-scramble-shRNA-eGFP were impaired in recognition, spending similar times at both objects (36 + 3% and 42 + 3%). The preference to stay at the less recent object can be quantified using the object discrimination index, which was close to 0 for the AAV-eGFP-Tau[R406W] + AAV-scramble-shRNA-eGFP group and significantly lower than for control group, therefore indicating impaired mPFC function due to the overexpression of Tau[R406W]. Strikingly, co-infection of AAV-eGFP-Tau[R406W] with AAV-5-HT7R-shRNA-eGFP increased the time spent at the less recent object relative to the most recent object (45 + 4% and 30 + 2%, respectively) and normalized the value of discrimination index (**Fig. 7E**). Taken together these results indicate, that the detrimental effects of eGFP-Tau[R406W] expression in the mPFC on

memory function can be fully prevented by knockdown of 5-HT7R.

**Example 9: 5-HT7R inverse agonists decrease 5-HT7R-induced Tau aggregation in HEK293 Tau-BiFCs.**

To determine which substance of the selected group of 5-HT7R inverse agonists (SB-269970, Amisulpride, Vortioxetine, Mianserin, Clozapine, Lurasidone) has the highest potential to inhibit 5-HT7R constitutive activity, we treated HEK 293 cells expressing the 5-HT7R with different concentrations of these drugs and measured their effects on basal cAMP production. These experiments revealed that SB-269970, Amisulpride, Lurasidone, Mianserin, Clozapine act as 5-HT7R inverse agonists, while Vortioxetine is a 5-HT7R antagonist. Table 1 shows the calculated half-maximal effective concentrations for inhibition of basal cAMP production ($IC_{50}$). SB-269970 is the most potent 5-HT7R inverse agonist. While the $IC_{50}$ value of Amisulpride is in a similar range as SB-269970, Clozapine, Mianserine and Lurasidone exhibit $IC_{50}$ values that are two orders of magnitude higher.

**Table 1: $IC_{50}$ values of 5-HT7R inverse agonists.**

| 5-HT7R inverse agonist | $IC_{50}$ (nM) |
|---|---|
| SB- 269970 | 11 ± 2 |
| Amisulpride | 73 ± 19 |
| Mianserin | 331 ± 9 |
| Clozapine | 225 ± 21 |
| Lurasidone | 445 ± 241 |
| Vorteoxetin | n.d. |
| Tiaprid | n.d. |

In order to study the potential of Amisulpride, Lurasidone, Mianserin, and Clozapine in preventing Tau aggregation, we utilized the HEK293 Tau-BiFC (Bimolecular Fluorescence Complementation) sensor cells (Tak et al., Bimolecular Fluorescence Complementation; Lighting-Up Tau-Tau Interaction in Living Cells 2013, PLOS ONE 8). This cell line stably expresses human Tau fused either to the non-fluorescent N- or C-terminal part of a Venus protein. While non-aggregated Tau does not exhibit any Venus fluorescence, Venus fluorescence becomes visible upon Tau aggregation. HEK293 Tau-BiFC cells were transfected with pcDNA or vector encoding 5-HT7R and stimulated with the indicated drugs for 24 h (**Fig. 11A**). 5-HT7R expression induced a more than 5-fold increase in BiFC fluorescence reflecting strong Tau aggregation (**Fig. 11B**). Treatment with Amisulpride, Lurasidone, Mianserin, and Clozapine significantly decreased Tau aggregation in 5-HT7R-expressing cells in the same extent as SB-269970. However, Tiapride, an anti-hyperkinetic drug that does not possess 5-HT7R affinity (**Table 1**), did not prevent 5-HT7R-induced Tau aggregation. Furthermore, we investigated whether increased Tau BiFC fluorescence correlates with Tau tangle formation. While HEK293 Tau-BiFC cells transfected with a control vector encoding mCerulean exhibit almost no Venus fluorescence, 5-HT7R-expressing cells show increased Venus fluorescence and obvious tangle-like structures. Upon treatment with SB-269970 and Amisulpride, the formation of Tau tangles was prevented (**Fig. 11C**). Next, we studied the effect of these 5-HT7R inverse agonists on Tau accumulation via Western blot with a total Tau antibody that detects both forms of the recombinant Tau, Tau tagged with the Venus N-terminus (VN 173) as well as Tau tagged with the Venus C-Terminus (VC 155). The 5-HT7R inverse agonists Amisulpride, Lurasidone, Mianserin, and Clozapine significantly reduced 5-HT7R-induced increase in total Tau levels, while Vortioxetine and Tiapride show no effect (**Fig. 11D**). Noteworthy that in contrast to Lurasidone, Mianserin, and Clozapine, Amisulpride does not negatively influence cell viability (data not shown). For that reason, we decided to study the effects of Amisulpride in preventing Tau pathology in more detail and determined its dose-response curve (**Fig. 11E**). The half-maximal inhibitory concentration ($IC_{50}$) of Amisulpride in Tau-BiFCs HEK293 for preventing of Tau aggregation was 1.47 μM**.**

**Example 12: 5-HT7R-induced Tau tangle formation and apoptosis in eGFP-Tau[R406W]-infected primary cortical neurons** is **reduced by Amisulpride.**

We also validated the therapeutic effects of Amisulpride using the mouse cortical neurons overexpressing the human Tau[R406W] mutant as a model. To this end, primary neurons derived from the prefrontal cortex of mice were infected at DIV 10 with AAV-eGFP-Tau[R406W] and treated with either $H_2O$, SB-269970, Amisulpride, Vortioxetine or Tiapride for three days (**Fig. 12A**). To study the treatment effect on Tau accumulation and tangle formation, we performed live-cell imaging and counted the neurons with obvious eGFP accumulation and fibrillary structures in the soma as tangle-positive (**Fig. 12B**). Quantification shows that application of SB-269970 and Amisulpride reduced the number of tangle-positive neurons by about 60 % (**Fig. 12C**). Vortioxetine and Tiapride, which do not exhibit 5-HT7R inverse agonism properties (**Table 1**), did not prevent the formation of Tau tangles. In line with this, in sarkosyl fractionation experiments, most eGFP-Tau[R406W] protein moves to the soluble fraction when neurons were treated with SB-269970 and Amisulpride. Furthermore, eGFP-Tau[R406W] levels in both the sarkosyl-soluble and sarkosyl-insoluble fractions were reduced in neurons after application of SB-269970 and Amisulpride compared to neurons treated with $H_2O$, Vortioxetine or Tiapride (**Fig. 12D**). Moreover, treatment of neurons with Amisulpride also significantly reduced the number of apoptotic cells by about 60 % (**Fig. 12E**).

Reference

**Example 13: Tau pathology *in vivo* can be ameliorated by treatment with Amisulpride.**

Finally, we evaluated the effects of Amisulpride *in vivo.* To this end, 500 nl AAV-eGFP-Tau[R406W] particles were injected into the prefrontal cortex (PFC) of mice. After three weeks of recovery time, animals were injected intraperitoneally with Amisulpride or vehicle every day at a dosage of 1 mg/kg over a period of 16 days (**Fig. 13A**). To investigate whether Amisulpride can improve AAV-eGFP-Tau[R406W]-induced cognitive deficits, we applied a test for temporal order recognition memory (recency test). Amisulpride-treated animals spent more time with the less recent object (38.3 $\pm$3.3 s v.s. 18.1$\pm$2.4 s, *P*<0.01) and exhibited a higher discrimination index (32.6$\pm$8.9 %) similar to that obtained in the control mice (GFP-AAV + vehicle) (28.4$\pm$7.9 %), indicating that Amisulpride rescues the pathological memory impairment induced by eGFP-Tau[R406W] mutant *in vivo* (-0.3$\pm$10.3 %) (**Fig. 13D**). The levels of phosphorylated Tau were then evaluated in the PFC slices from these animals using immunohistochemistry with phospho-Tau specific antibodies. While the Tau phosphorylation was robustly increased within the injection site in mice treated with vehicle, the phospho-Tau signal in Amisulpride-treated animals was reduced (**Fig. 13B**). Accordingly, quantification of confocal images revealed significantly decreased pTau/eGFP-Tau[R406W] ratio after Amisulpride application (**Fig. 13C**).

## Claims

1. A 5-HT7 receptor antagonist for use in preventing or treating a tauopathy by prevention of both the receptor-induced phosphorylation and accumulation of Tau proteins,

   wherein the 5-HT7 receptor antagonist has a structure according to the following formula (I),

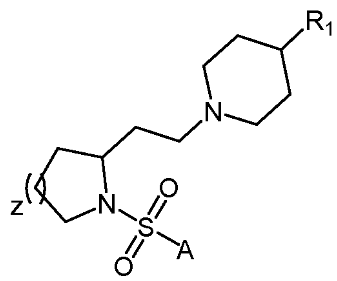

(I)

   wherein in formula (I)
   $R_1$ is -($C_1$-$C_6$)alkyl, preferably methyl, ($C_6$-$C_{10}$)aryl, -O($C_6$-$C_{10}$)aryl, or ($C_5$-$C_{10}$)heteroaryl,
   which are optionally substituted with one or more substituents independently selected from the group consisting of: halogen, preferably -Cl, or ($C_1$-$C_6$)Alkyl;
   A is ($C_6$-$C_{10}$)aryl, or ($C_5$-$C_{10}$)heteroaryl,
   which are optionally substituted with one or more substituents independently selected from the group consisting of: halogen, -OH, ($C_1$-$C_6$)alkyl, or -O($C_1$-$C_6$)alkyl, preferably the at least one substituent is a meta-substituent;
   z is 1 ;
   or a pharmaceutically acceptable salt, enantiomer, diastereomer, racemic mixture, crystalline form, amorphous, unsolved form or solvate of the general formula (I).

2. The5-HT7 receptor antagonist for use of claim 1, wherein the 5-HT7 receptor antagonist is SB-269970.

3. The 5-HT7 receptor antagonist for use of any one of the preceding claims, wherein the antagonist is a small organic molecule comprising at least two carbon atoms having a molecular weight in the range between 100 and 1000 Dalton.

4. A pharmaceutical composition for use in preventing or treating a tauopathy by prevention of both the receptor-induced phosphorylation and accumulation of Tau proteins, comprising the 5-HT7 receptor antagonist according to any one of the claims 1 to 3.

5. The 5-HT7 receptor antagonist for use according to any one of the claims 1 to 3, or the pharmaceutical composition for use of claim 4, wherein tauopathy is dementia-associated tauopathy.

6. The 5-HT7 receptor antagonist for use according to any one of the claims 1 to 3, or the pharmaceutical composition for use of claim 4, wherein tauopathy is selected from the group consisting of Alzheimer's disease, frontotemporal dementia, primary age-related tauopathy (PART), chronic traumatic encephalopathy, progressive supranuclear palsy (PSP), corticobasal degeneration, dementia with Lewy Bodies (DLB), frontotemporal dementia and parkinsonism linked to chromosome 17 (FTDP-17), argyrophilic grain disease (AGD), Huntington disease, glial globular tauopathy, amyotrophic lateral sclerosis (ALS), Parkinson's disease, spinal muscular atrophy (SMA), cerebral amyloid angiopathy (CAA).

## Patentansprüche

1. 5-HT7-Rezeptorantagonist zur Verwendung in der Vorbeugung oder Behandlung einer Tauopathie durch Verhinderung sowohl der Rezeptor-induzierten Phosphorylierung als auch der Akkumulation von Tau-Proteinen,

wobei der 5-HT7-Rezeptorantagonist eine Struktur gemäß der folgenden Formel (I) aufweist,

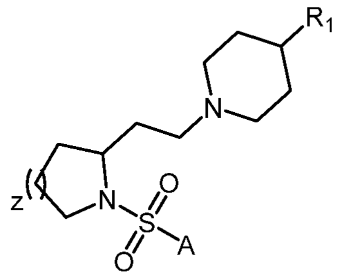

(I)

wobei in der Formel (I)
$R_1$ ist -($C_1$-$C_6$)alkyl, vorzugsweise Methyl, ($C_6$-$C_{10}$)aryl, -O($C_6$-$C_{10}$)aryl oder ($C_5$-$C_{10}$)heteroaryl,
die optional mit einem oder mehreren Substituenten substituiert sind, die unabhängig voneinander aus der Gruppe bestehend aus Halogen, vorzugsweise -Cl oder ($C_1$-$C_6$)Alkyl ausgewählt sind;
A ist ($C_6$-$C_{10}$)aryl oder ($C_5$-$C_{10}$)heteroaryl,
die optional mit einem oder mehreren Substituenten substituiert sind, die unabhängig voneinander ausgewählt sind aus der Gruppe bestehend aus: Halogen, -OH, ($C_1$-$C_6$)alkyl oder -O($C_1$-$C_6$)alkyl, vorzugsweise ist der mindestens eine Substituent ein Meta-Substituent;
z ist 1;
oder ein pharmazeutisch verträgliches Salz, Enantiomer, Diastereomer, racemisches Gemisch, kristalline Form, amorphe, ungelöste Form oder Solvat der allgemeinen Formel (I).

2. 5-HT7-Rezeptorantagonist zur Verwendung nach Anspruch 1, wobei der 5-HT7-Rezeptorantagonist SB-269970 ist.

3. 5-HT7-Rezeptorantagonist zur Verwendung eines der vorhergehenden Ansprüche, wobei der Antagonist ein kleines organisches Molekül ist, das mindestens zwei Kohlenstoffatome mit einem Molekulargewicht im Bereich zwischen 100 und 1000 Dalton umfasst.

4. Pharmazeutische Zusammensetzung zur Verwendung in der Vorbeugung oder Behandlung einer Tauopathie durch Verhinderung sowohl der Rezeptor-induzierten Phosphorylierung als auch der Akkumulation von Tau-Proteinen,

umfassend den 5-HT7-Rezeptorantagonisten gemäß einem der Ansprüche 1 bis 3.

5. 5-HT7-Rezeptorantagonist zur Verwendung gemäß einem der Ansprüche 1 bis 3 oder die pharmazeutische Zusammensetzung zur Verwendung nach Anspruch 4, wobei Tauopathie eine Demenz-assoziierte Tauopathie ist.

6. 5-HT7-Rezeptorantagonist zur Verwendung nach einem der Ansprüche 1 bis 3 oder die pharmazeutische Zusammensetzung zur Verwendung nach Anspruch 4, wobei die Tauopathie ausgewählt ist aus der Gruppe bestehend aus, Alzheimer-Krankheit, frontotemporale Demenz, primäre altersbedingte Tauopathie (PART), chronische traumatische Enzephalopathie, progressive supranukleäre Lähmung (PSP), kortikobasale Degeneration, Demenz mit Lewy-Körpern (DLB), frontotemporal Demenz und Parkinsonismus in Verbindung mit Chromosom 17 (FTDP-17), argyrophile Getreidekrankheit (AGD), Huntington-Krankheit, gliale globuläre Tauopathie, amyotrophe Lateralsklerose (ALS), Parkinson-Krankheit, spinale Muskelatrophie (SMA), zerebrale Amyloid-Angiopathie (CAA).

## Revendications

1. Antagoniste du récepteur 5-HT7 destiné à la prévention ou au traitement d'une tauopathie par prévention de la phosphorylation induite par le récepteur et de l'accumulation de protéines Tau,

   dans lequel l'antagoniste du récepteur 5-HT7 a une structure selon la formule suivante (I),

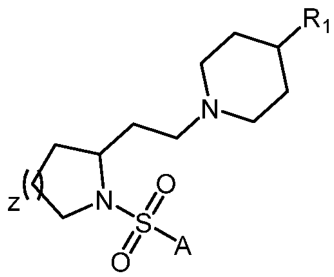

(I)

   où dans la formule (I)
   R1 est -(C1-$C_6$)alkyle, de préférence méthyle, (C6-C10)aryle, -O(C6-C10)aryle ou (C5-C10)hétéroaryle, qui sont éventuellement substitués par un ou plusieurs substituants choisis indépendamment dans le groupe constitué de : halogène, de préférence -Cl, ou (C1-$C_6$)alkyle ;
   A est (C6-$C_{10}$)aryle, ou (C 5-C10)hétéroaryle,
   qui sont éventuellement substitués par un ou plusieurs substituants choisis indépendamment dans le groupe constitué de : halogène, -OH, (C1-$C_6$)alkyle ou -O(C 1-C6)alkyle, de préférence le ou les substituants sont des méta-substituants ;
   z est 1 ;
   ou un sel, un énantiomère, un diastéréoisomère, un mélange racémique, une forme cristalline, une forme amorphe, une forme non résolue ou un solvate de formule générale (I) pharmaceutiquement acceptable.

2. Antagoniste du récepteur 5-HT7 pour l'utilisation de la revendication 1, dans lequel l'antagoniste du récepteur 5-HT7 est le SB-269970.

3. Antagoniste du récepteur 5-HT7 pour l'utilisation de l'une quelconque des revendications précédentes, dans lequel l'antagoniste est une petite molécule organique comprenant au moins deux atomes de carbone ayant un poids moléculaire compris entre 100 et 1000 Dalton.

4. Composition pharmaceutique destinée à être utilisée dans la prévention ou le traitement d'une tauopathie par prévention de la phosphorylation et de l'accumulation de protéines Tau induites par le récepteur, comprenant l'antagoniste du récepteur 5-HT7 selon l'une quelconque des revendications 1 à 3.

5. Antagoniste du récepteur 5-HT7 destiné à être utilisé selon l'une quelconque des revendications 1 à 3, ou composition pharmaceutique destinée à l'utilisation de la revendication 4, dans laquelle la tauopathie est une tauopathie associée

à la démence.

6. Antagoniste des récepteurs 5-HT7 destiné à être utilisé selon l'une quelconque des revendications 1 à 3, ou composition pharmaceutique destinée à l'utilisation selon la revendication 4, dans lequel la tauopathie est choisie dans le groupe constitué par la maladie d'Alzheimer, la démence frontotemporale, la tauopathie primitive liée à l'âge (PART), l'encéphalopathie traumatique chronique, la paralysie supranucléaire progressive (PSP), la dégénérescence corticobasale, la démence à corps de Lewy (DLB), démence frontotemporale et parkinsonisme liés au chromosome 17 (FTDP-17), maladie des grains argyrophiles (AGD), maladie de Huntington, tauopathie globulaire gliale, sclérose latérale amyotrophique (SLA), maladie de Parkinson, amyotrophie spinale (SMA), angiopathie amyloïde cérébrale (AAC).

Figure 1

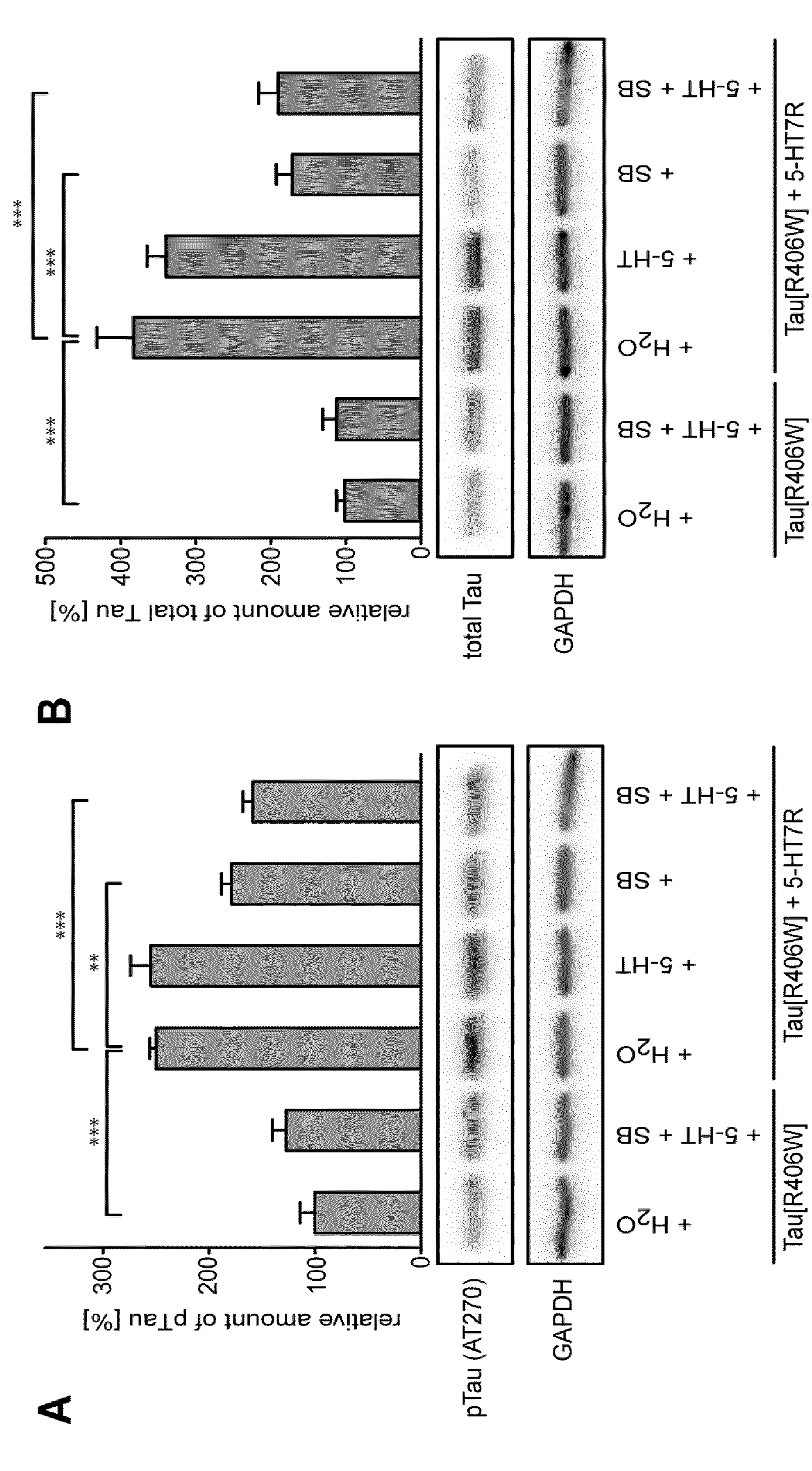
A
relative amount of pTau [%]
0
100
200
300
**
***
pTau (AT270)
GAPDH
+ H2O
+ 5-HT + SB
+ 5-HT
+ SB
Tau[R406W]
Tau[R406W] + 5-HT7R
B
relative amount of total Tau [%]
400
500
total Tau

Figure 1 (cont.)

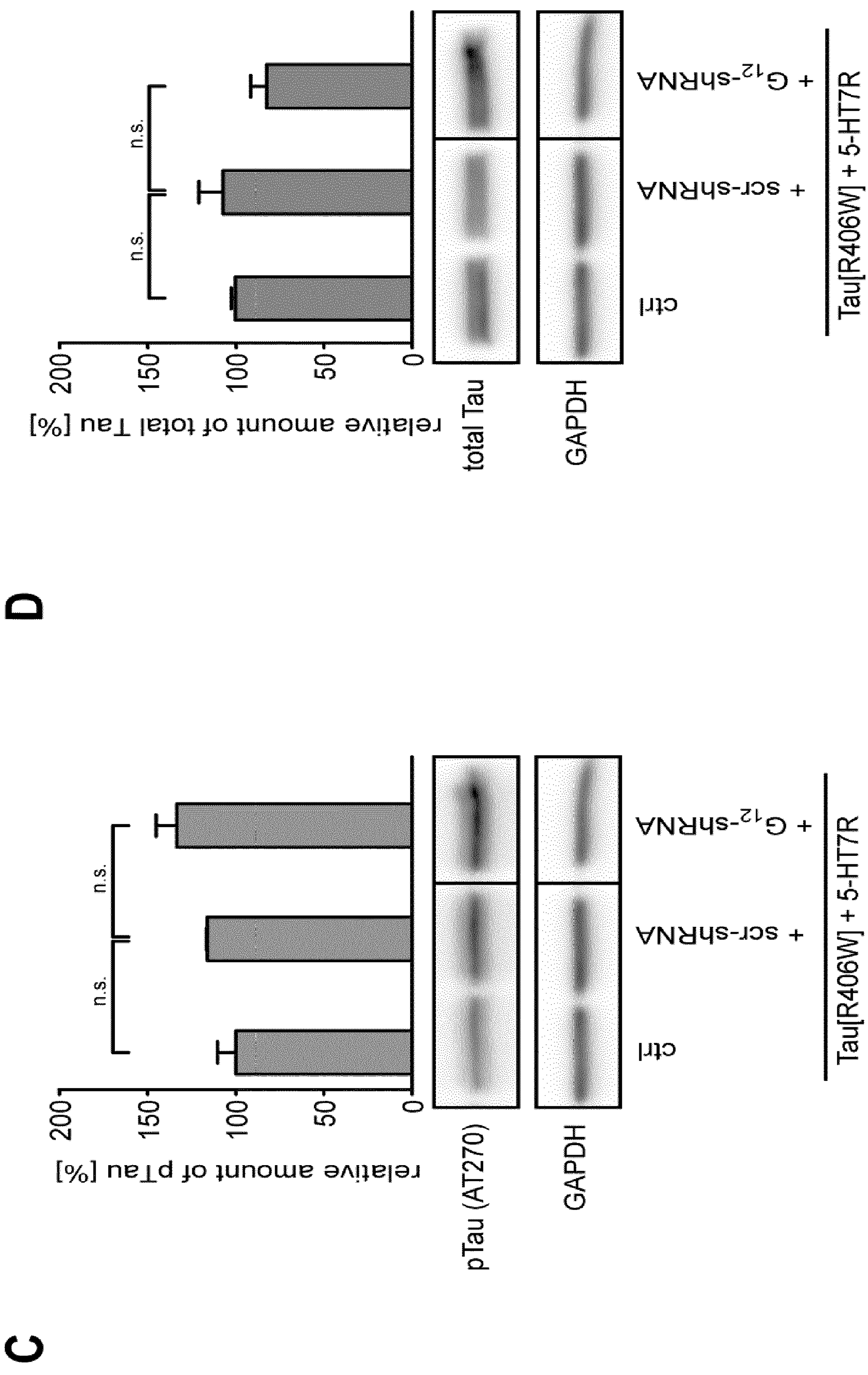

Figure 2

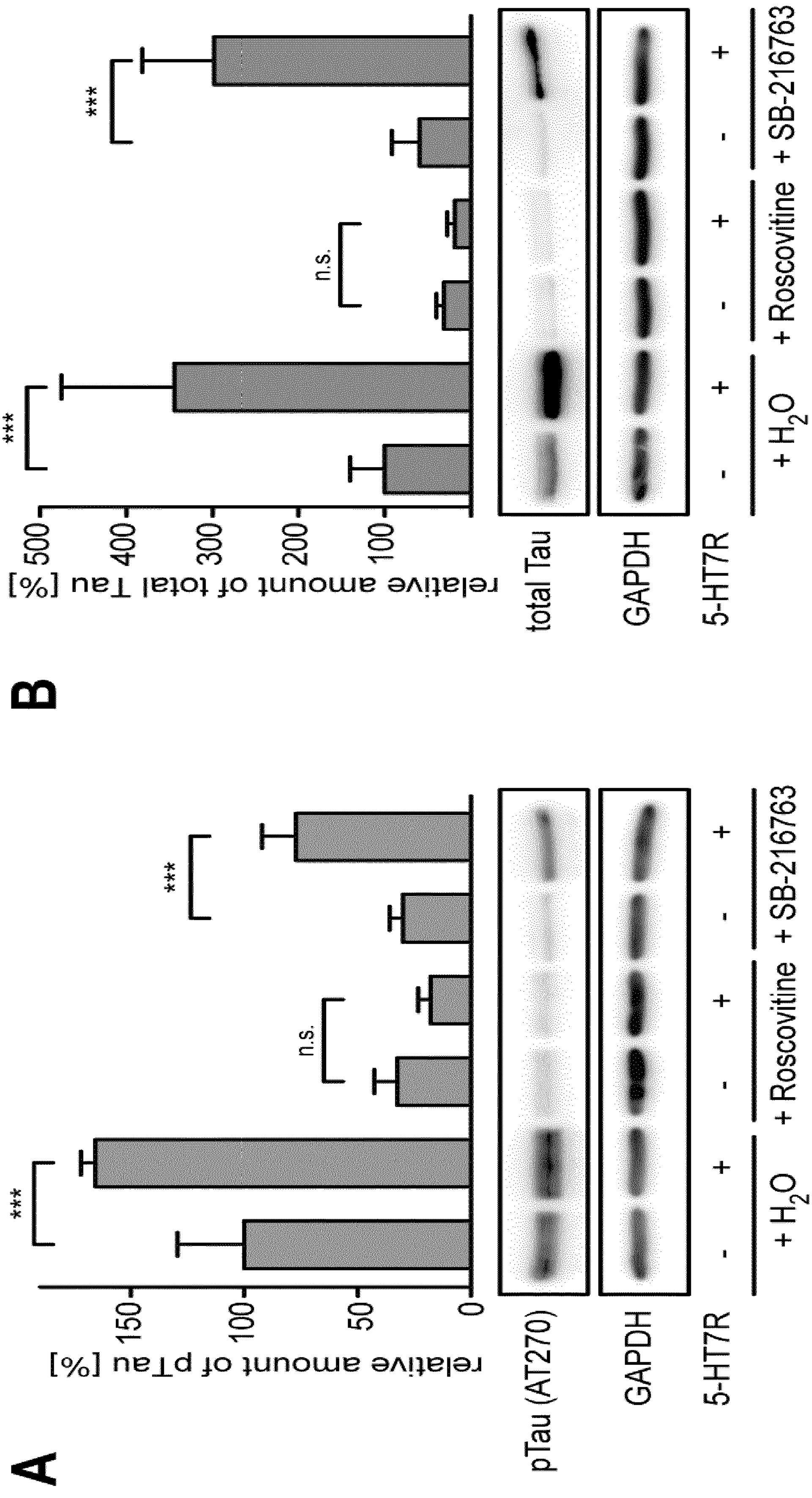
A
B
relative amount of pTau [%]
relative amount of total Tau [%]
pTau (AT270)
total Tau
GAPDH
5-HT7R
+ H2O
+ Roscovitine
+ SB-216763
n.s.
***

Figure 2 (cont.)

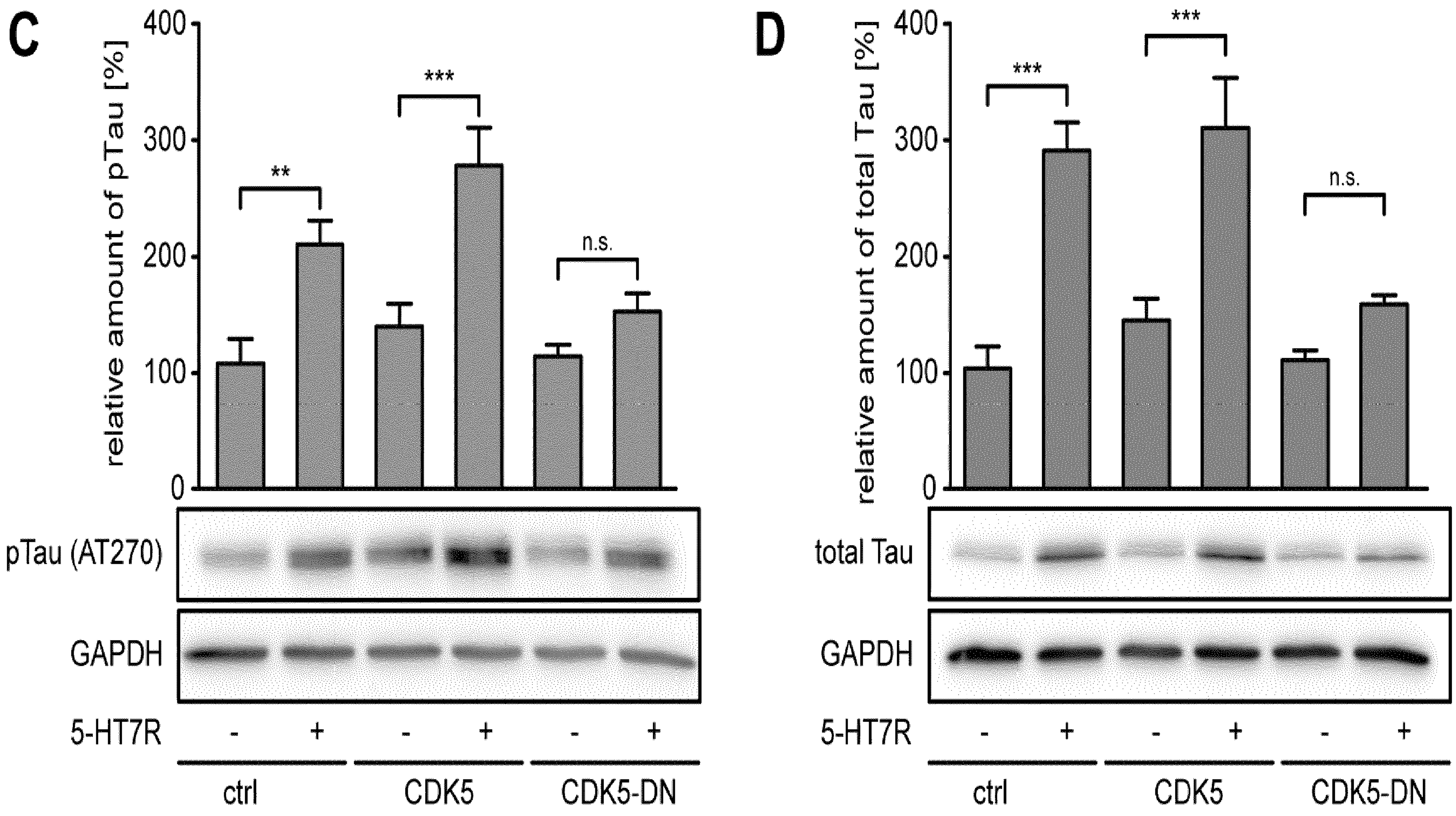

Figure 3

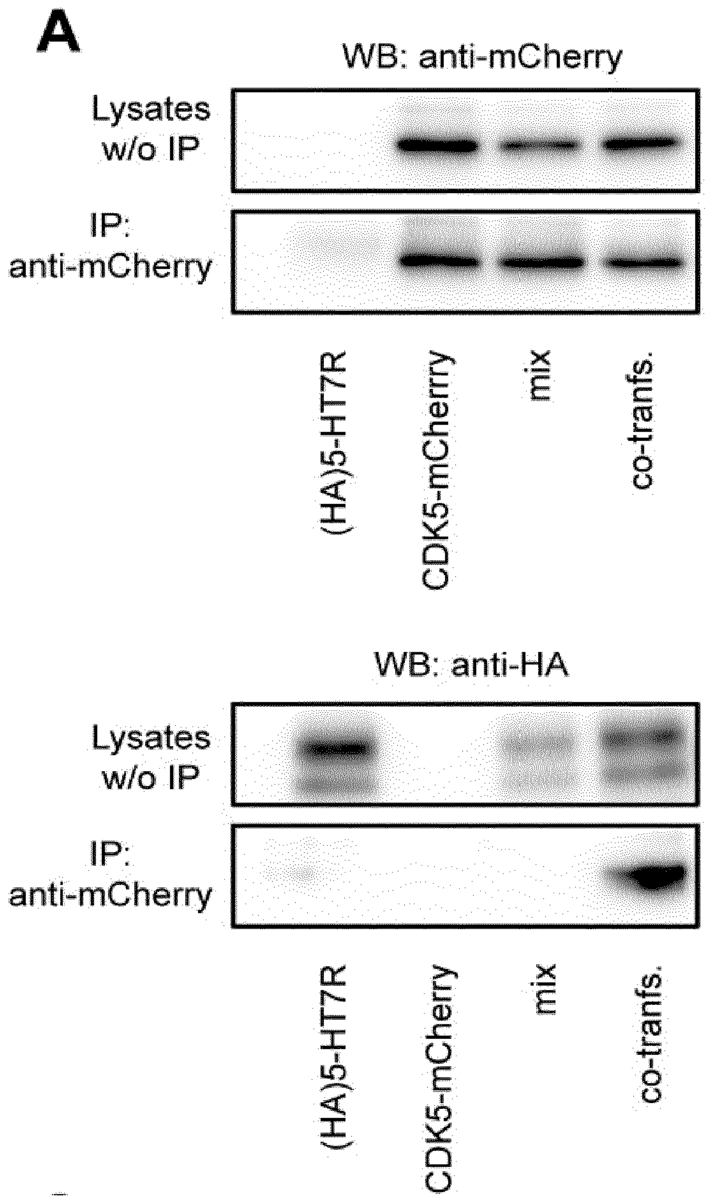
A
WB: anti-mCherry
Lysates w/o IP
IP: anti-mCherry
(HA)5-HT7R
CDK5-mCherry
mix
co-tranfs.
WB: anti-HA
Lysates w/o IP
IP: anti-mCherry
(HA)5-HT7R
CDK5-mCherry
mix
co-tranfs.

Figure 3 (cont.)

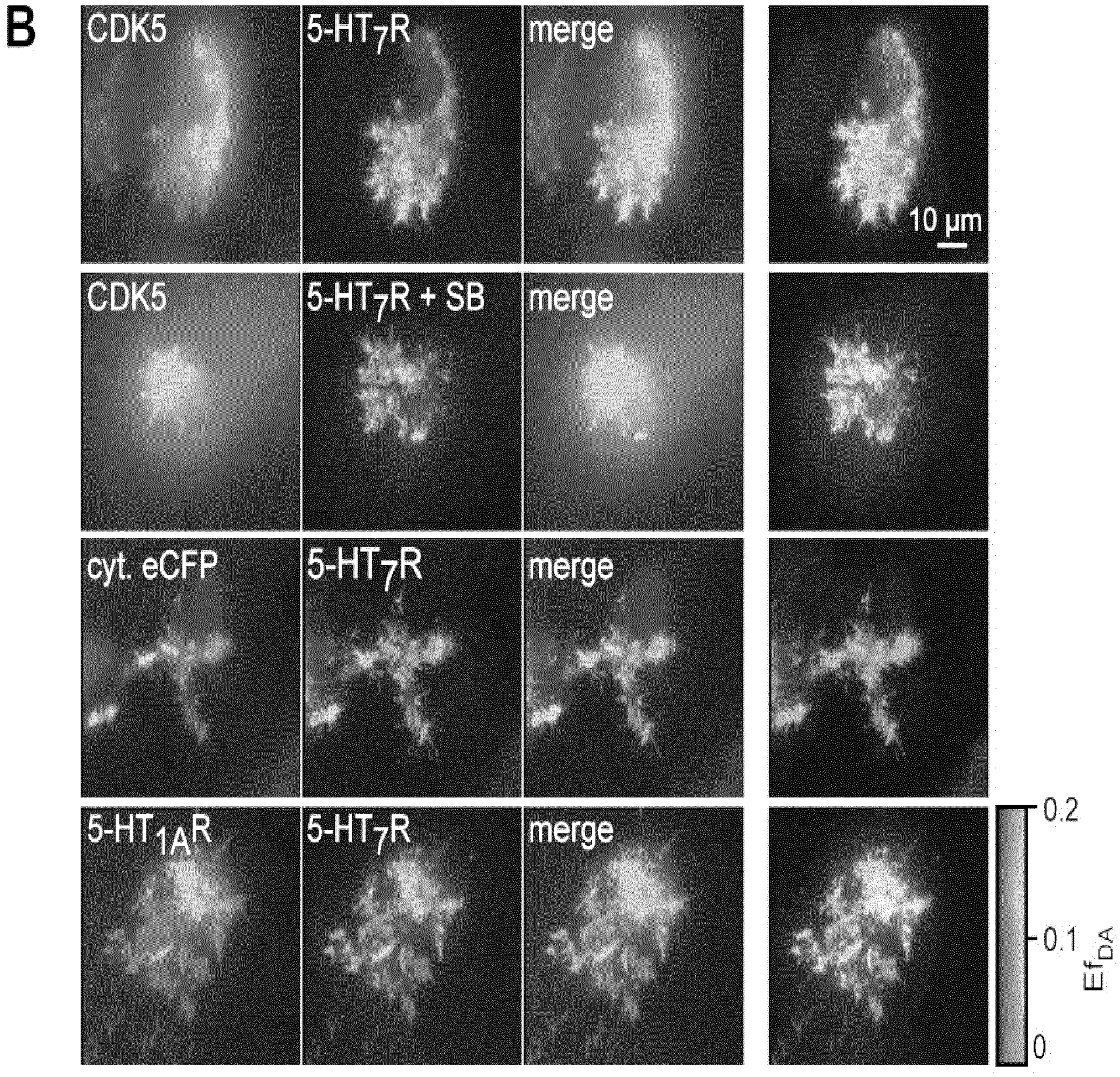

Figure 3 (cont.)

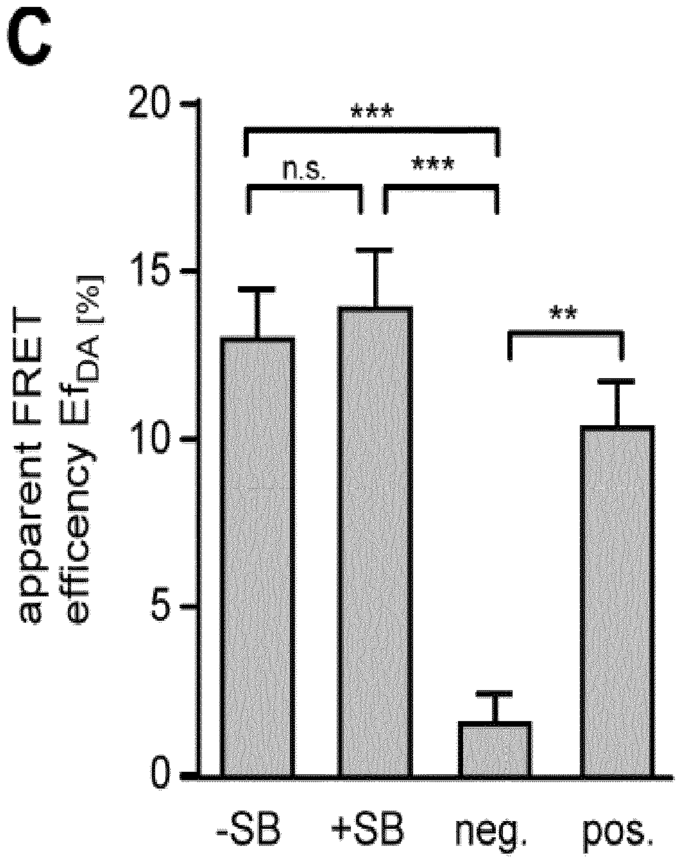

Figure 4

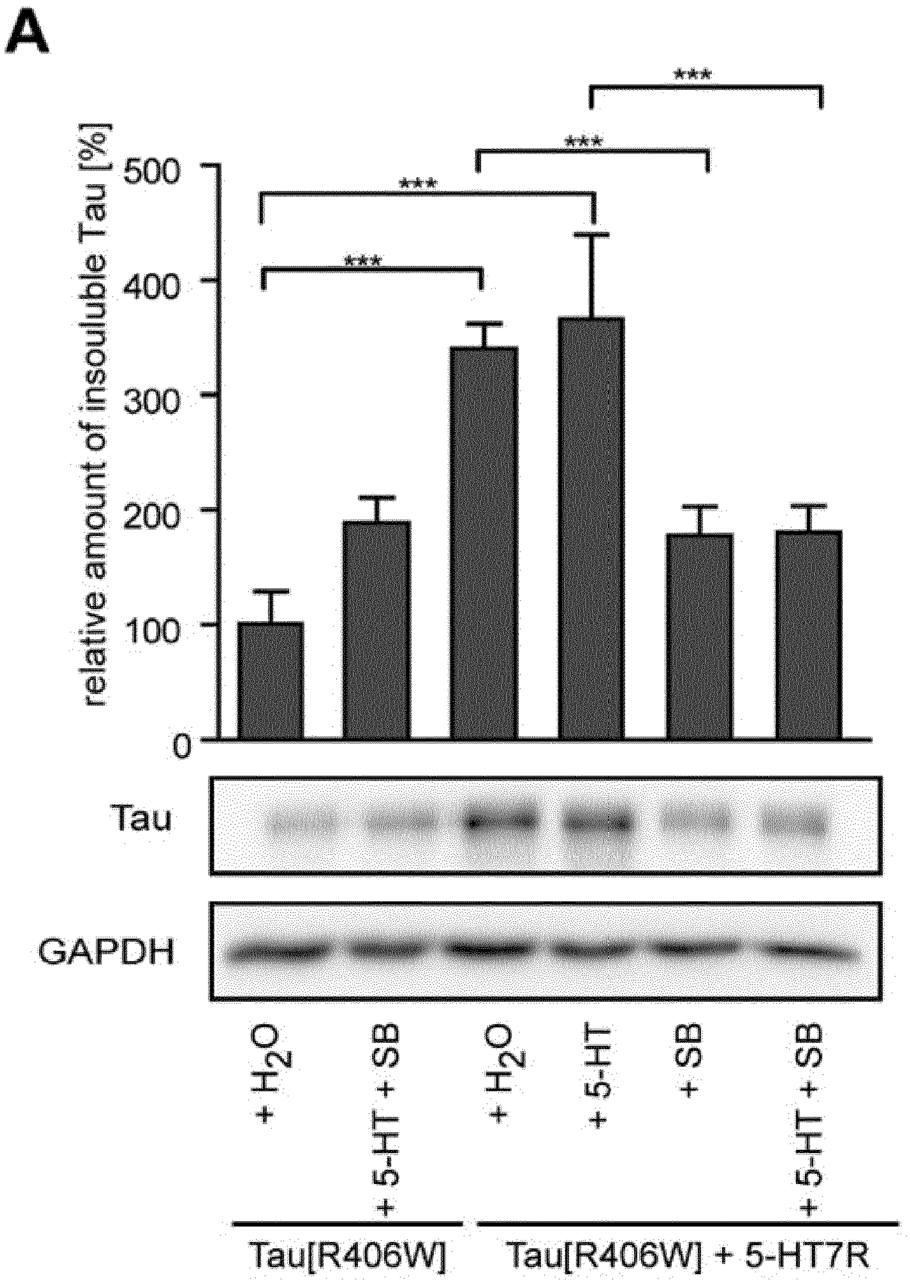
A
***
***
***
***
relative amount of insouluble Tau [%]
500
400
300
200
100
0
Tau
GAPDH
+ H2O
+ 5-HT + SB
+ H2O
+ 5-HT
+ SB
+ 5-HT + SB
Tau[R406W]
Tau[R406W] + 5-HT7R

Figure 4 (cont.)

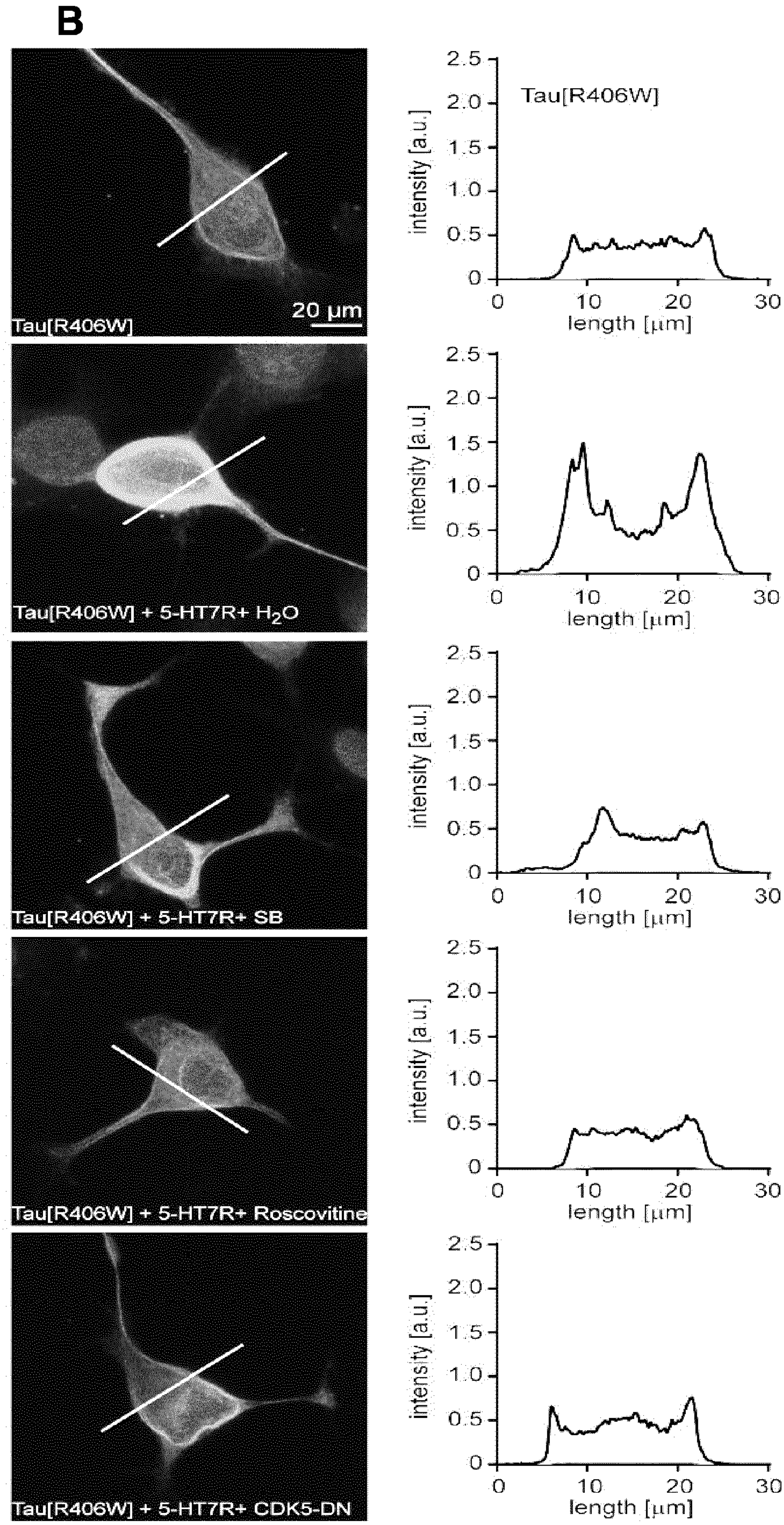

**Figure 4 (cont.)**

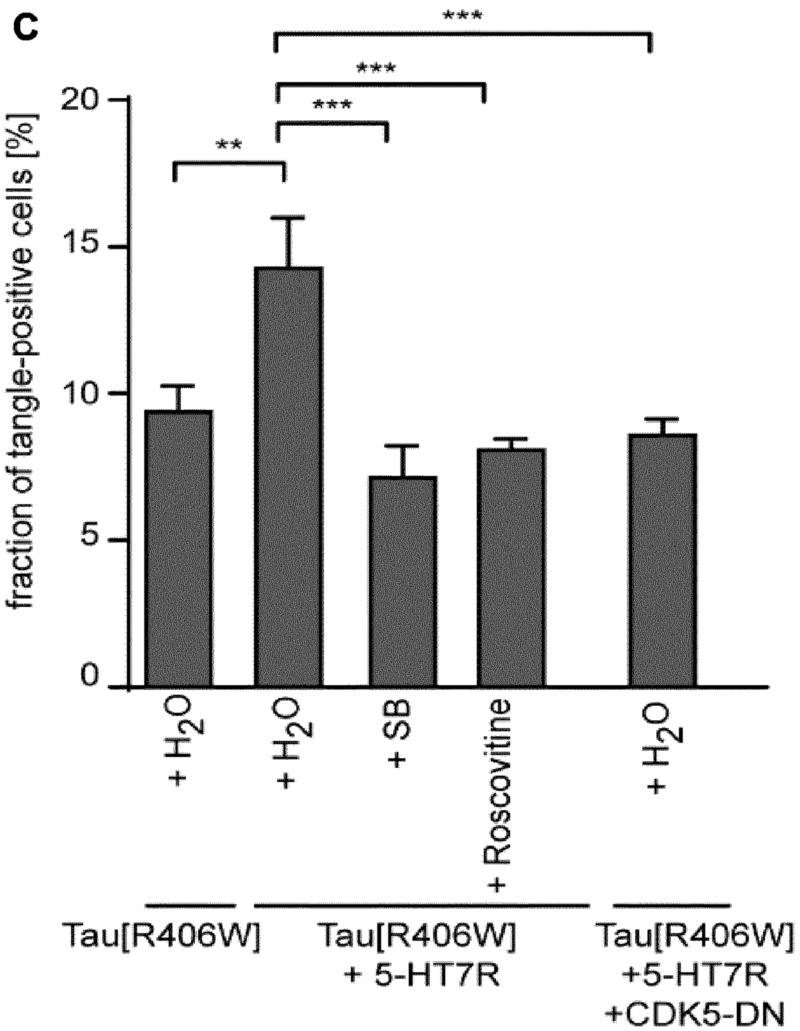

Figure 5

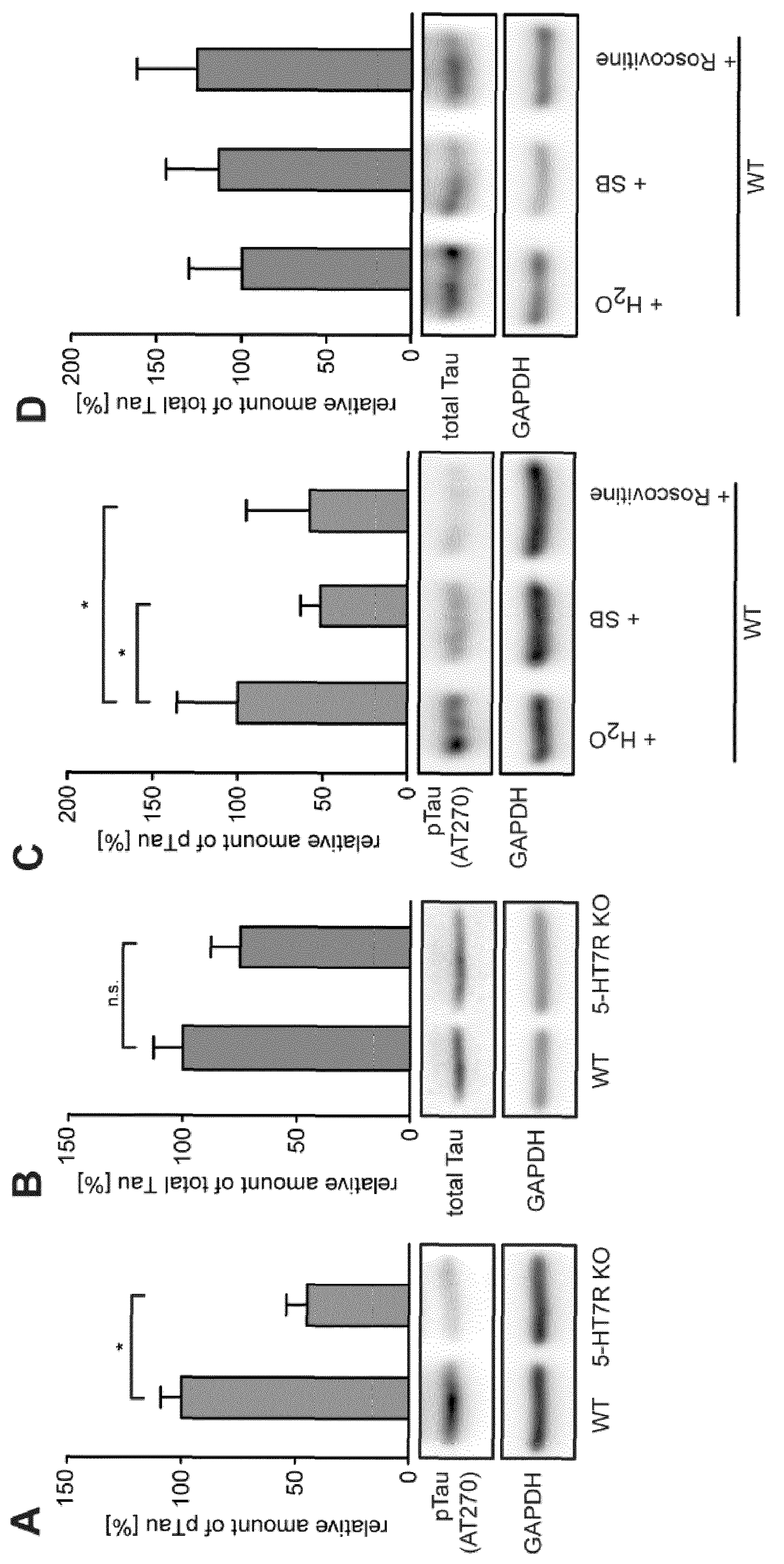
A
relative amount of pTau [%]
150
100
50
0
*
pTau (AT270)
GAPDH
WT
5-HT7R KO
B
relative amount of total Tau [%]
150
100
50
0
n.s.
total Tau
GAPDH
WT
5-HT7R KO
C
relative amount of pTau [%]
200
150
100
50
0
*
*
pTau (AT270)
GAPDH
+ $H_2O$
+ SB
+ Roscovitine
WT
D
relative amount of total Tau [%]
200
150
100
50
0
total Tau
GAPDH
+ $H_2O$
+ SB
+ Roscovitine
WT

Figure 5 (cont.)

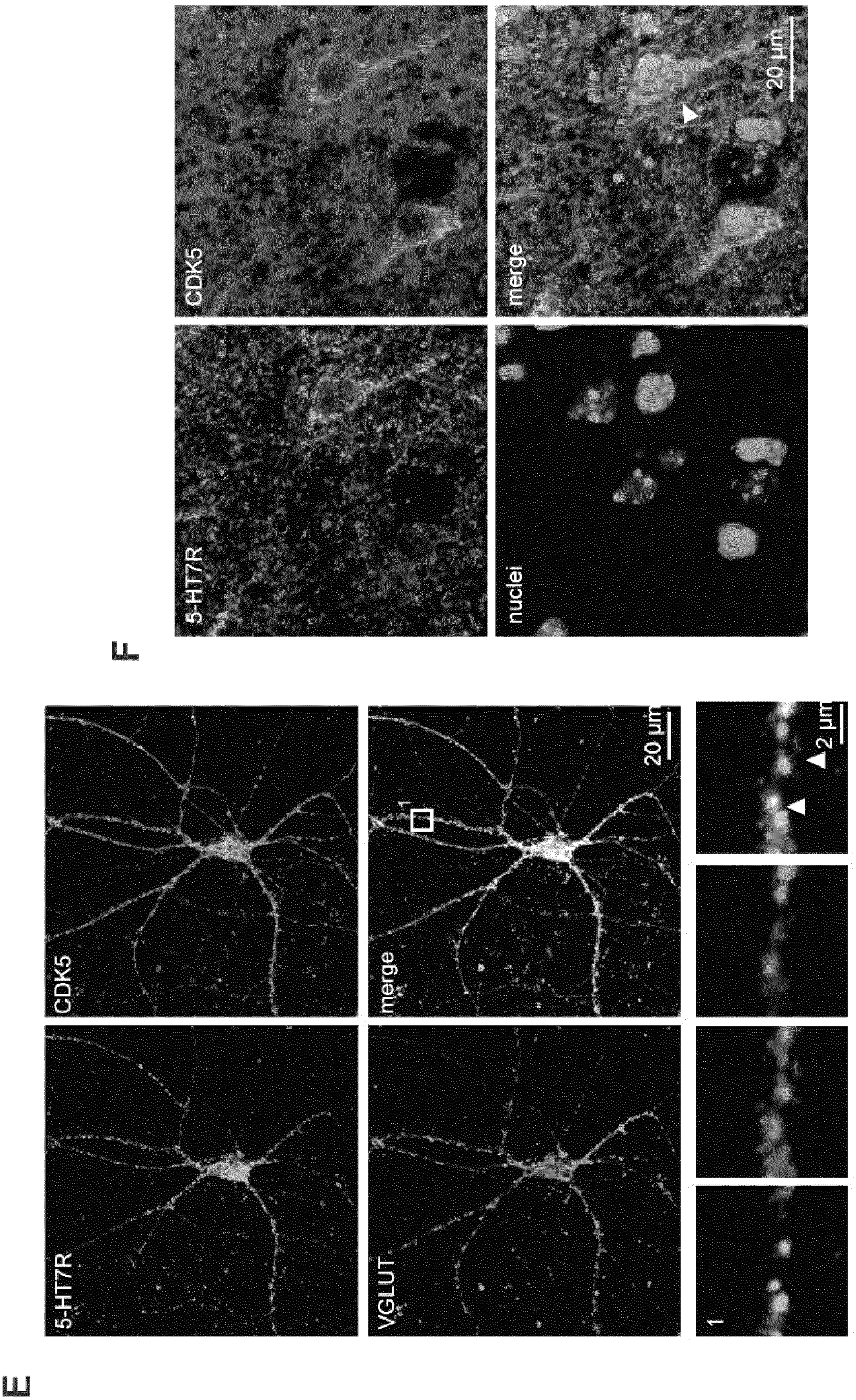

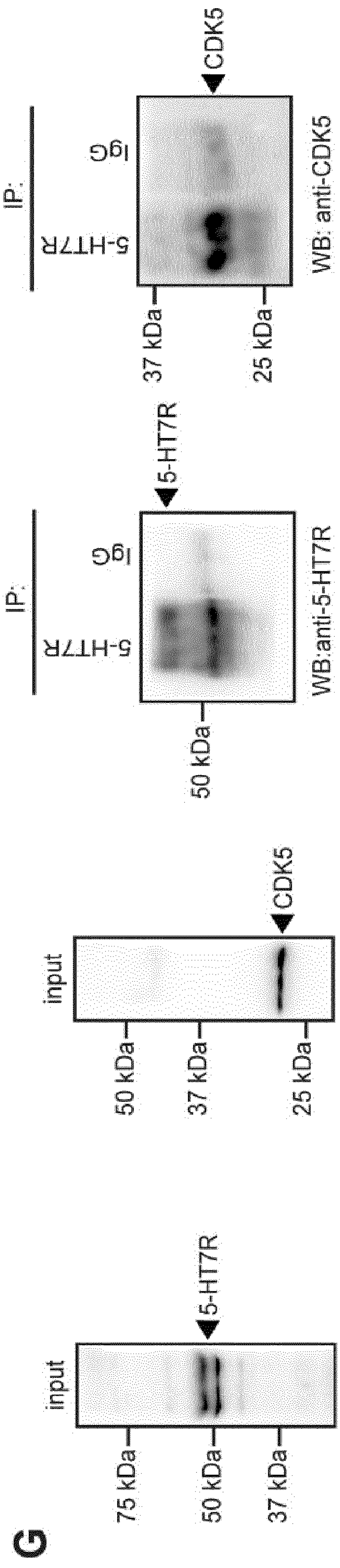


Figure 5 (cont.)

Figure 6

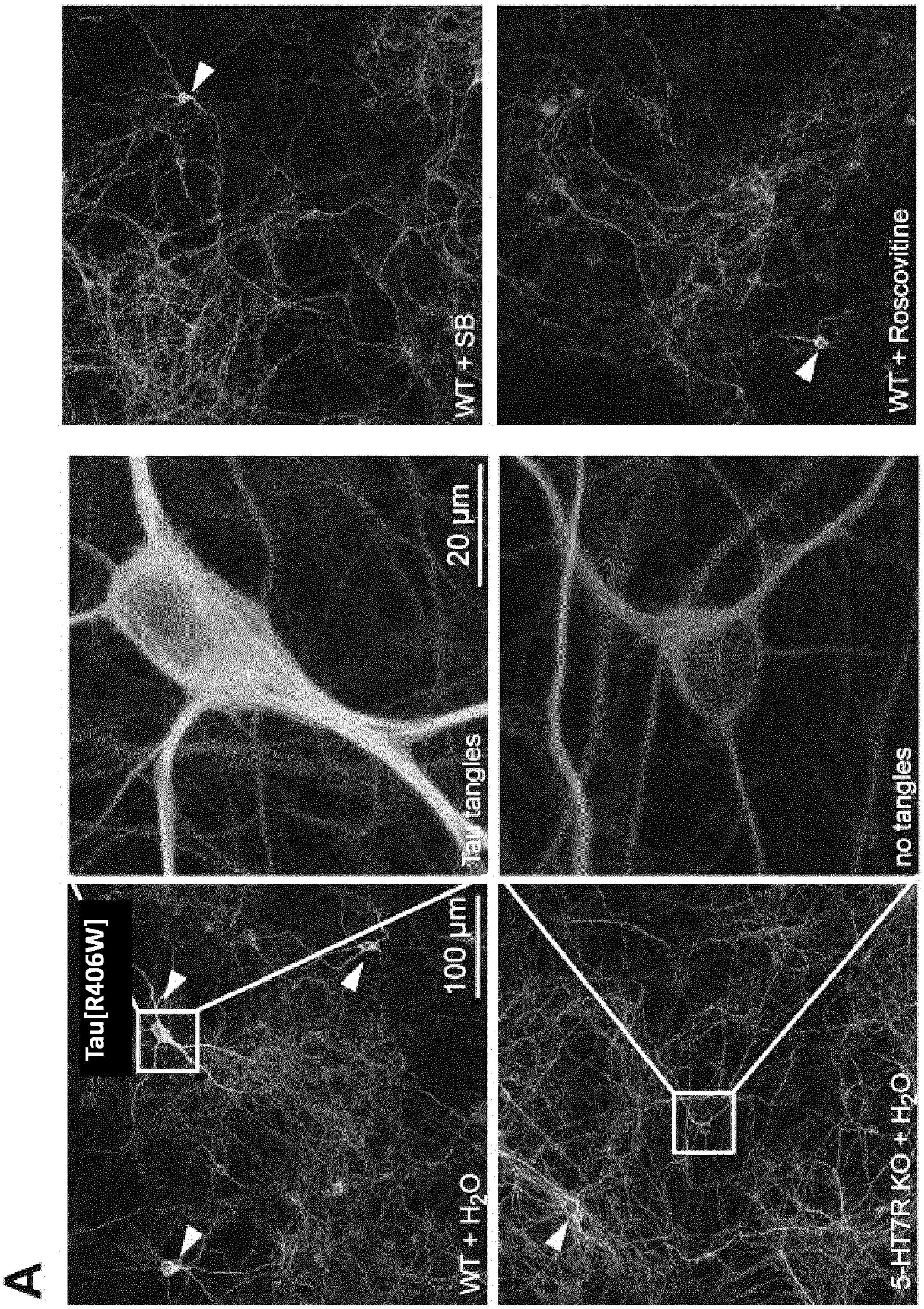
A
Tau[R406W]
100 µm
WT + $H_2O$
5-HT7R KO + $H_2O$
20 µm
Tau tangles
no tangles
WT + SB
WT + Roscovitine

Figure 6 (cont.)

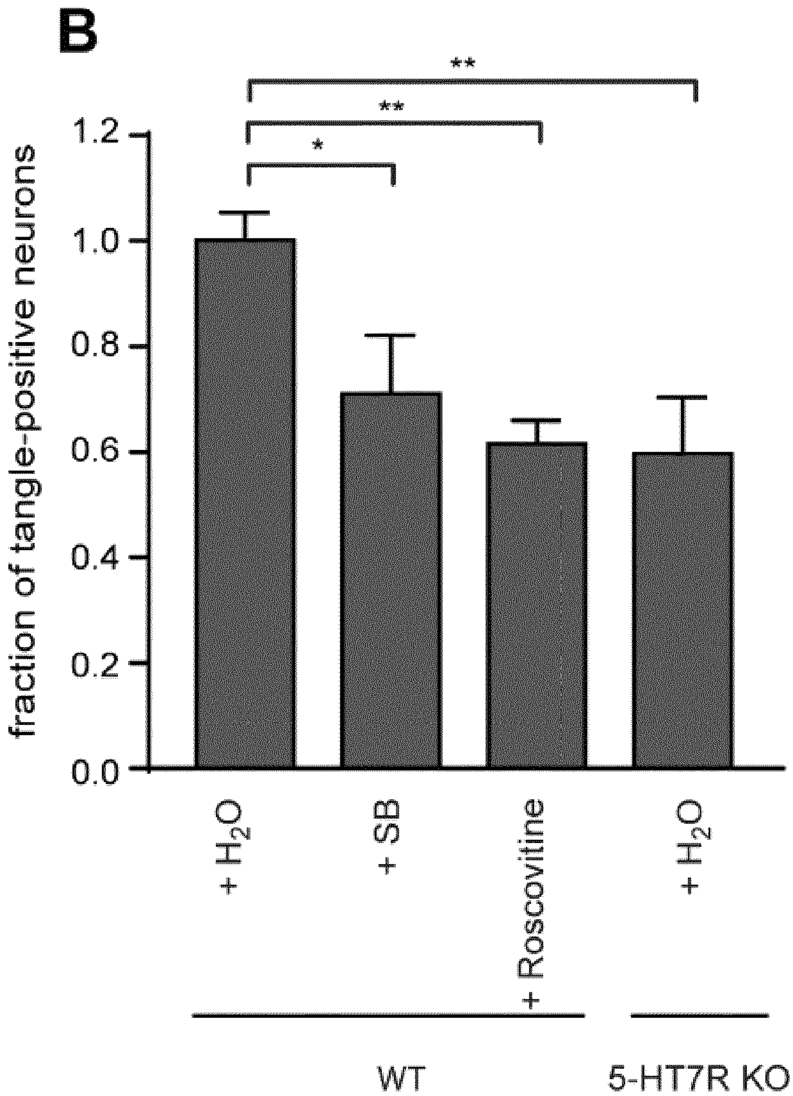

Figure 6 (cont.)

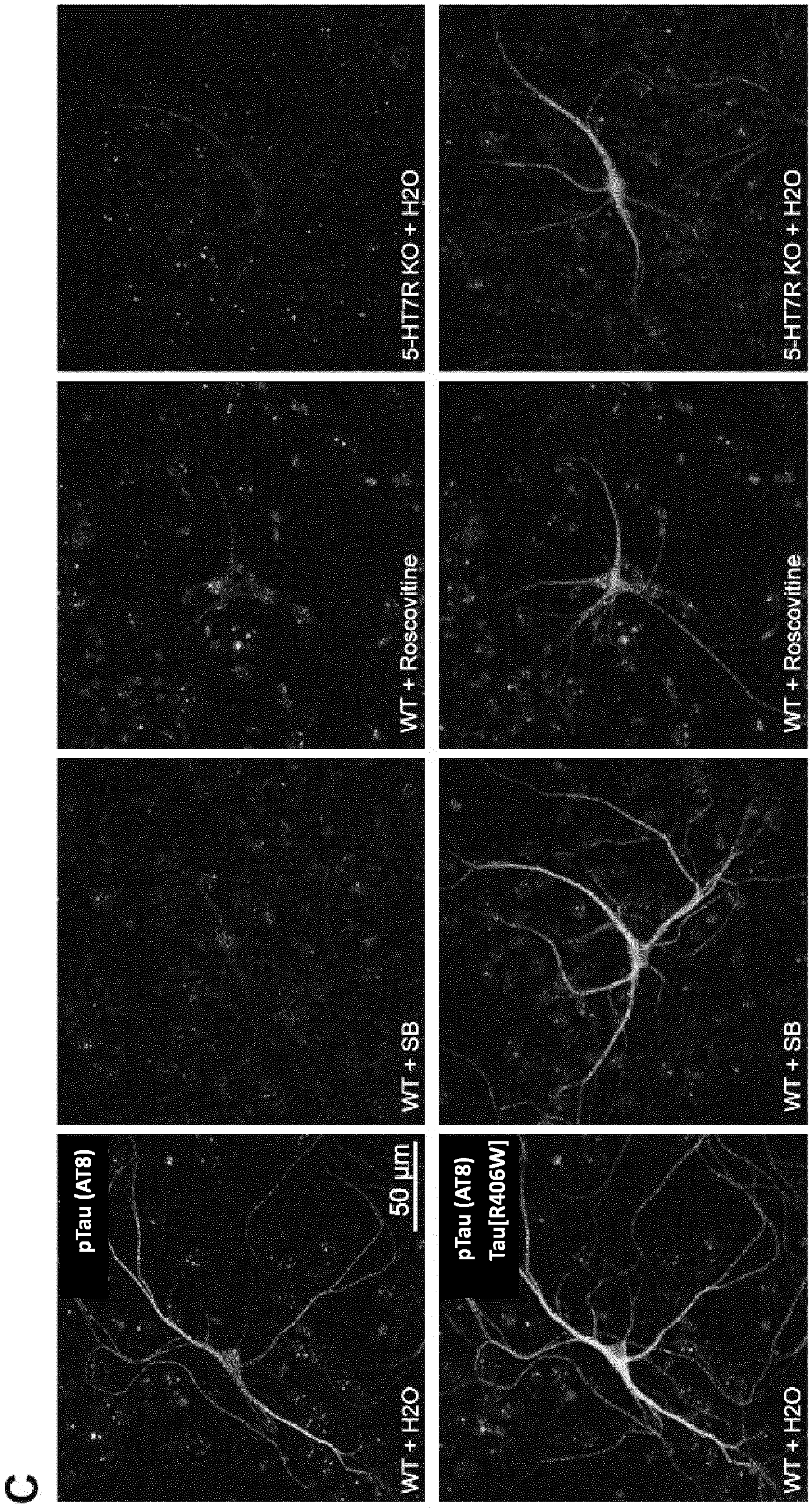

Figure 6 (cont.)

D

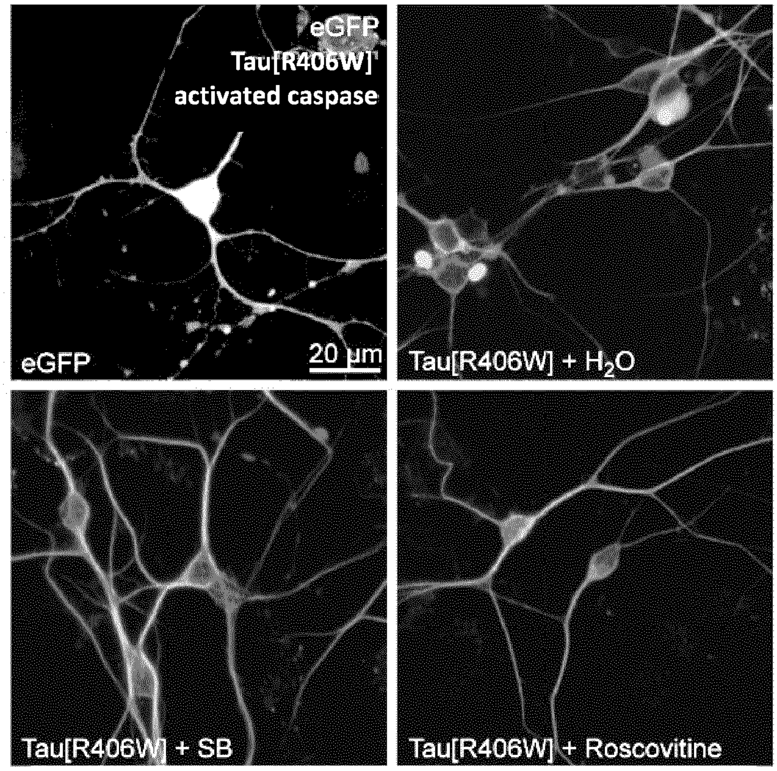


E

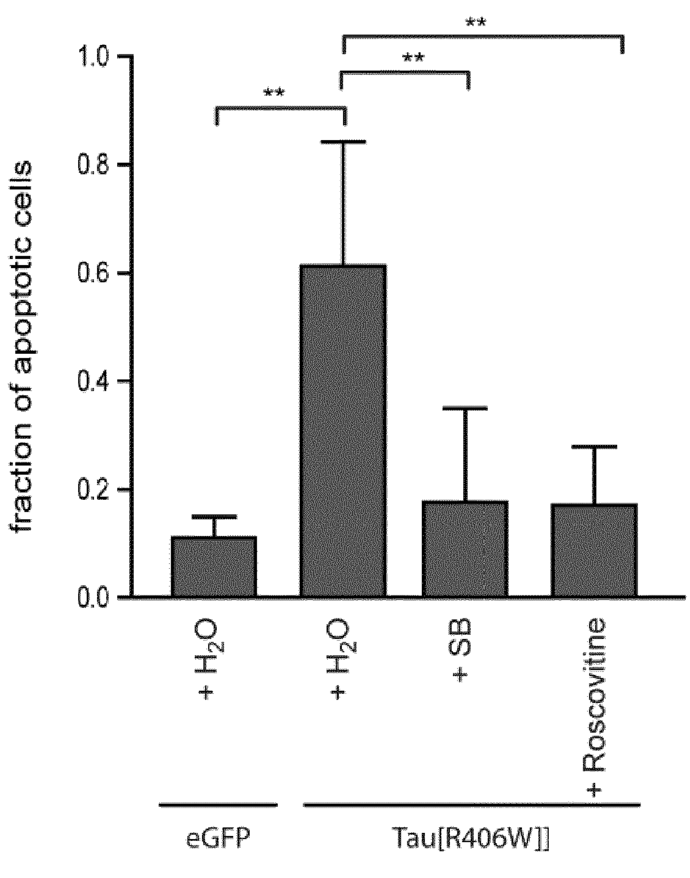

Figure 7

A

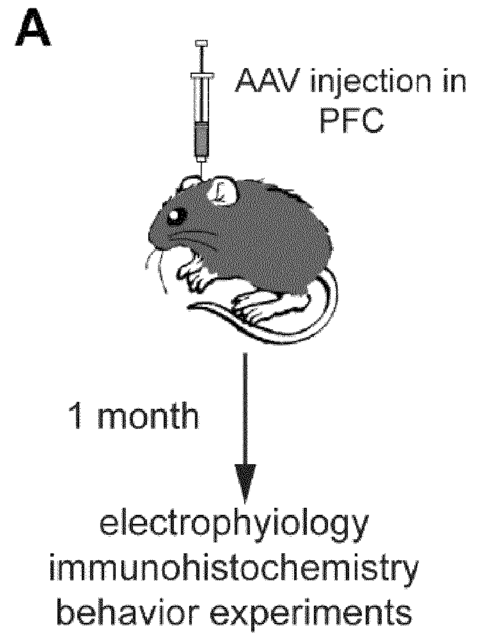
AAV injection in PFC
1 month
electrophyiology
immunohistochemistry
behavior experiments

B

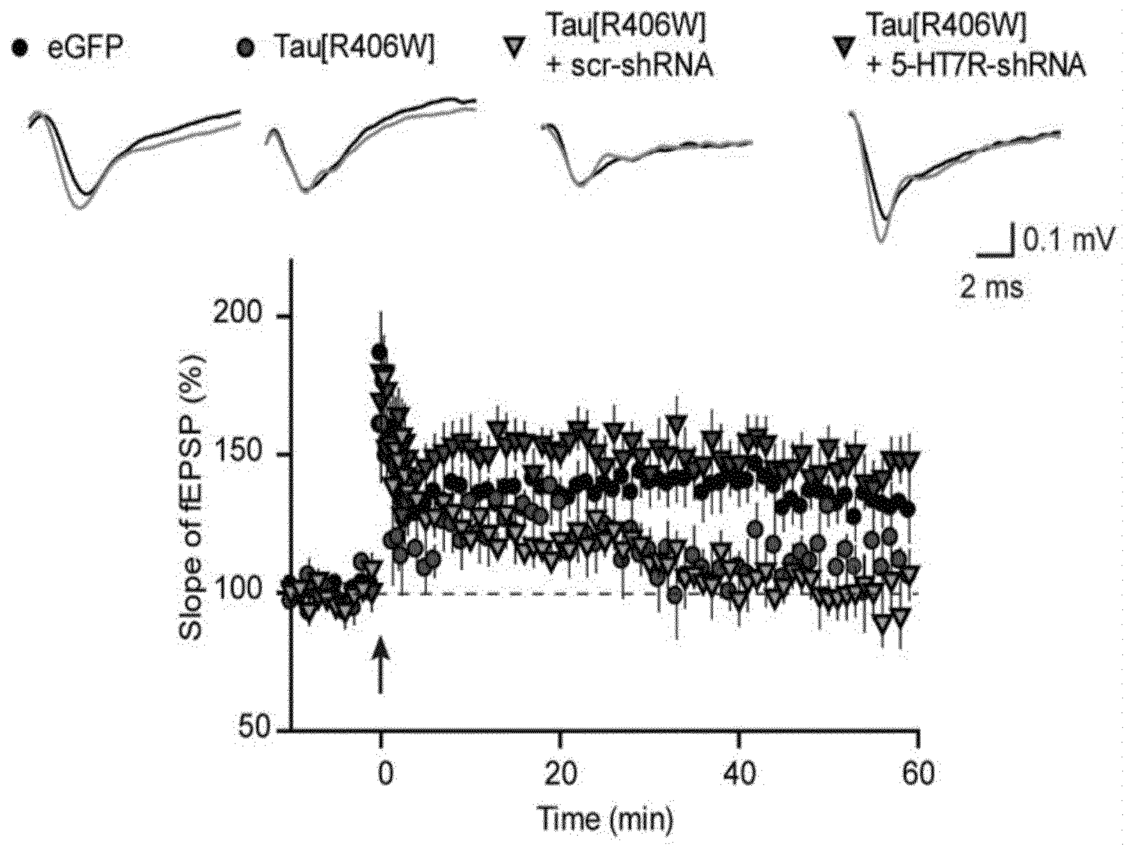
eGFP
Tau[R406W]
Tau[R406W] + scr-shRNA
Tau[R406W] + 5-HT7R-shRNA
0.1 mV
2 ms
Slope of fEPSP (%)
200
150
100
50
0
20
40
60
Time (min)

Figure 7 (cont.)

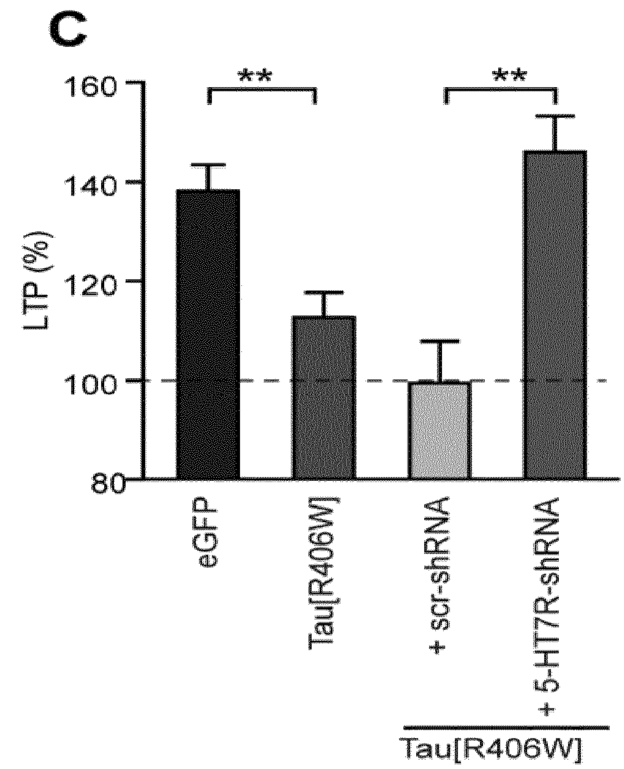


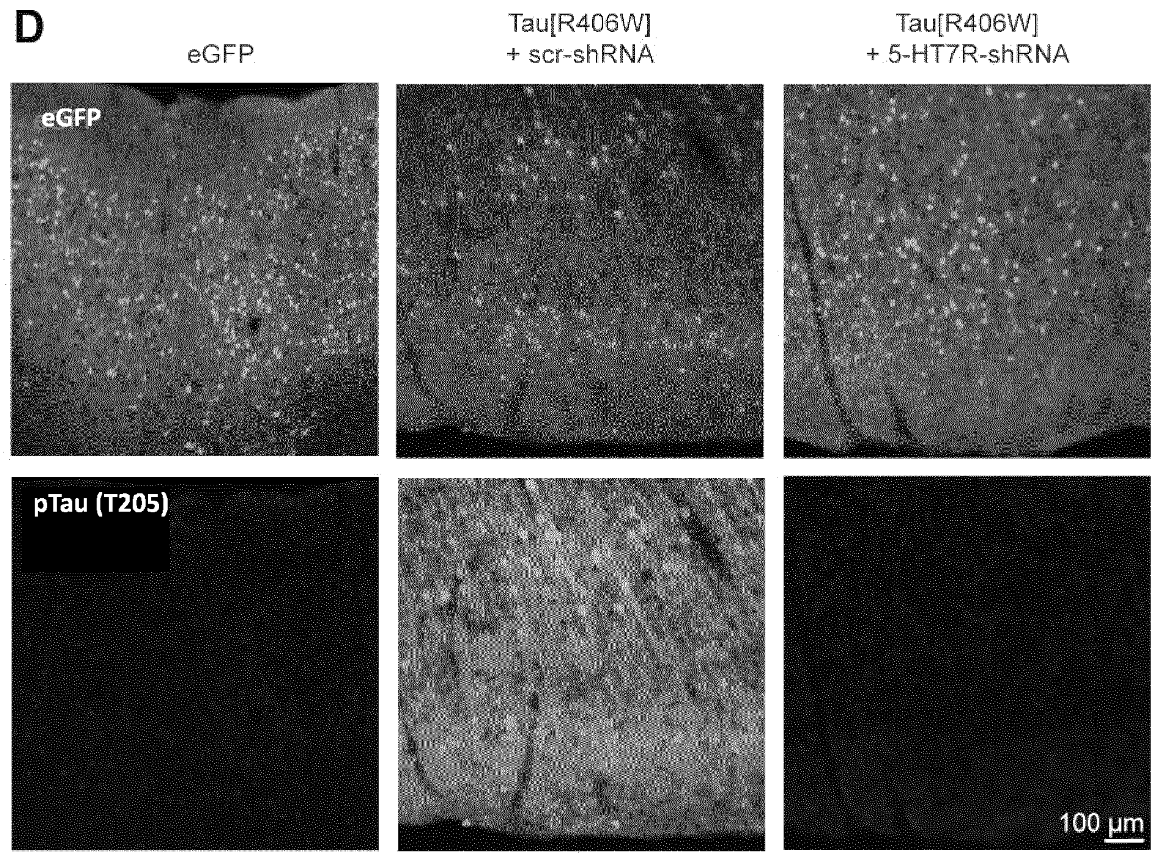

Figure 7 (cont.)

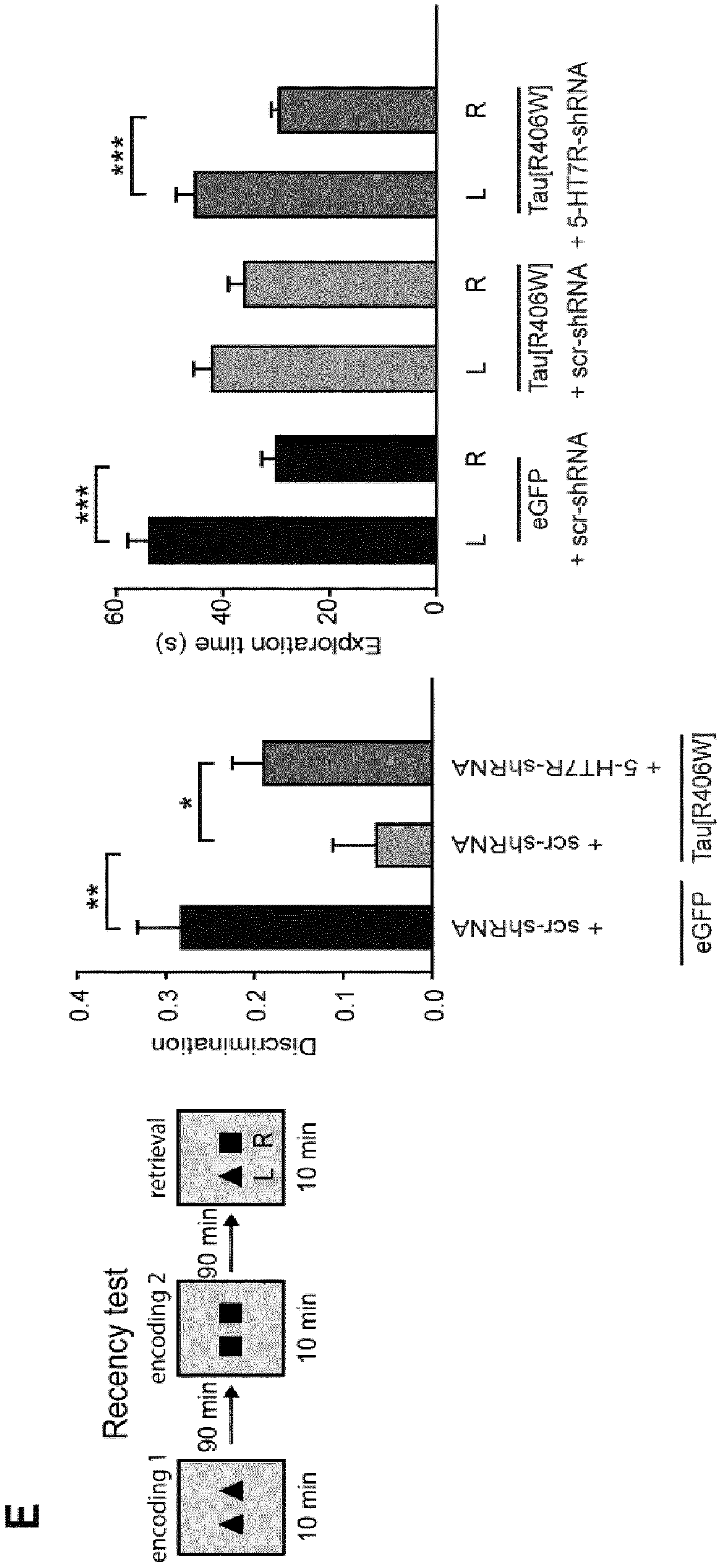

**Figure 8**

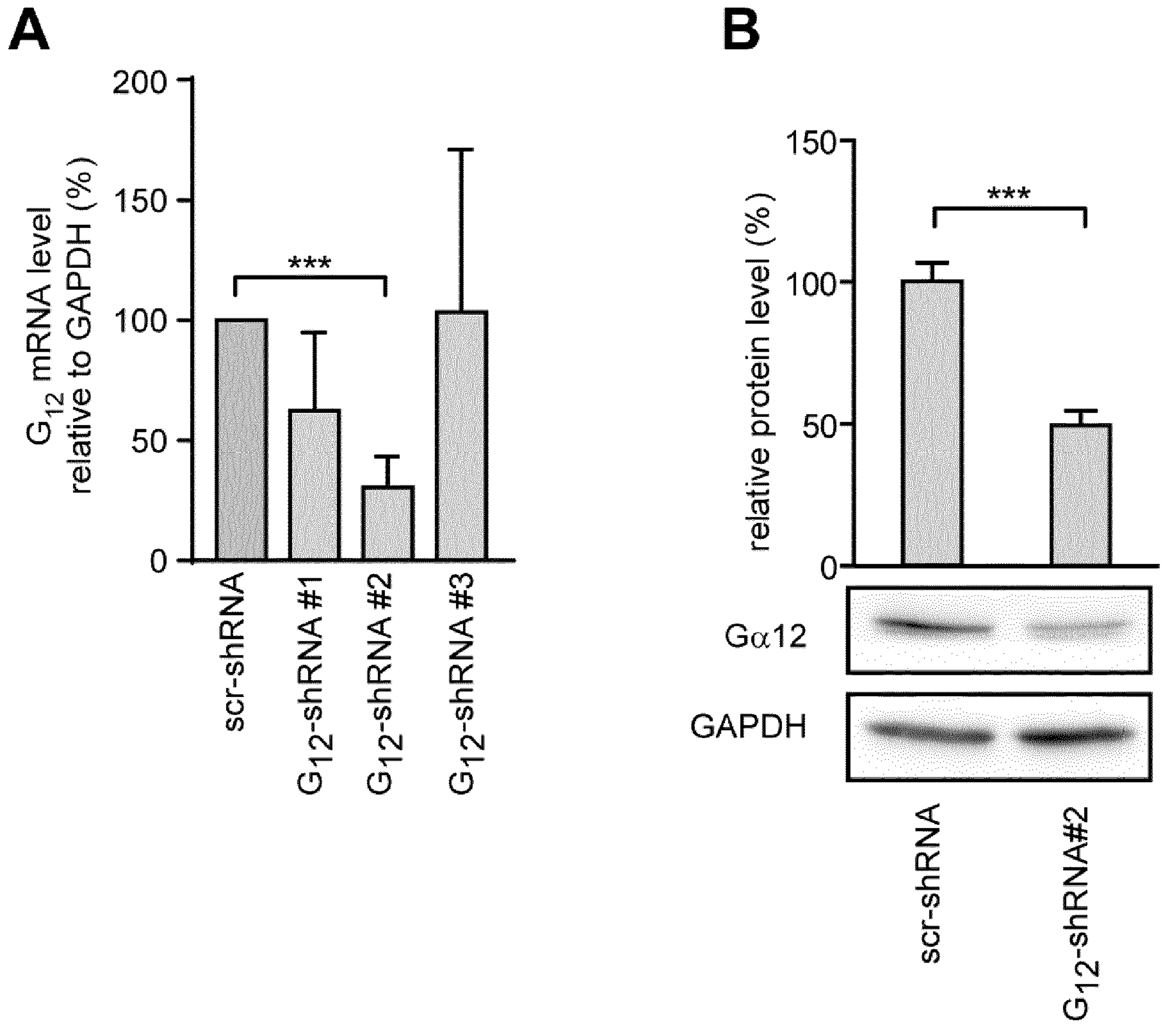
A
200
150
100
50
0
$G_{12}$ mRNA level relative to GAPDH (%)
***
scr-shRNA
$G_{12}$-shRNA #1
$G_{12}$-shRNA #2
$G_{12}$-shRNA #3
B
150
100
50
0
relative protein level (%)
***
Gα12
GAPDH
scr-shRNA
$G_{12}$-shRNA#2

Figure 9

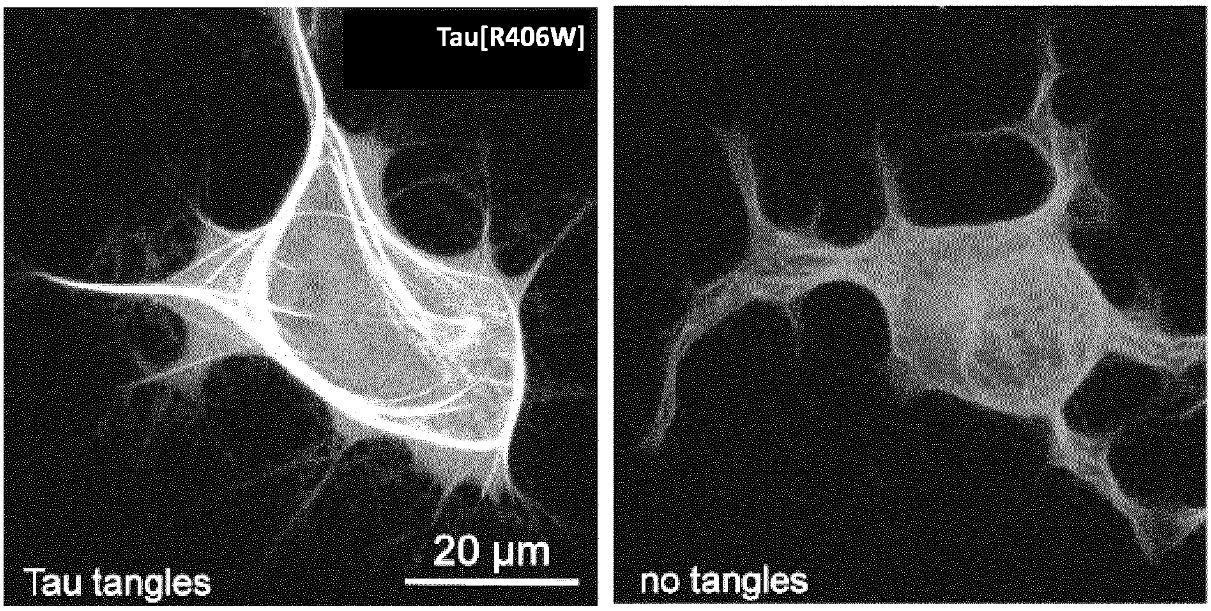

**Figure 10**

A

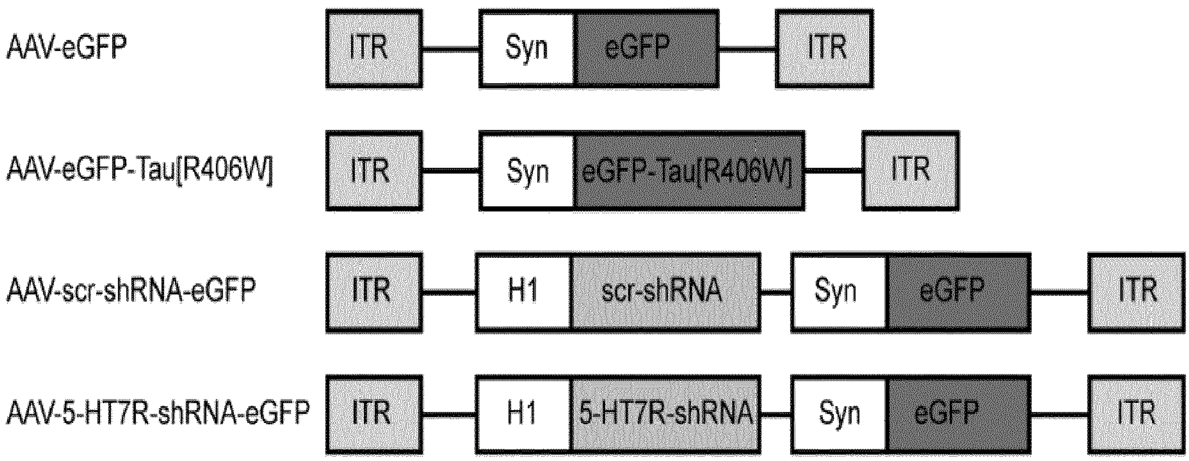
AAV-eGFP
ITR
Syn
eGFP
ITR
AAV-eGFP-Tau[R406W]
ITR
Syn
eGFP-Tau[R406W]
ITR
AAV-scr-shRNA-eGFP
ITR
H1
scr-shRNA
Syn
eGFP
ITR
AAV-5-HT7R-shRNA-eGFP
ITR
H1
5-HT7R-shRNA
Syn
eGFP
ITR

B

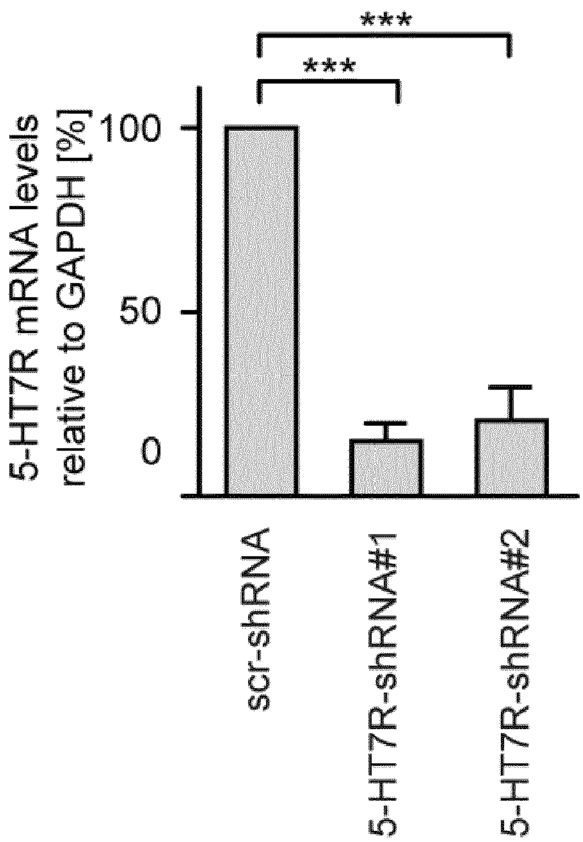
5-HT7R mRNA levels relative to GAPDH [%]
100
50
0
***
***
scr-shRNA
5-HT7R-shRNA#1
5-HT7R-shRNA#2

Figure 10 (cont.)

C

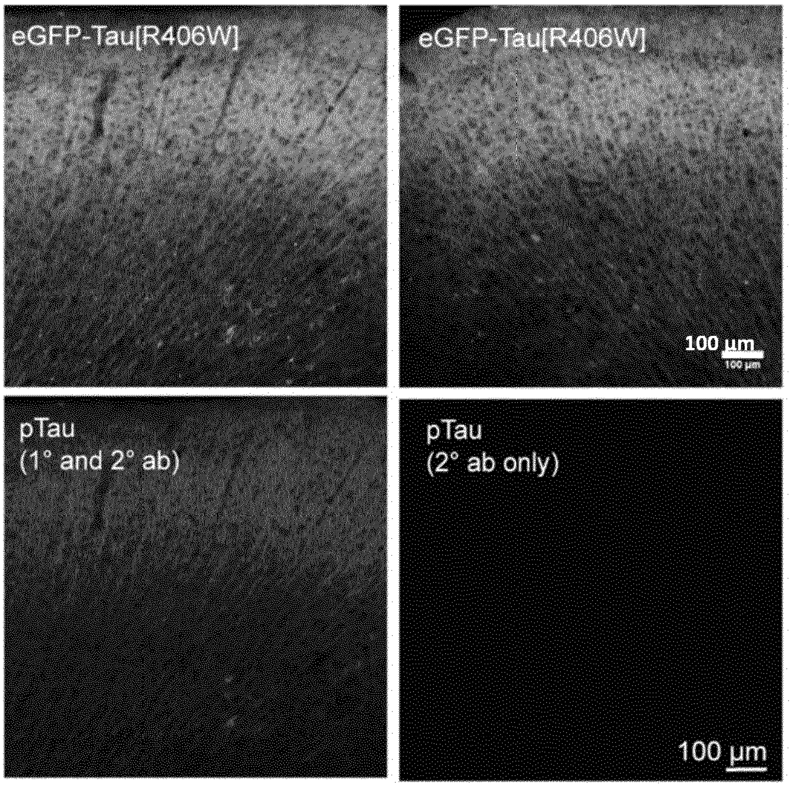


D

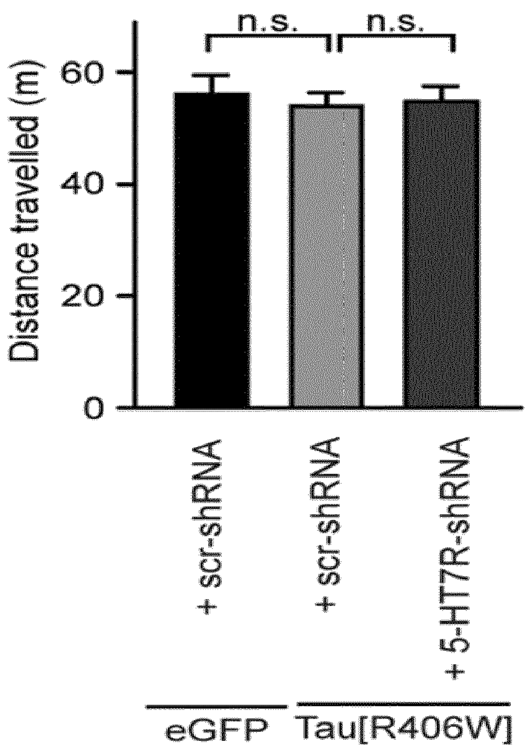

Figure 10 (cont.)

E

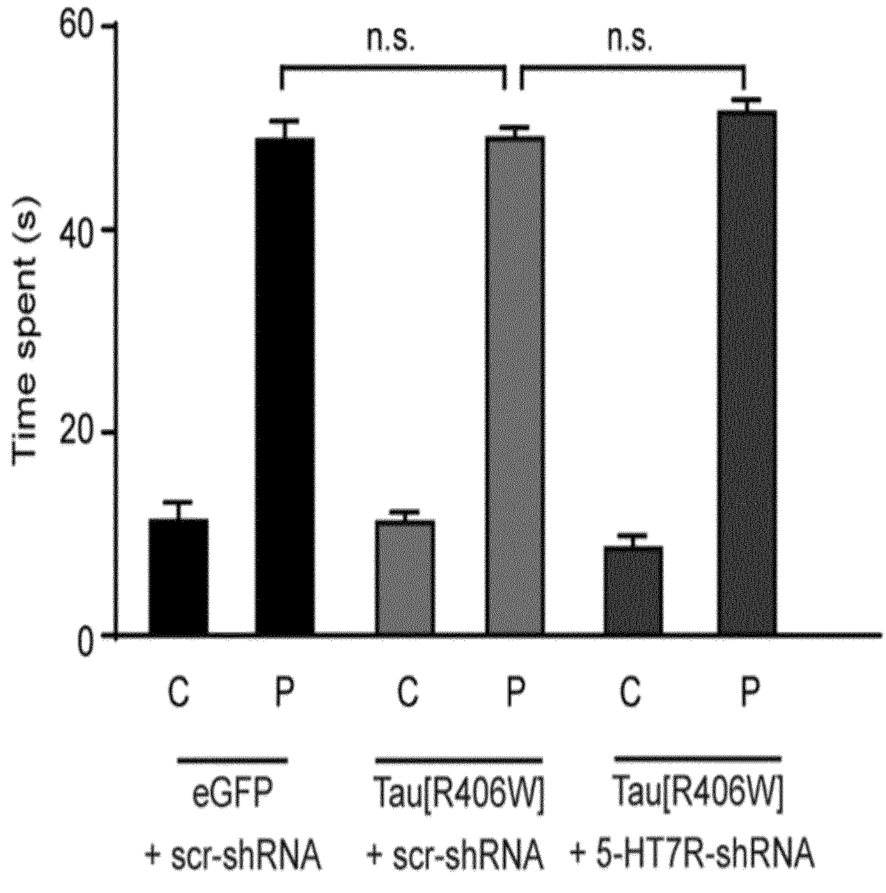

Figure 11

A

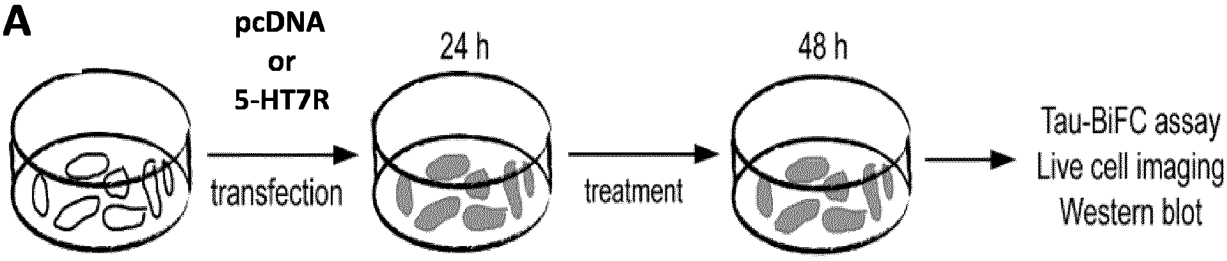
pcDNA
or
5-HT7R
transfection
24 h
treatment
48 h
Tau-BiFC assay
Live cell imaging
Western blot

B

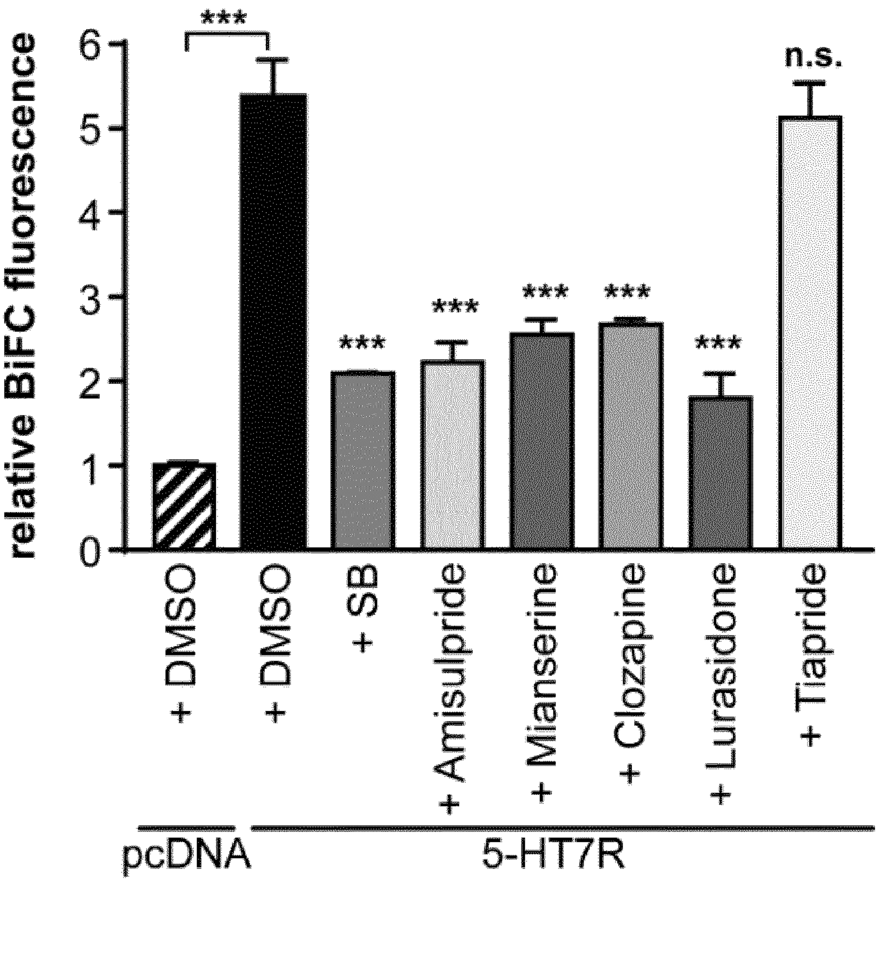
relative BiFC fluorescence
0
1
2
3
4
5
6
***
***
***
***
***
***
n.s.
+ DMSO
+ DMSO
+ SB
+ Amisulpride
+ Mianserine
+ Clozapine
+ Lurasidone
+ Tiapride
pcDNA
5-HT7R

Figure 11 (cont.)

C

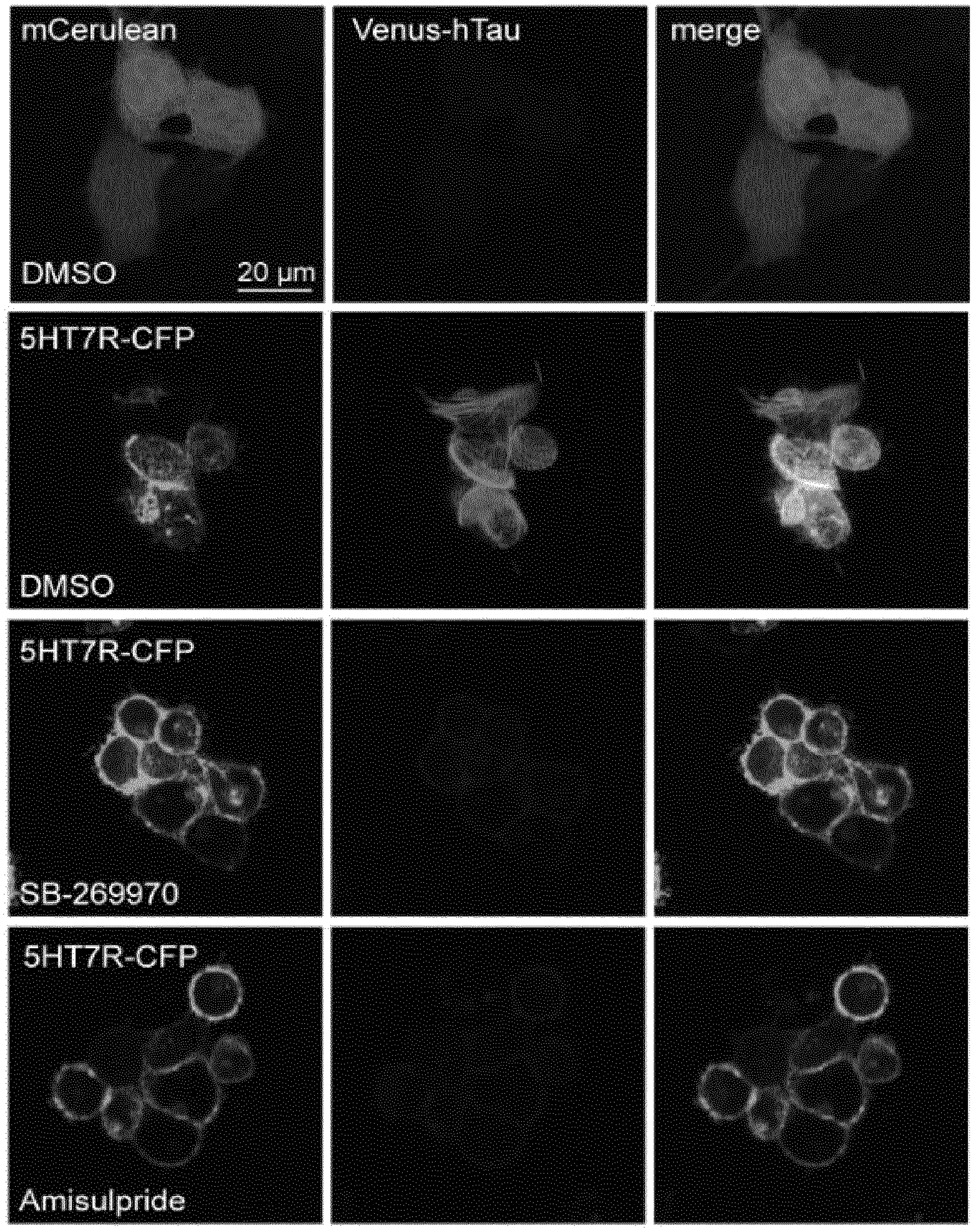

Figure 11 (cont.)

D

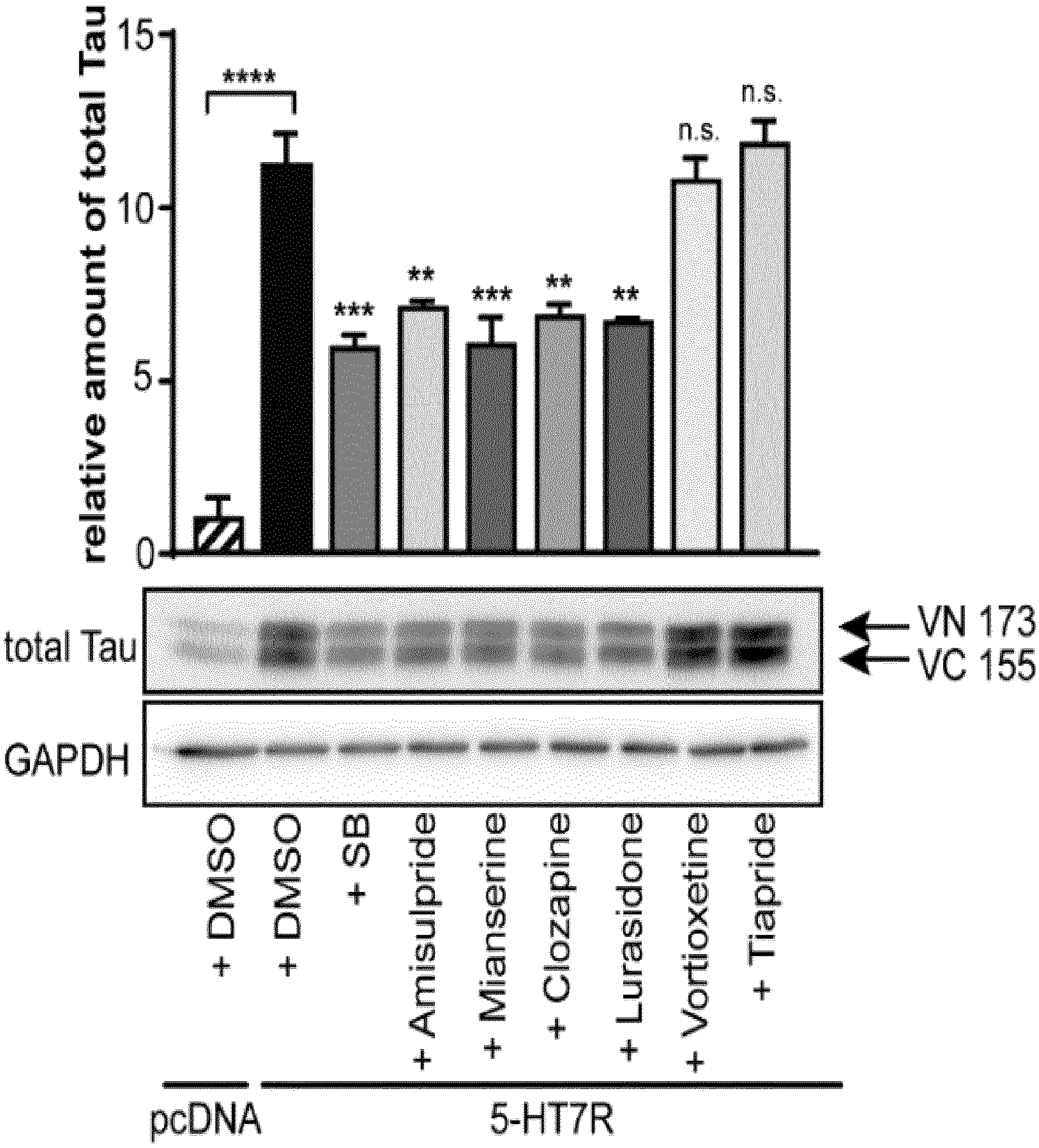

Figure 11 (cont.)

E

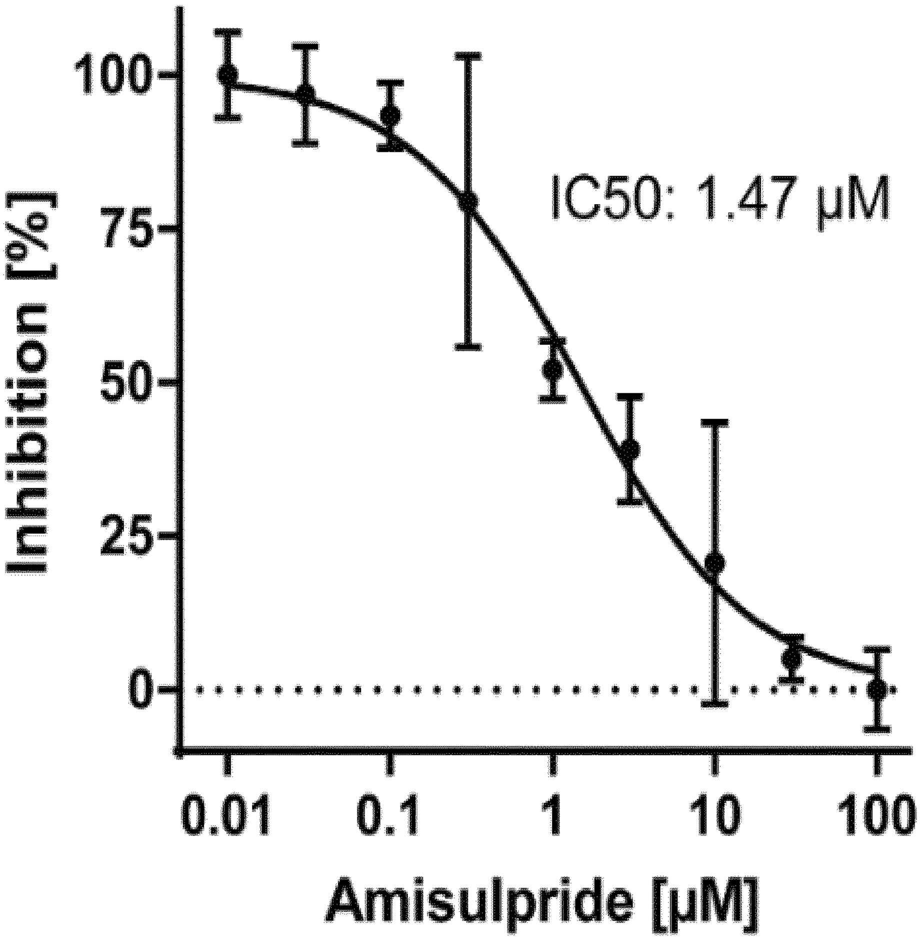

Figure 12

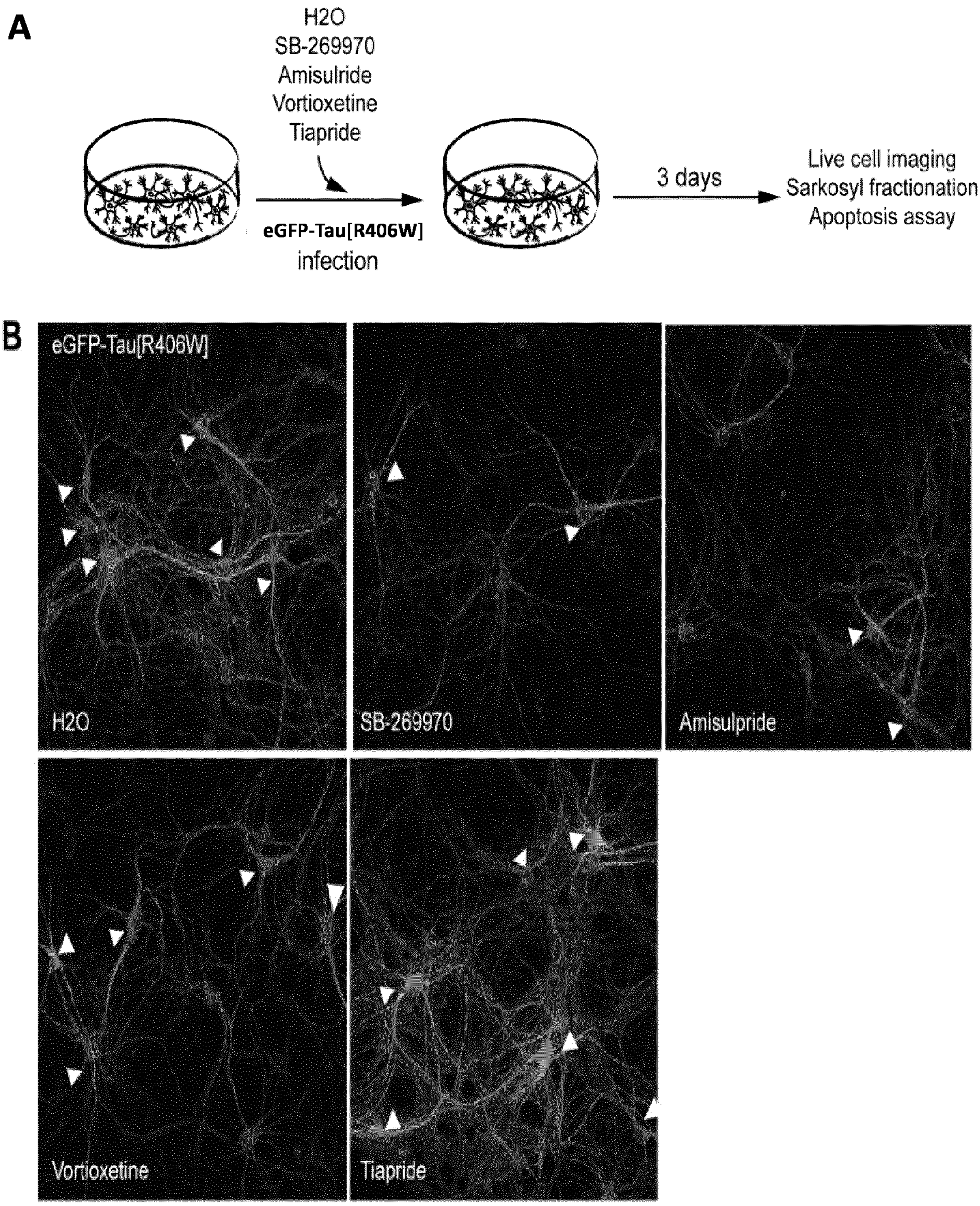
A
H2O
SB-269970
Amisulride
Vortioxetine
Tiapride
eGFP-Tau[R406W]
infection
3 days
Live cell imaging
Sarkosyl fractionation
Apoptosis assay
B
eGFP-Tau[R406W]
H2O
SB-269970
Amisulpride
Vortioxetine
Tiapride

Figure 12 (cont.)

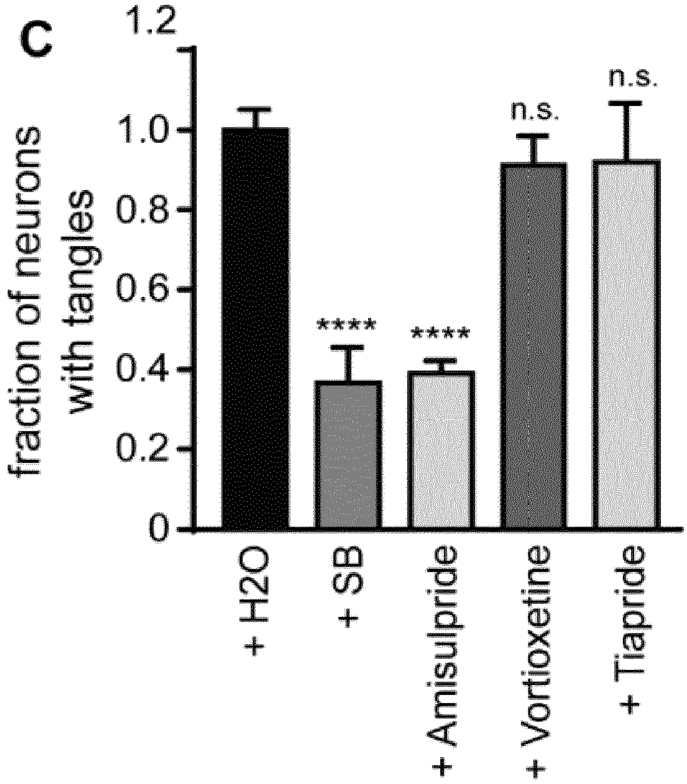


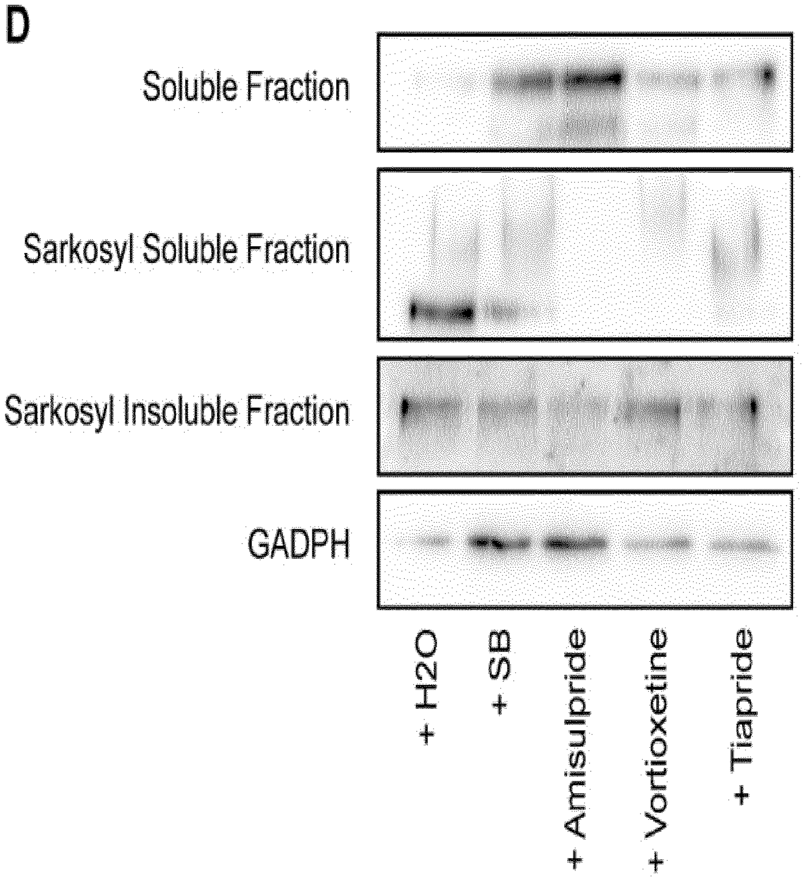

Figure 12 (cont.)

E

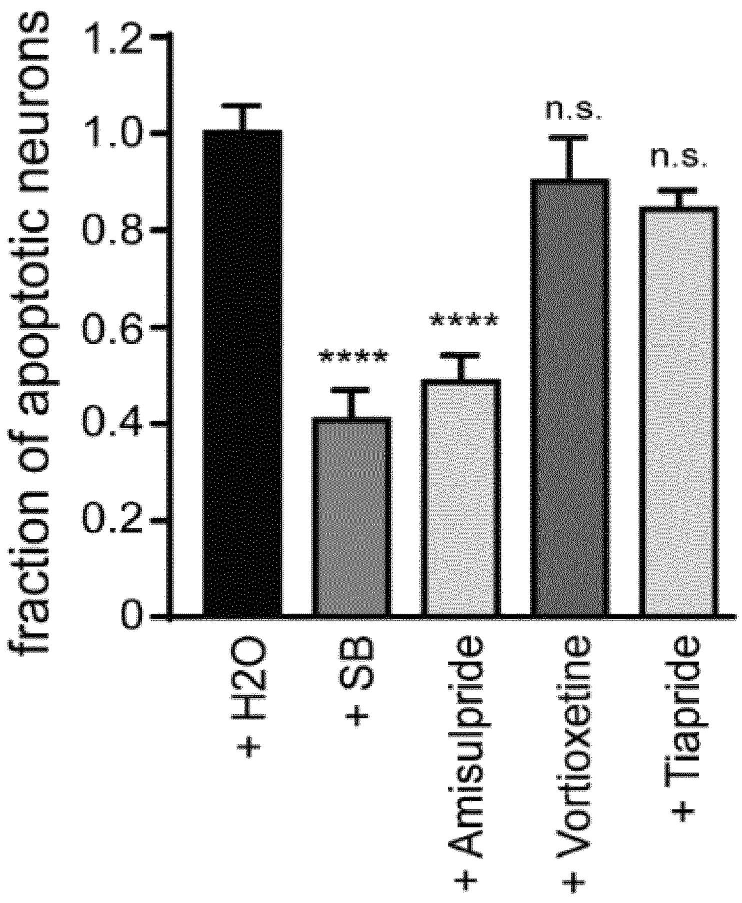

Figure 13

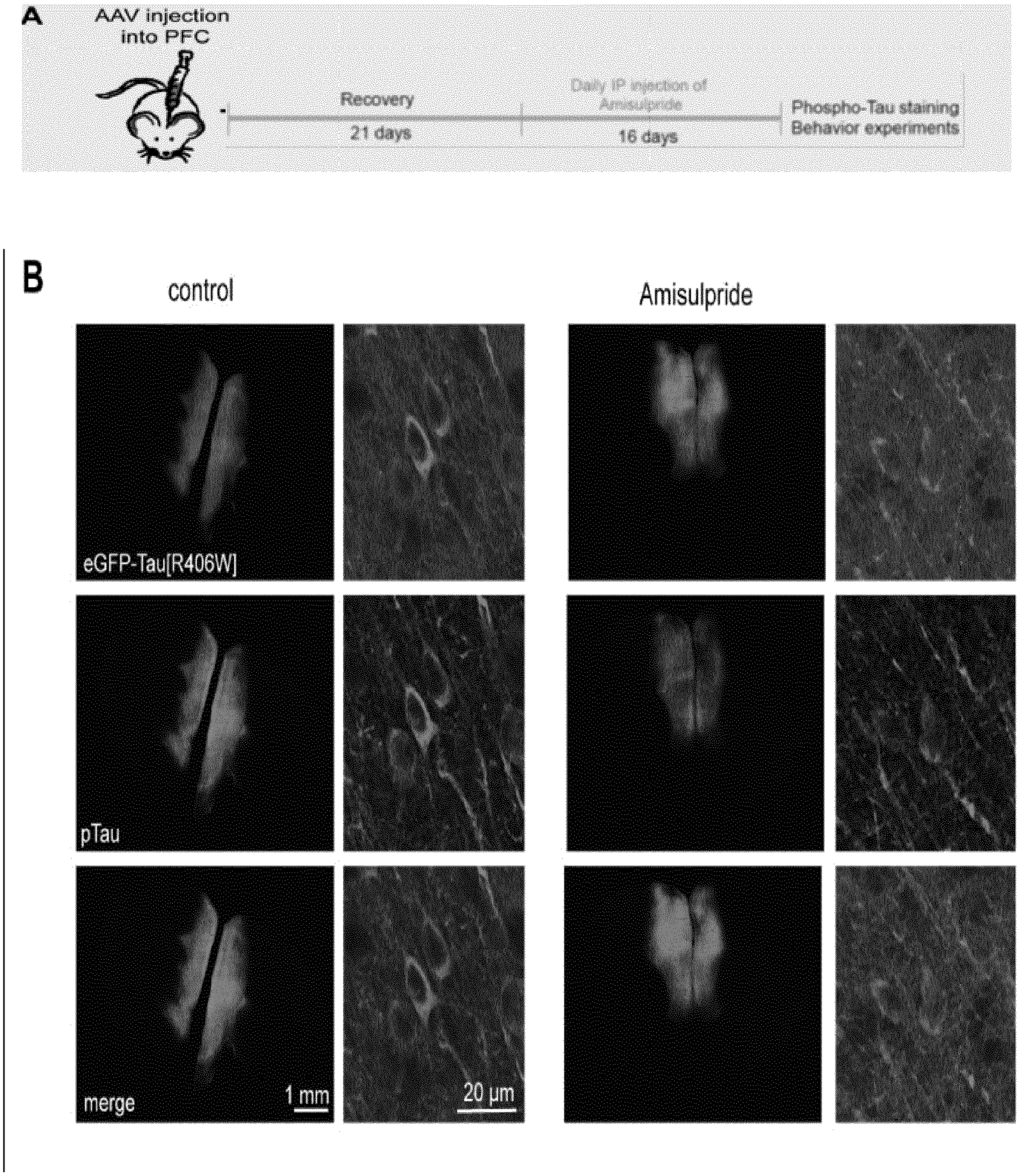
A
AAV injection
into PFC
Recovery
21 days
Daily IP injection of
Amisulpride
16 days
Phospho-Tau staining
Behavior experiments
B
control
Amisulpride
eGFP-Tau[R406W]
pTau
merge
1 mm
20 µm

Figure 13 (cont.)

C

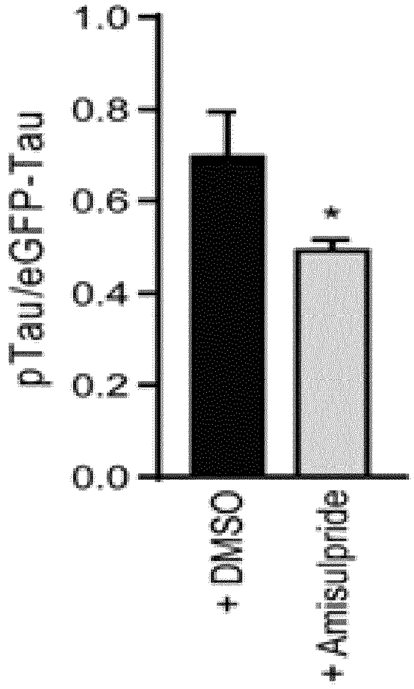


D

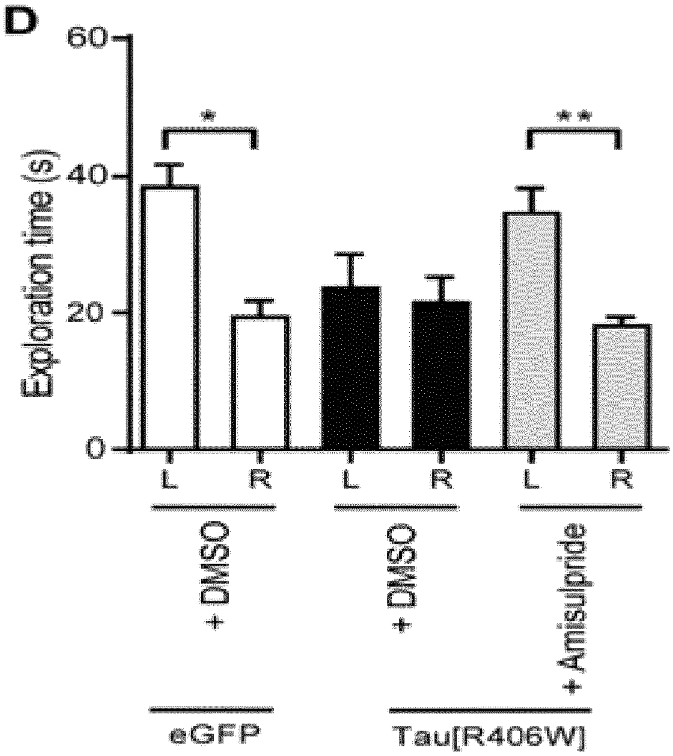


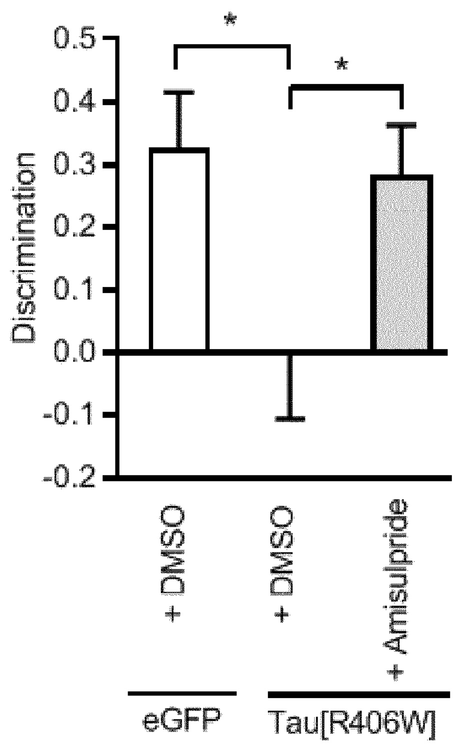

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ARENDT et al.** *Brain Res. Bull.*, 2016, vol. 126, 238-292
- **DUTHEY**. Background paper 6.11: Alzheimer disease and other dementias. *A public Healthy Approach to Innovation*, 2013
- **JOSEPHS**. *Mayo Clin. Proc.*, 2017, vol. 92, 1291-1303
- **WANG ; MANDELKOW**. *Nat. Rev. Neurosci.*, 2015, vol. 17, 22-35
- **BUEE et al.** *Brain Res Brain Res Rev*, 2000, vol. 33, 95-130
- **GASBARRI A et al.** *Behavioural Brain Research*, 16 December 2008, vol. 195 (1), 164-170
- **STRAC et al.** *Transi Neurosci.*, 2016, vol. 7 (1), 35-492
- **GOEDERT et al.** *The Neuroscientist.*, 1997, vol. 3, 131-141
- **BRAAK ; BRAAK**. *Acta Neuropathologica.*, 1991, vol. 82, 239-259
- **ARRIAGADA et al.** *Neurology*, 1992, vol. 42 (3), 631
- **MAHE et al.** *Eur J Pharmacol*, 2004, vol. 495 (2-3), 97-102
- **THOMAS et al.** *British Journal of Pharmacology*, 1998, vol. 124, 1300-1306
- Remington, ''The Science and Practice of Pharmacy. Pharmaceutical Sciences, September 2012
- **ANSEL et al.** Pharmaceutical Dosage Forms and Drug Delivery Systems. Lippincott Williams & Wilkins Publishers, 1999
- **SPERANZA et al.** *Neuropharmacology*, 2013, vol. 67, 155-167
- **JO et al.** *Nature Communcations*, 2013
- **RENNER et al.** *J. Cell Sci.*, 2012, vol. 125, 2486-2499
- **WLODARCZYK et al.** *Biophys. J.*, 2008, vol. 94, 986-1000
- **BRENNAMAN et al.** *Neurobiology of Disease*, 2011, vol. 43, 372-378
- **GUSEVA et al.** *Brain*, 2009, vol. 132, 2180-95
- **NELSON et al.** *Behavioral neuroscience*, 2011, vol. 125, 396-403
- **TAK et al.** *Bimolecular Fluorescence Complementation*, 2013
- Lighting-Up Tau-Tau Interaction in Living Cells. *PLOS ONE*, 2013, vol. 8
- **MACCIONI et al.** *Arch. Med. Res.*, 2001, vol. 41, 226-231
- **HONG et al.** *Science*, 1998, vol. 282, 1914
- **PEREZ et al.** *J. Neurochem.*, 2000, vol. 74, 2583-2589
- **SAKAUE et al.** *J. Biol. Chem.*, 2005, vol. 280, 31522-31529
- **KVACHNINA**. *J. Neurosci.*, 2005, vol. 25, 7821-7830
- **SAYAS et al.** *J. Neurosci.*, 2002, vol. 22, 6863-6875
- **LUND et al.** *Neurochem.*, 2001, vol. 76, 1221-1232
- **LIU et al.** *Lett.*, 2002, vol. 530, 209-214
- **NIKOLIC et al.** *Genes Dev.*, 1996, vol. 10, 816-825
- **GREENBERG ; DAVIES**. *Proc. Natl. Acad. Sci.*, 1990, vol. 87, 5827-5831
- **REN ; SAHARA**. *Front. Neurol.*, 2013, vol. 4
- **BERGER et al.** *J. Neurosci.*, 2007, vol. 27, 3650-3662
- **ROBERSON et al.** *Science*, 2007, vol. 316, 750-754
- **GARETH et al.** *J. Neuro.*, 2007, vol. 27 (11), 2948-2957
- **NELSON et al.** *Behavioral neuroscience.*, 2011, vol. 125, 396-403
- **TAK et al.** Bimolecular Fluorescence Complementation; Lighting-Up Tau-Tau Interaction in Living Cells 2013. *PLOS ONE*, vol. 8